# EUROPEAN PATENT SPECIFICATION

(11) **EP 0 347 168 B1**
(45) Date of publication and mention of the grant of the patent: **01.09.1993**
(21) Application number: 89305959.2
(22) Date of filing: 13.06.1989
(51) Int. Cl.: C07C 311/15, A61K 31/18, C07C 311/37, C07D 213/76, C07D 233/56, C07D 319/20, C07D 207/277, C07C 323/22, C07C 323/16, C07D 213/42, C07D 211/60

(54) **Derivatives of p-substituted phenyl ester of pivalic acid**
Derivate von p-substituierten Phenylestern von Pivaliksäure
Dérivés d'ester phényl-p-substitués de l'acide pivalique

(30) Priority: 13.06.1988 JP 145450/88; 06.03.1989 JP 53541/89
(43) Date of publication of application: 20.12.1989
(73) Proprietor: ONO PHARMACEUTICAL CO., LTD., Chuo-ku Osaka 541 (JP)
(72) Inventor: Imaki, Katsuhiro Ono Pharmaceutical Co.Ltd. Minase, Mishima-gun Osaka (JP); Arai, Yoshinobu Ono Pharmaceutical Co.Ltd. Minase, Mishima-gun Osaka (JP); Okegawa, Tadao Ono Pharmaceutical Co.Ltd. Minase, Mishima-gun Osaka (JP)
(74) Representative: Bentham, Stephen

(56) References cited:
- DE-A- 2 109 842
- GB-A- 2 136 980
- US-A- 3 793 292
- US-A- 4 188 390

## Description

This invention relates to p-substituted phenyl ester derivatives of pivalic acid having an inhibitory activity on elastase.

Lysosomal hydrolases of neutrophils have an important role in defense reactions against tissue damage caused by microbes or inflammation.

Elastase and cathepsin G, which are neutral serine proteinases exist locally in azurophil granules, and play a part in the decomposition of connective tissue.

In particular, elastase degrades elastic connective tissue by cleaving the cross-linking of elastin which directly maintains the elasticity of e.g. lung tissue, by cleaving the hydrophobic part of protein [J.Cell.Biol., 40, 366 (1969)] and selectively degrading the cross-linking of collagen as well as elastin [J.Biochem., 84, 559 (1978)]. It also acts on tissue proteins such as proteoglycans [J.Clin.lnvest., 57, 615 (1976)]. It will be seen therefore, that elastase plays an important role in the metabolism of connective tissue.

Elastase is inactivated by aᵢ-proteinase inhibitor (a₁-PI) which is a common inhibitor for serine proteinases in vivo and an imbalance of enzyme and inhibitor causes the destruction of tissue [Schweiz. Med. Wshr., 114, 895 (1984)].

The turnover of elastin in normal tissue is very slow [Endocrinology, 120, 92 (1978)], but pathological acceleration in degradation of elastin is found in various diseased conditions such as pulmonary emphysema [Am. Rev. Respir. DIS., 110, 254 (1974)], atherosclerosis [Lab. Invest., 22, 228 (1970)] and rheumatoid arthritis [in Neutral Proteases of Human Polymorphonuclear Leukocytes, Urban and Schwarzen- berg, Baltimore - Munich (1978), page 390], which suggests a relationship between elastase and these diseases [infection·inflammation·Immunity, 13, 13 (1983)].

In view of this, many studies on the development of elastase inhibitors have been conducted recently, various substances inhibiting elastase have been proposed, and many patent applications have been filed.

In particular, recently, in the specification of United States Patent No. 4683241, the compounds of the general formula were disclosed: 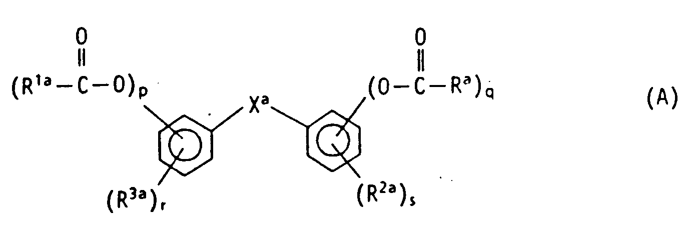 wherein Xa represents a group selected from the carbonyl group, methylene group, oxygen atom, azo group, sulfonyl group, -CH(OH)-, 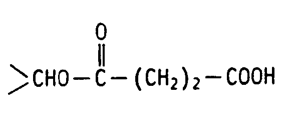 together with the benzene rings represents a group 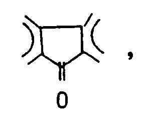
R^{a} and R^{1a} each represents an alkyl group, an acylaminoalkyl group of 2 to 6 carbon atoms, alkoxy group, alkenyl group, carboxyalkyl group of up to 6 carbon atoms, cycloalkyl group of 3 to 6 carbon atoms or alkoxycarbonylalkyl group of up to 10 carbon atoms,
R^{2a} and R^{3a} represent a hydroxy group, halogen atom, pyranyloxy, alkyl, alkenyl, hydroxyalkyl, formylalkyl group of up to 4 carbon atoms or carboxyalkyl group of up to 6 carbon atoms.

As a result of experimentation and research to find new elastase inhibitory agents having quite different chemical structure from conventional ones, the inventors have now found that the compounds of the general formula (I) possess inhibitory activity on elastase.

In the specification of United States Patent No. 4683241 mentioned above, no benzoylphenyl esters or benzenesulfonylphenyl esters of pivalic acid of any kind were disclosed as inhibitory agents on elastase.

The compounds of the present invention are sulfamoylphenyl esters and carbamoylphenyl esters differing substantially in structure from the compounds of US 4683241, and it was therefore unexpected that the compounds of the present invention would have an inhibitory effect on elastase. The present invention provides compounds of the general formula: 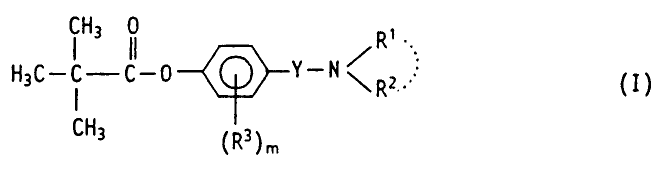 wherein Y represents a sulfonyl (-SO₂-) group or carbonyl 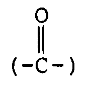 group,
(i) R¹ and R² each independently represents, a hydrogen atom;
   an alkyl group of up to 16 carbon atoms or an alkyl group of up to 16 carbon atoms substituted by a carboxy (-COOH) group;
   a group of the formula: 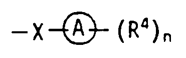 wherein X represents a single bond, a sulfonyl group, an alkylene group of up to 4 carbon atoms or an alkylene group of up to 4 carbon atoms substituted by a carboxy or benzyloxycarbonyl 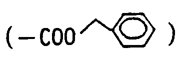 group,
   Ⓐ represents a carbocyclic ring or heterocyclic ring,
   n represents an integer of 1 to 5;
   the groups R⁴ represent independently, a hydrogen atom or an alkyl group of up to 8 carbon atoms;
   an alkoxy group of up to 14 carbon atoms;
   an alkylthio group of up to 6 carbon atoms;
   a hydroxy group, halogen atom, nitro group or trihalomethyl group;
   a group of the formula -NR⁴¹R⁴²
   wherein R⁴¹ and R⁴² each represents, independently, a hydrogen atom or an alkyl group of up to 4 carbon atoms;
   a tetrazole group;
   a sulfonic acid (-SO₃H) group or
   hydroxymethyl (-CH₂0H) group;
   a group of the formula: ^{-S02} NR4 R⁴²
   wherein R⁴¹ and R⁴² are as hereinbefore defined;
   a group of the formula: -Z4'-COOR43 wherein Z⁴¹ represents a single bond, an alkylene group of up to 4 carbon atoms or an alkenylene group of from 2 to 4 carbon atoms and
   R⁴³ represents a hydrogen atom, an alkyl group of up to 4 carbon atoms or a benzyl group;
   a group of the formula -CONR⁴¹R⁴²
   wherein R⁴' and R⁴² are as hereinbefore defined;
   a group of the formula -COO-Z⁴²⁻COO_{R}⁴3
   wherein Z⁴² represents an alkylene group of up to 4 carbon atoms, and
   R⁴³ is as hereinbefore defined;
   a group of the formula: -COO-Z⁴²-CONR⁴¹R⁴²
   wherein Z⁴², R⁴' and R⁴² are as hereinbefore defined;
   a group of the formula: -OCO-R⁴⁵
   wherein R⁴⁵ represents an alkyl group of up to 8 carbon atoms or a p-guanidinophenyl group;
   a group of the formula: -CO-_{R}⁴⁶
   wherein R⁴⁶ represents an alkyl group of up to 4 carbon atoms;
   a group of the formula: -O-Z⁴³-COOR^{45o}
   wherein R⁴³ represents an alkylene group of up to 6 carbon atoms, and
   R⁴⁵⁰ represents a hydrogen atom, an alkyl group of up to 8 carbon atoms or a p-guanidinophenyl group;
   a group of the formula: 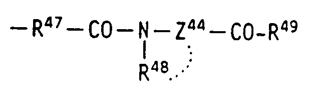 wherein 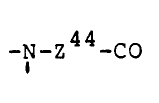 represents an amino acid residue,
   R⁴⁷ represents, a single bond or an alkyl group of up to 4 carbon atoms, R⁴⁸ represents a hydrogen atom or an alkyl group of up to 4 carbon atoms, and R⁴⁹ represents a hydroxy group, alkoxy group of up to 4 carbon atoms, amino group, amino group substituted by one or two alkyl groups of up to 4 carbon atoms, carbamoylmethoxy or carbamoylmethoxy group substituted by one or two alkyl groups of up to 4 carbon atoms at the nitrogen atom of the carbamoyl group, or wherein 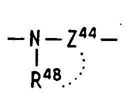 represents a heterocyclic ring containing 4 to 7 atoms in the ring including the nitrogen atom to which Z⁴⁴ and R⁴⁸ are attached and R⁴⁷ and R⁴⁹ each have the same meaning as described hereinbefore, or (ii) R¹, R² and the nitrogen atom bonded to R¹ and R² together represent a heterocyclic ring containing at least one nitrogen atom and substituted by -COOH, or an unsubstituted heterocyclic ring containing at least one nitrogen atom, and R³ represents
   (1) a hydrogen atom;
   (2) a hydroxy group;
   (3) an alkyl group of up to 6 carbon atoms;
   (4) a halogen atom;
   (5) an alkoxy group of up to 4 carbon atoms or
   (6) an acyloxy group of 2 to 5 carbon atoms, and m represents an integer of up to 4, and pharmaceutically acceptable salts thereof.

In this specification including the accompanying claims, the terms "alkyl group", "alkylene group", "alkenylene group", "alkoxy group" and "acyloxy group" mean straight- or branched-chain alkyl group, alkylene group, alkenylene group, alkoxy group and acyloxy groups.

In general formula (I) sulfonyl and carbonyl groups represented by Y are preferred.

In the general formula (I), as an alkyl group of up to 6 carbon atoms represented by R³, methyl, ethyl, propyl, butyl, pentyl and hexyl groups and the isomers thereof are cited and all of them are preferred.

In the general formula (I), as halogen atoms represented by R³ and R⁴, a fluorine atom, a chlorine atom, a bromine atom and an iodine atom are cited.

In the general formula (I), as an alkoxy group of up to 4 carbon atoms represented by R³, methoxy, ethoxy, propoxy and butoxy groups and the isomers thereof are cited and all of them are preferred.

In general formula (I), as an acyloxy group of 2 to 5 carbon atoms represented by R³, acetoxy, propionyloxy, butyryloxy and valeryloxy groups and the isomers thereof are cited and all of them are preferred.

In the general formula (I), as an alkyl group of up to 16 carbon atoms represented by R¹ and R², methyl, ethyl, propyl, butyl, pentyl, hexyl, heptyl, octyl, nonyl, decyl, undecyl, dodecyl, tridecyl, tetradecyl, pentadecyl and hexadecyl groups and the isomers thereof are cited and all of them are preferred.

In the general formula (I), as an alkylene group of up to 4 carbon atoms represented by X and Z4 methylene, ethylene, trimethylene and tetramethylene groups and the isomers thereof are cited, and all of them are preferred.

In the general formula (I), carbocyclic rings represented by Ⓐ are mono- or bi-aromatic carbocyclic rings containing not more than 12 carbon atoms and corresponding rings which may be partially or fully saturated rings.

Examples of these rings mentioned above are benzene, naphthalene, indene, azulene rings and partially or fully saturated derivatives thereof.

In the general formula (I), heterocyclic rings represented by Ⓐ are mono- or bi-aromatic heterocyclic rings containing not more than 12 carbon and hetero atoms and corresponding rings which may be partially or fully saturated; rings containing one or two hetero atoms are preferred.

Examples of the rings mentioned above are furan, thiophene, pyrrole, oxazole, isoxazole, thiazole, isothiazole, imidazole, pyrazole, furazane, pyran, pyridine, pyridazine, pyrimidine, pyrazine, indole, isoindole, benzofuran, benzothiophen, indolidine, chromen, quinoline, isoquinoline, quinolidine, purine, indazole, quinazoline, cinnoline, quinoxaline, phthalazine, pteridine rings and partially or fully saturated derivative thereof.

In the general formula (I), as an alkyl group of up to 8 carbon atoms represented by R⁴, methyl, ethyl, propyl, butyl, pentyl, hexyl, heptyl and octyl and the isomers thereof are cited and all of them are preferred.

In the general formula (I), as an alkoxy group of up to 14 carbon atoms represented by R⁴, methoxy, ethoxy, propoxy, butoxy, pentyloxy, hexyloxy, heptyloxy, octyloxy, nonyloxy, decyloxy, undecycloxy, dodecyloxy, tridecyloxy and tetradecyloxy groups and the isomers thereof are cited and all of them are preferred: particularly methoxy, pentyloxy, decyloxy and the isomers thereof are preferred.

In the general formula (I), as an alkylthio group of up to 6 carbon atoms represented by R⁴, methylthio, ethylthio, propylthio, butylthio, pentylthio and hexylthio groups and the isomers thereof are cited and all of them are preferred.

In the general formula (I), as R⁴, halogen, trihalomethyl, nitro, hydroxy, tetrazole, sulfonic acid and hydroxymethyl are preferred.

In the general formula (I), as an alkyl group of up to 4 carbon atoms represented by R⁴¹, R⁴², R⁴³ and R⁴⁶, methyl, ethyl, propyl and butyl groups and the isomers thereof are cited, and all of them are preferred.

In the general formula (I), as an alkenylene group of 2 to 4 carbon atoms represented by Z⁴¹, vinylene, propenylene and butenylene groups and the isomers thereof are cited, and all of them are preferred.

In the general formula (I), as an alkyl group of up to 8 carbon atoms represented by R⁴⁵, methyl, ethyl, propyl, butyl, pentyl, hexyl, heptyl and octyl groups and the isomers thereof are cited, and all of them are preferred.

In the general formula (I), as an alkylene group of up to 6 carbon atoms represented by Z⁴³, methylene, ethylene, trimethylene, tetramethylene, pentamethylene and hexamethylene groups and the isomers thereof are cited, and all of them are preferred.

In the general formula (I), the amino acid-residue represented by formula

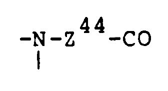

may be any amino acid-residue, including those residues in which the carboxy group is esterified.

Preferably the amino acid-residue is a neutral, acidic or basic amino acid-residue. Specific examples of the residues mentioned above are glycine, alanine, ,8-alanine, valine, phenylalanine, lysine, methionine, tyrosine, proline, leucine, tryptophan, 4-amino butyric acid, 6-aminocaproic acid, 1-amino-1-phenylacetic acid, 2-amino-2-phenylpropionic acid, m-aminobenzoic acid and p-aminobenzoic acid.

As an alkyl group of up to 4 carbon atoms represented by R⁴⁸, methyl, ethyl, propyl and butyl groups and the isomers thereof are cited. As an alkyl group, in the alkoxy group represented by R⁴⁹, as a substituent of the amino group or as a substituent of a carbamoylmethoxy group, methyl, ethyl, propyl and butyl groups and the isomers thereof are cited. As heterocylic rings represented by 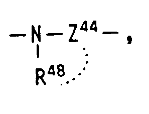 azetidine, pyrrolidine, piperidine and perhydroazepine are cited.

In the general formula (I), heterocyclic rings represented by R¹, R² and the nitrogen atom to which they are attached include mono-heterocyclic rings containing 3 to 6 carbon atoms and 1 or 2 hetero atoms which may be nitrogen and/or oxygen.

Examples of the rings mentioned above are pyrrole, imidazole, pyrazole, pyrroline, pyrrolidine, imidazoline, imidazolidine, pyrazoline, pyrazolidine, piperidine, piperazine, morpholine and azetidine.

Pharmaceutically acceptable salts of the compounds of general formula (I) are preferably non-toxic and water-soluble. They include acid addition salts.

Suitable acid addition salts include, inorganic acid addition salts such as the hydrochloride, hydrobromide, hydroiodide, sulfate, phosphate and nitrate, and organic acid addition salts such as the acetate, lactate, tartrate, benzoate, citrate, methanesulfonate, ethanesulfonate, benzenesulfonate, toluenesulfonate, isethionate, glucuronate and gluconate.

Suitable pharmaceutically acceptable salts also include salts of alkali metals (e.g. sodium or potassium), salts of alkaline earth metals (e.g. calcium or magnesium), ammonium salts, and salts of pharmaceutically acceptable organic amines (e.g. tetramethylammonium, triethylamine, methylamine, dimethylamine, cyclopentylamine, benzylamine, phenethylamine, piperidineamine, monoethanolamine, diethanolamine, tris (hydroxymethyl)amine, lysine, arginine, and N-methyl-D-glucamine salts.

The compounds of the present invention of the general formula (I) may be converted into corresponding salts by any known method.

According to a feature of the present invention, the compounds of the general formula (I) may be prepared by any of the steps described hereinafter.

In the following formula:
R¹¹ and R²¹ have the same meaning as that of R¹ and R², provided that at least one of R¹¹ and R²¹ represents a group containing a benzyloxycarbonyl group,
R¹² and R²² have the same meaning as that of R¹ and R² provided that at least one of R¹² and R²² represents a group containing a carboxyl group,
R¹³ has the same meaning that of R¹ or R² other than a hydrogen atom,
R14 represents an alkyl group of up to 4 carbon atoms,
X represents a halogen atom, and
R³¹ represents an acyloxy group.

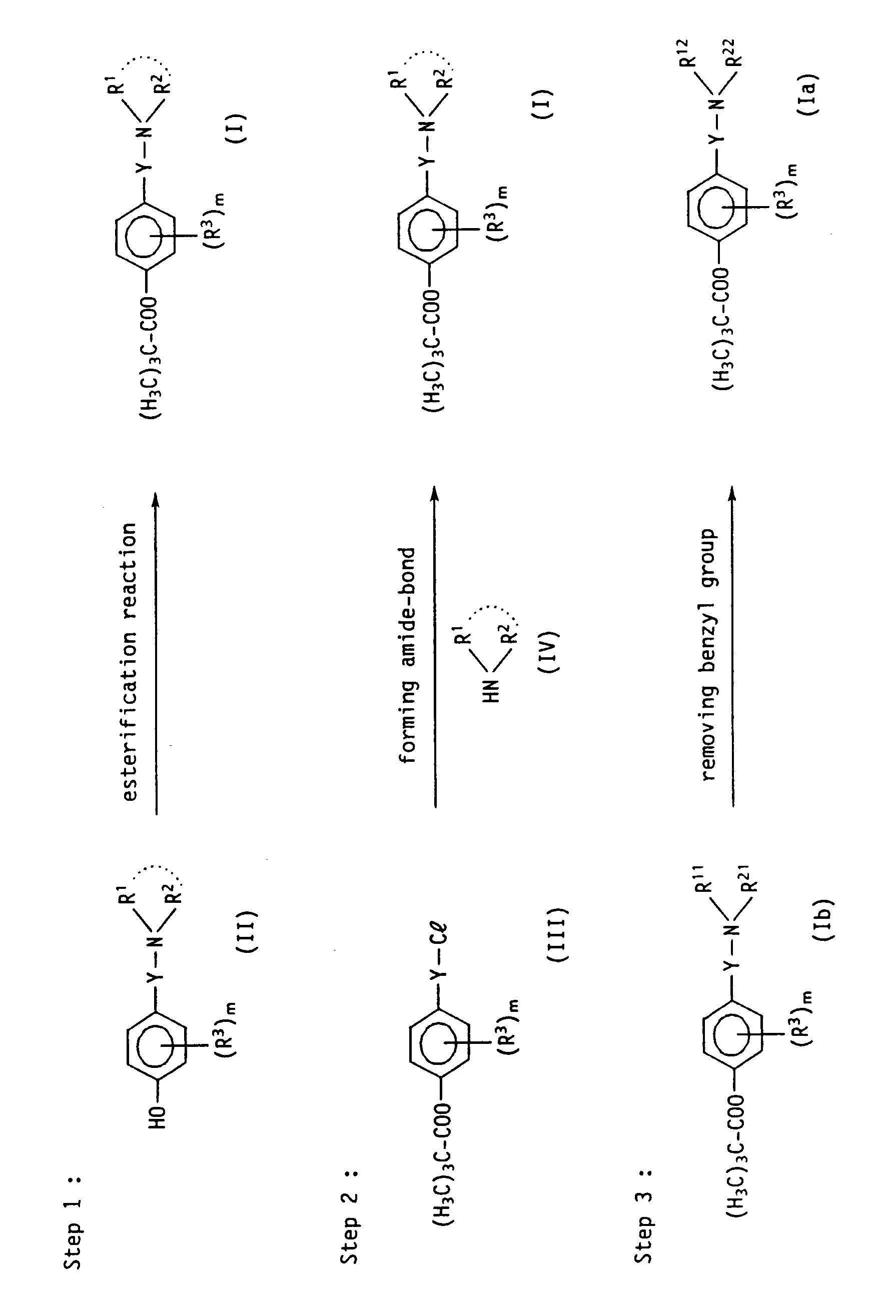

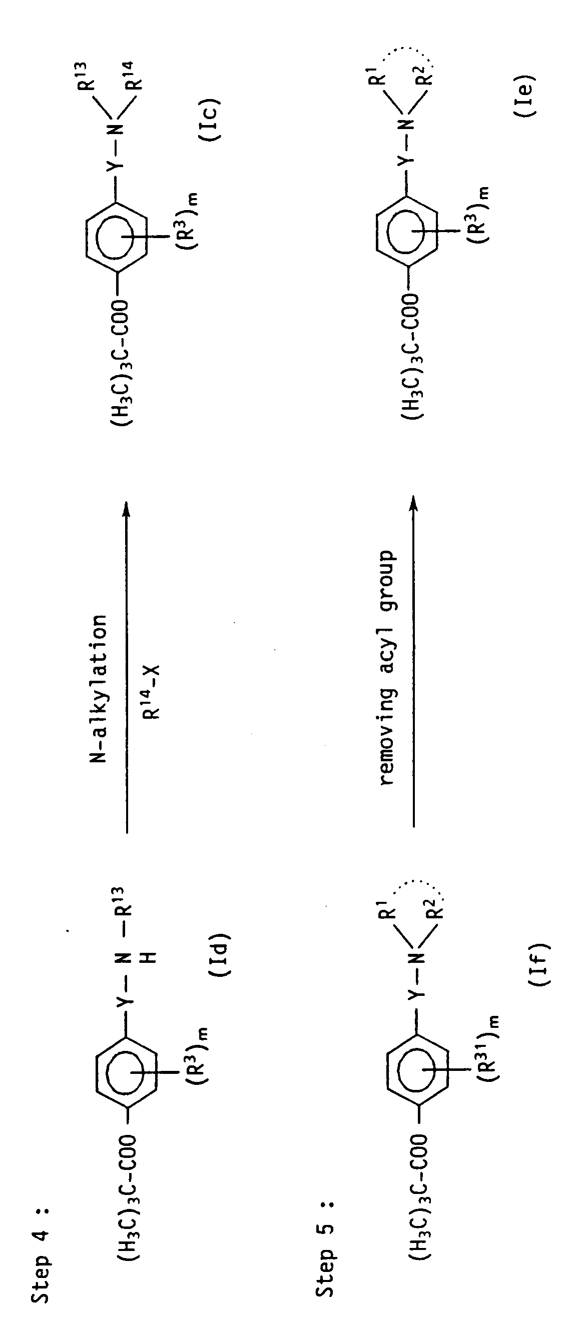

Step 1, which is an esterification reaction, may be carried out for example, by reacting a compound of general formula (II) with pivaloyl halide at room temperature in the presence of a dehydrohalogenation agent in an inert organic solvent (for example, methylene chloride, ethyl acetate, benzene, hexane, diethylether).

As the dehydrohalogenation agent, there can be used a tertiary organic amine, or if desired, an inorganic base such as an alkali metal bicarbonate.

As the tertiary organic amine, there can be used aliphatic, aromatic or heterocyclic amine, for example, triethylamine, tributylamine, dimethylaniline, pyridine.

Pyridine is particularly preferred because it may also be used as a solvent.

Step 2, which is a reaction forming an amide-bond, may be carried out, for example, by reacting the compound of the general formula (III) with an amine in an inert organic solvent (for example methylene chloride), in the presence of organic or inorganic base (for example, a tertiary amine such as triethylamine), at a temperature of -20°C - 0°C (preferably cooling with ice).

Step 3, which is a reaction for removing a benzyl group, may be carried out, for example, by reacting a compound of general formula (Ib) under an atmosphere of hydrogen gas, using palladium-carbon as catalyst in the mixture of inert organic solvents (for example acetic acid and THF), at a temperature of 0°C to 40°C.

Step 4, which is an N-alkylation reaction, may be carried out, for example, by reacting a compound of general formula (Id) with an alkyl halide in a suitable inert organic solvent (for example, benzene, tetrahydrofuran or dimethylformamide), in the presence of a suitable base (for example, sodium hydride), at from room temperature to reflux temperature.

Step 5, which is a reaction of eliminating acyl group, may be carried out for example, by reacting a compound of general formula (If) in methanol, in the presence of a catalyst (for example, triethylamine), at room temperature.

The compounds of the general formula (II) and (III) used in the reactions described above may be prepared by the application of known methods, for example according to scheme A hereinafter.

In the formula in scheme A, G represents a methoxy group or acetoxy group, and the other symbols are as hereinbefore defined.

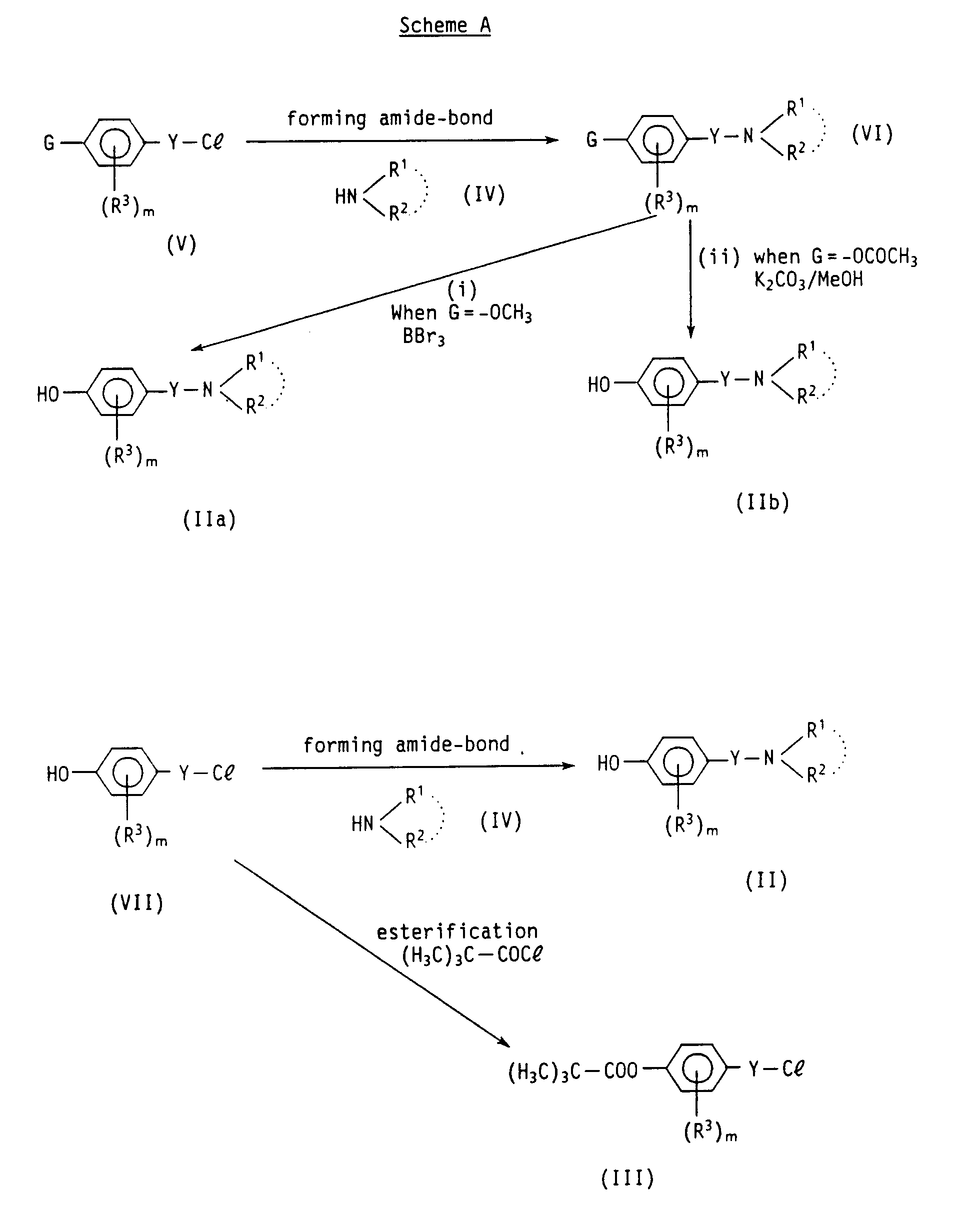

All reactions in the above scheme may be carried out by known methods.

In each reaction in the present specification, products may be purified in known manner. For example, purification may be carried out by distillation at atmospheric or reduced pressure, by high performance liquid chromatography, thin layer chromatography or column chromatography using silica gel or magesium silicate, or by washing or recrystallization. Purification may be carried out after each reaction, or after a series of reactions.

The starting materials of formula (IV), (V) and (VII) in Scheme A are known compounds, or may be prepared by known methods.

For example, the compounds of the general formula (IV) wherein R¹ represents a hydrogen atom and R² represents a group of the formula -X-Ⓐ-(R⁴)n, in which X represents a single-bond, 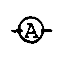 represents a benzene ring and at least one R⁴ represents a group of the formula 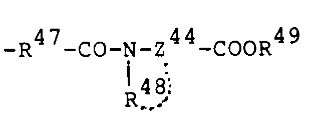 (wherein the various symbols have the meanings hereinbefore described), may be prepared by the scheme B hereinafter.

In the formula, the sum of p and r represents an integer from 1 to 5, and r does not represent zero. R²⁴ represents a hydrogen atom or an alkyl, or alkoxy group as hereinbefore defined for R^{4.}

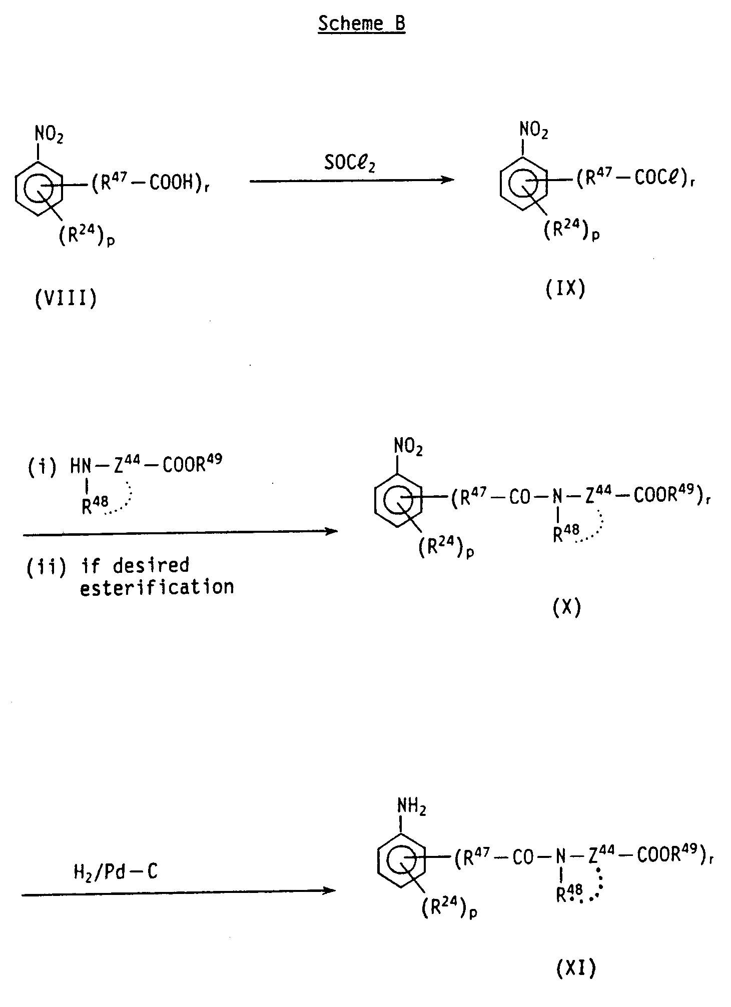

Derivatives of p-substituted phenyl esters of pivalic acid of the general formula (1) of the present invention, and non-toxic acid and an acid addition salts thereof, have an inhibitory effect on elastase.

Accordingly, they are useful for the treatment and/or prevention of diseases induced by abnormal enhancing of the degradation of elastin, collagen fiber and/or proteoglican, by the action of elastase, in mammals, especially in human beings.

Examples of such diseases are pulmonary emphysema, atherosclerosis and rheumatoid arthritis.

The inhibitory effect of compounds on elastase were confirmed by the screening system described.

### (1) Experimental method

The test was carried out by slight modification of the method of Bieth et al [see Biochem. Med., 75, 350 (1974)] using elastase from human neutrophil.

It is a spectrophotometric method using the synthesized substrate [succinyl-alanyl-prolyl-alanyl-p-nitroanilide (Suc-Ala-Pro-Ala-pNA, produced by peptide laboratory)] which has comparatively high specificity on neutrophil elastase.

The reaction mixture consisted of 1 mM Suc-Ala-Pro-Ala-pNA (dissolving in N-methylpyrrolidone to the concentration of 100 mM, and then adding 1/100 amount of the solution to the reaction mixture.), 0.1 M buffer solution of tris-hydrochloric acid (pH 8.0), 0.2 M sodium chloride aqueous solution, the sample solution of various concentrations and enzyme solution in a final volume of 1.0 m was incubated at 37°C for 30 minutes.

The reaction was stopped by the addition of 100 µℓ of 50% acetic acid into the reaction mixture, and then p-nitro anilide released was measured by the absorbance at 405 nm.

Inhibition percentage of the test compounds was calculated by the following equation:

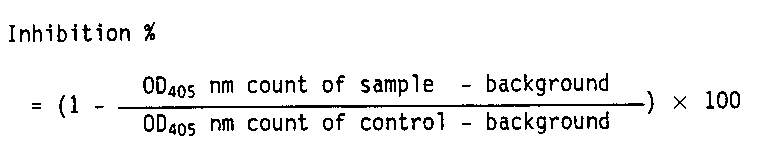

### (2) Results

The results are shown in Table 1.

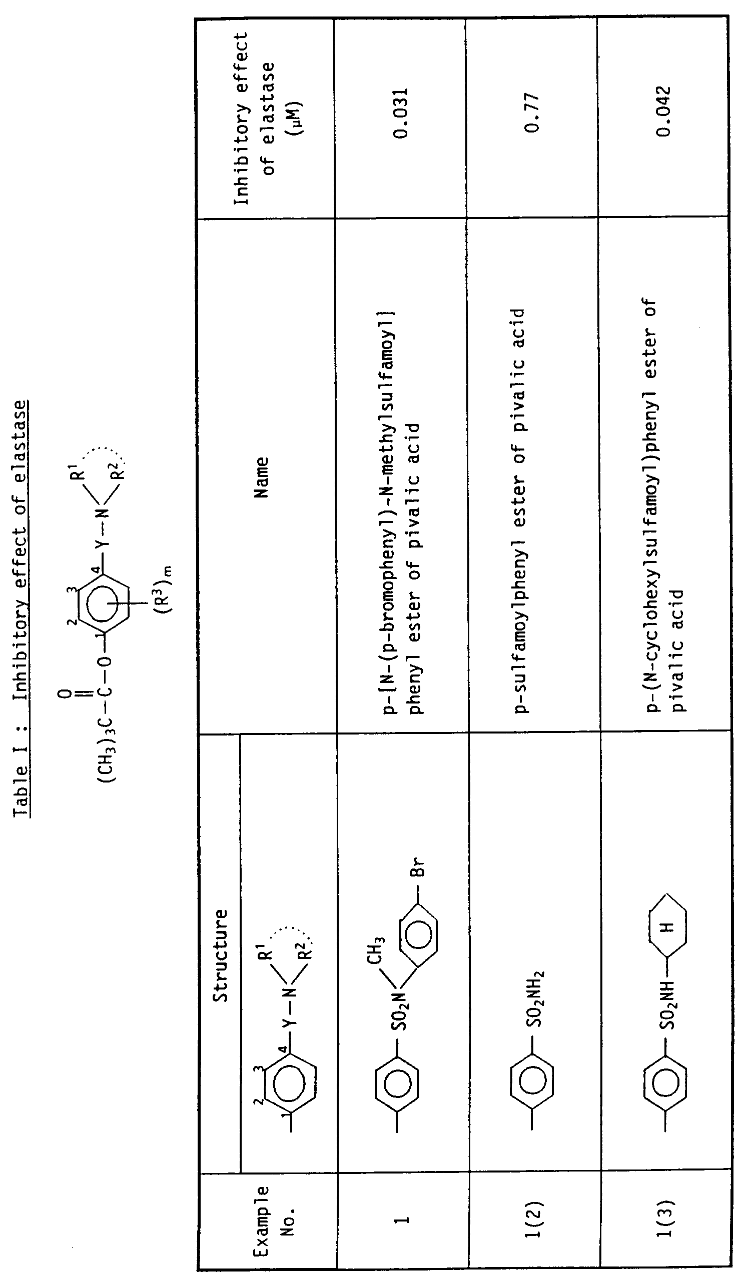

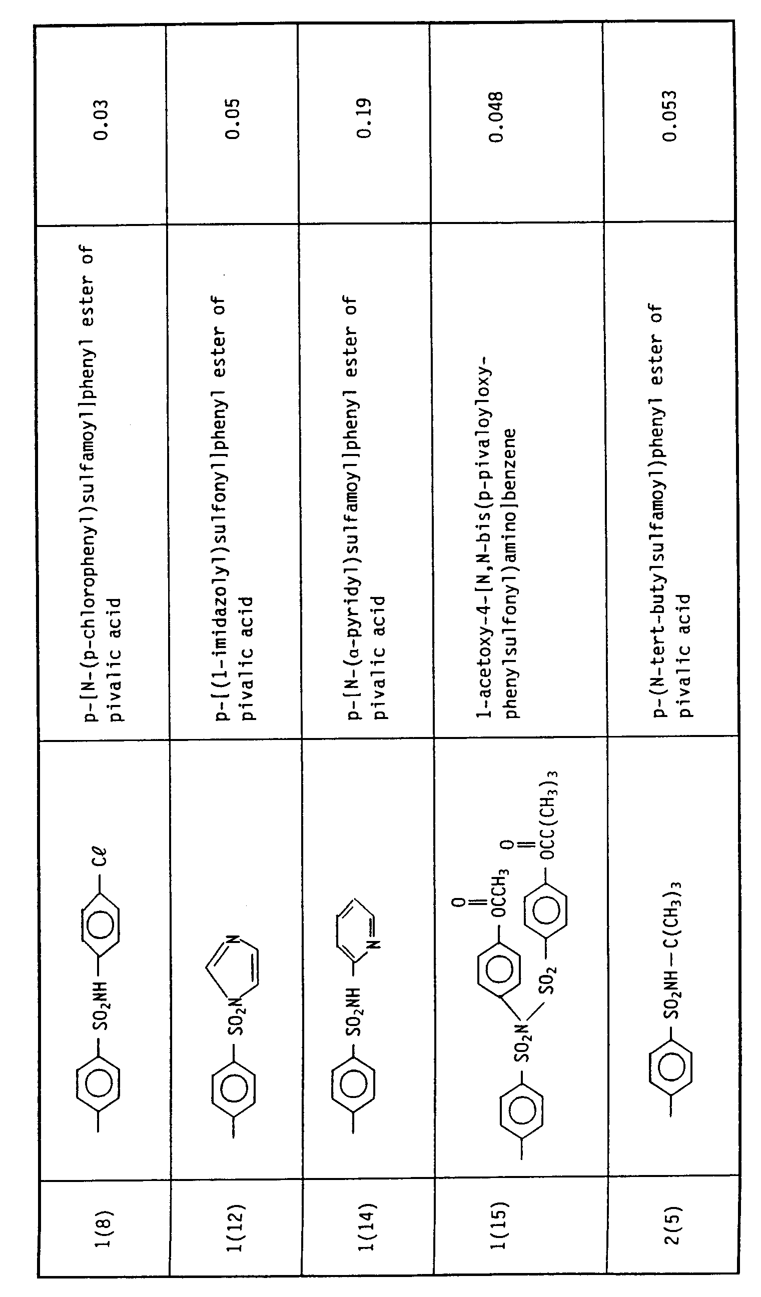

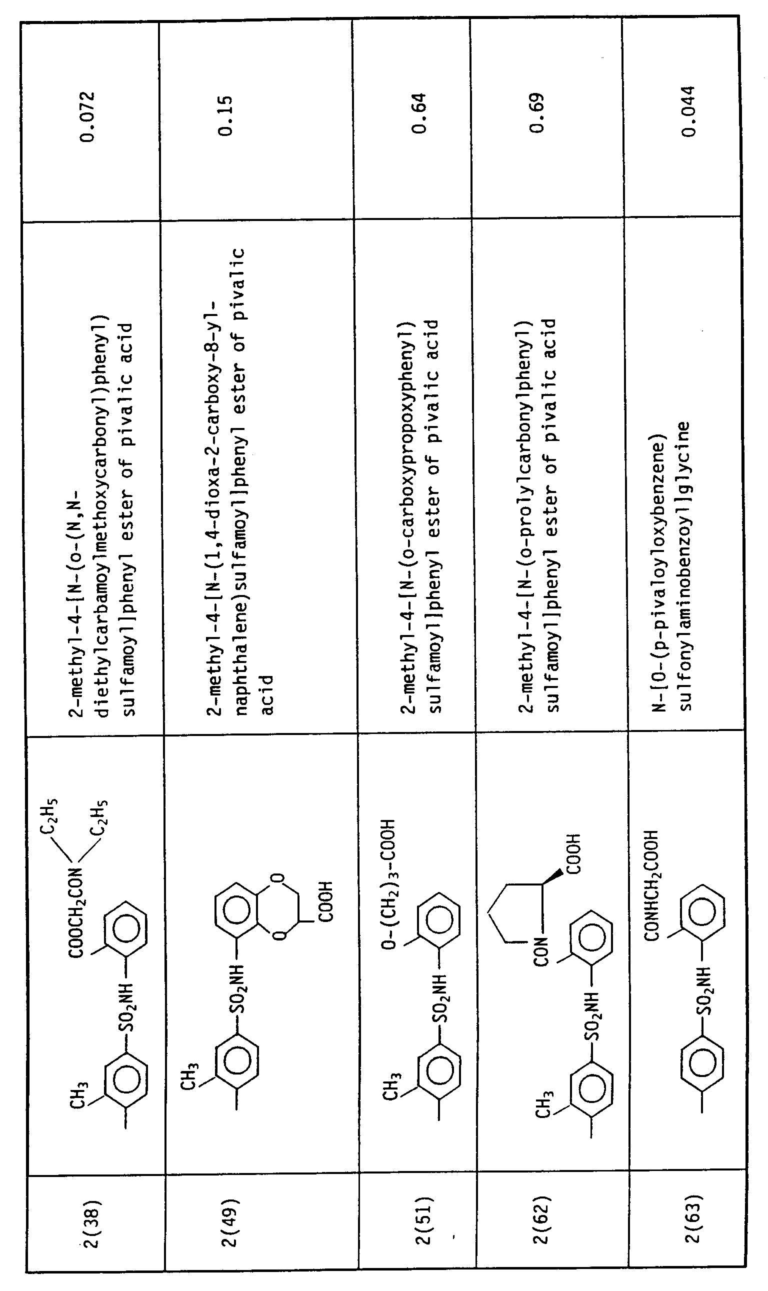

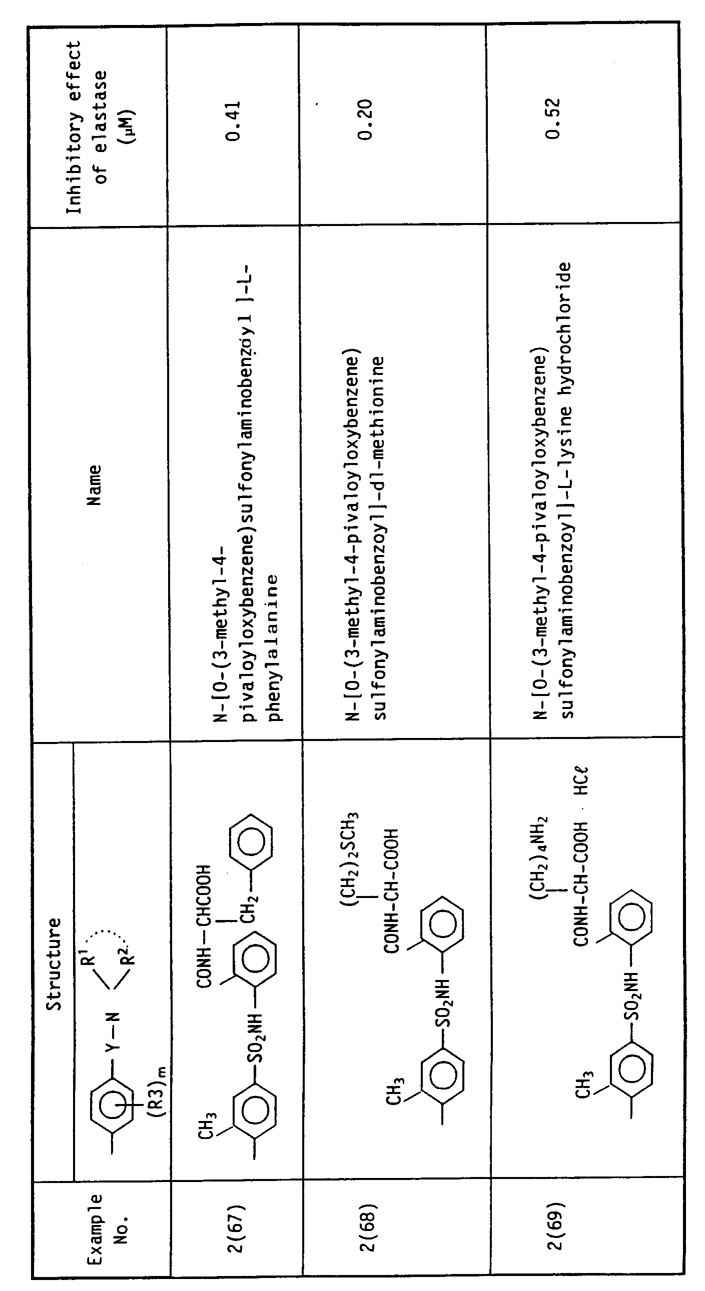

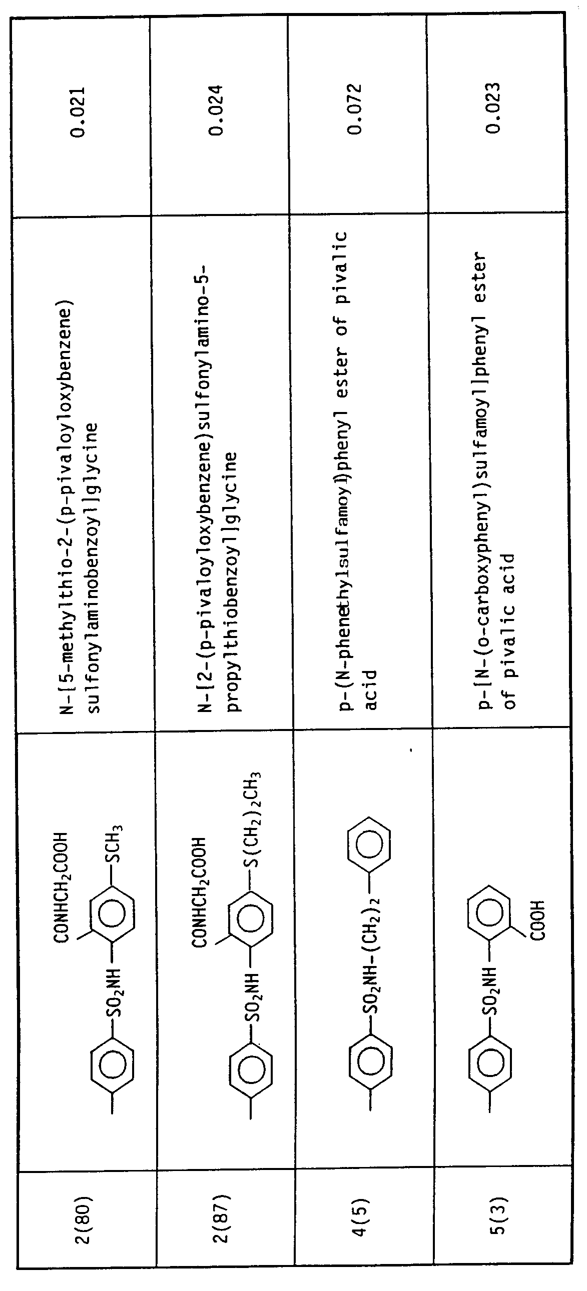

The results of the experiment showed that the compounds of the present invention have an inhibitory effect on elastase.

Further, it was confirmed that the toxicity of the compounds of the present invention is sufficiently low for them to be useful safely as pharmaceuticals.

Accordingly, it was confirmed that the compounds of the present invention can be useful for the treatment and/or prevention of diseases induced by abnormal enhancement of degradation of proteins such as elastin, by the action of elastase in mammals, especially in human beings.

For the purpose mentioned above, the compounds of general formula (I) or salts thereof will normally be administered systemically or partially, usually by oral or parenteral administration.

The dose to be administered is determined depending upon, for example, the age, body weight, symptom, desired therapeutic effect, route of administration, and the duration of the treatment. In the human adult, the doses per person are generally from 1 mg to 500 mg, by oral administration up to several times per day, and from 0.1 mg to 200 mg, by parenteral administration (preferably by intravenous administration) up to several times per day.

As mentioned above, the doses to be used depend on various conditions. Therefore, there are cases in which doses lower than the ranges specified above and doses greater than the ranges specified above, may be used.

Solid compositions according to the present invention for oral administration include compressed tablets, dispersible powders and granules. In such solid compositions, one or more of the active compound-(s) is, or are, admixed with at least one inert diluent such as lactose, mannitol, glucose, hydroxypropyl cellulose, microcrystalline cellulose, starch, polyvinyl-pyrrolidone or magnesium metasilicate aluminate. The compositions may also comprise, as is normal practice, additional substances other than inert diluents e.g. lubricating agents such as magnesium stearate, disintegrating agents such as cellulose calcium glycolate, stabilizing agents such as lactose, and solubilizers such as glutamic acid and asparaginic acid. The tablets or pills may, if desired, be made into gastric film-coated or enteric film-coated tablets or pills, such as sugar-coated, gelatin-coated, hydroxypropylcellulose-coated or hydroxypropylmethylcellulose phthalate- coated tablets or pills; two or more layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more of the active compound(s). Besides inert diluents such compositions may also comprise stabilizers such as sodium bisulfite and buffer for isotonicity, for example sodium chloride, sodium citrate or citric acid.

The manufacturing methods of spray compositions have been described in detail in, For example, the specifications of United States Patent No. 2868691 and No. 3095355.

Preparations for injection according to the present invention for parenteral administration include sterile aqueous or non-aqueous solutions, suspensions or emulsions. Example of aqueous solvents or suspending media are distilled water for injection and physiological salt solution. Examples of non-aqueous solvents or suspending media are propylene glycol, polyethylene glycol, vegetable oils such as olive oil, alcohols such as ehtanol, Polysorbate 80 (registered Trade Mark). These compositions may also include adjuvants such as preserving, wetting, emulsifying, dispersing and stabilizing agents (e.g. lactose) and solubilizers (e.g. glutamic acid and asparaginic acid). They may be sterilized, for example, by filtration through a bacteria- retaining filter, by incorporation of sterilizing agents in the compositions or by irradiation. They may also be manufactured in the form of sterile solid compositions which can be dissolved in sterile water or some other sterile injectable medium immediately before use.

Other compositions for parenteral administration include liquids for external use, and endermic liniments such as ointments, suppositories for rectal administration and pessaries for vaginal administration which comprise one or more of the active compound(s) and may be prepared by known methods.

The following Reference Examples and Examples illustrate the preparation of compounds of the present invention. In the Reference Examples and Examples, "TLC" "NMR" and "IR" each represents "thin layer chromatography", "nuclear magnetic resonance" and "infrared absorption spectrum".

The solvents in the parentheses show the developing or eluting solvents and the ratios of the solvents used are by volume in chromatographic separation.
Unless otherwise specified, "IR" were measured by the KBr method, and "NMR" were measured in deuterochloroform (CDC1₃).

### Reference Example 1

### 1-(N-methyl-N-phenyl)sulfamoyl-4-methoxybenzene

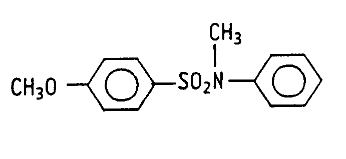

p-methoxybenzenesulfonyl chloride (965 mg) was dissolved in the mixture of triethylamine (2 ml), methylaniline (500 mg) and methylene chloride (10 ml) under cooling with ice, and the mixture was stirred for 30 minutes.

The reaction solution was stirred overnight at room temperature. After the reaction was finished, the reaction solution was extracted with ether. The extract was washed with successive, 1N-HCR, water and saturated an aqueous solution of sodium chloride.

The solution was dried over sodium sulfate, and distilled off under reduced pressure to give the title compounds.

### Reference Example 2

### p-[N-methyl-N-(p-bromophenyl)sulfamoyl]phenol

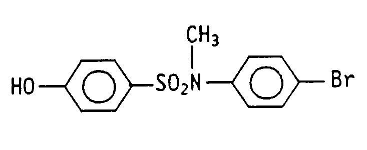

Boron tribromide (2.2 ml) was added to the solution of methylene chloride (10 ml) of the compound obtained by Referene Example 1 under cooling with ice, and stirred for 2 hours at room temperature.

The reaction solution was allowed to cool to -20°C ~ -30 °C, and water was added thereto, and the solution obtained was extracted with ethyl acetate. The extract was washed with successive, water and saturated aqueous solution of sodium chloride.

The solution was dried over sodium sulfate, and distilled off under reduced pressure and the residue was purified by the column chromatography on silica-gel (methylene chloride: ehtyl acetate = 10:1) to give the title compound (900 mg) having the following physical data;

TLC : Rf 0.20 (methylene chloride: ethyl acetate = 30:1).

### Reference Example 3

### p-[N-[(p-tolyl)carbamoyl]phenol

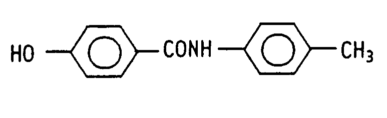

Potassium carbonate (500 mg) was added into methanol solution (50 ml) of [p-acetoxy-N-(p-tolyl)]-benzamide (300 mg) obtained by the same procedure as Reference Example 1, and the mixture was stirred overnight.

The obtained reaction solution was distilled off under reduced pressure, and extracted with ethyl acetate. The extract was washed with successive, 1 N-hydroic acid, water and saturated aqueous solution of sodium chloride.

The solution was dried with magnesium sulfate, and distilled off under reduced pressure to give the title compound having the following physical data.

TLC : Rf 0.31 (methylene chloride : ethyl acetate = 10:1).

### Example 1

### p-[N-(p-bromophenyl)-N-methylsulfamoyl]phenyl ester of pivalic acid

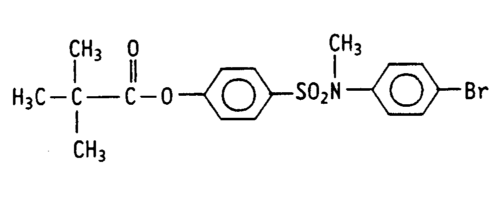

Pivaloyl chloride (0.5 ml) was added to the mixture solution of triethylamine (1.5 ml)-methylene chloride (5 ml) of the compounds obtained by procedure of Reference Example 2 under cooling with ice. The reaction solution was allowed to stand for 10 minutes, and stirred for one hour at room temperature.

The reaction solution was extracted with ether, and the extract was washed with successive, water, 1 N-HCî, water, saturated aqueous solution of sodium bicarbonate, water and saturated aqueous solution of sodium chloride. The solution was dried with sodium sulfate, and distilled off under reduced pressure.

The concentrate was recrystalized with ethyl acetate-hexane to give the title compound (510 mg) having the following physical data.

TLC : Rf 0.81 (methylene chloride : ethyl acetate = 30:1);

IR : 1750, 1590, 1460, 1400, 1350 cm-¹.

The compounds, described in the following Tables II and III, were obtained by using corresponding starting materials and by the same procedure as Reference Example 1 - Reference Example 2 (or Reference Example 3) - Example 1.

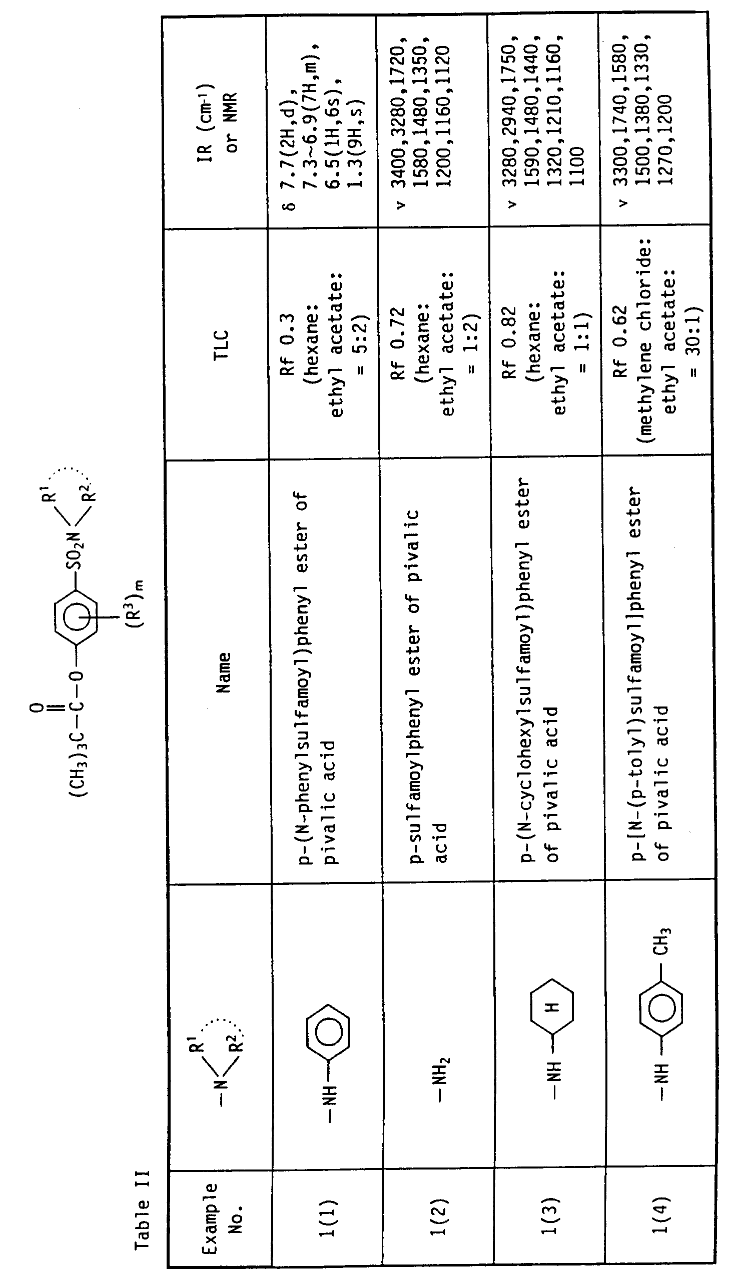

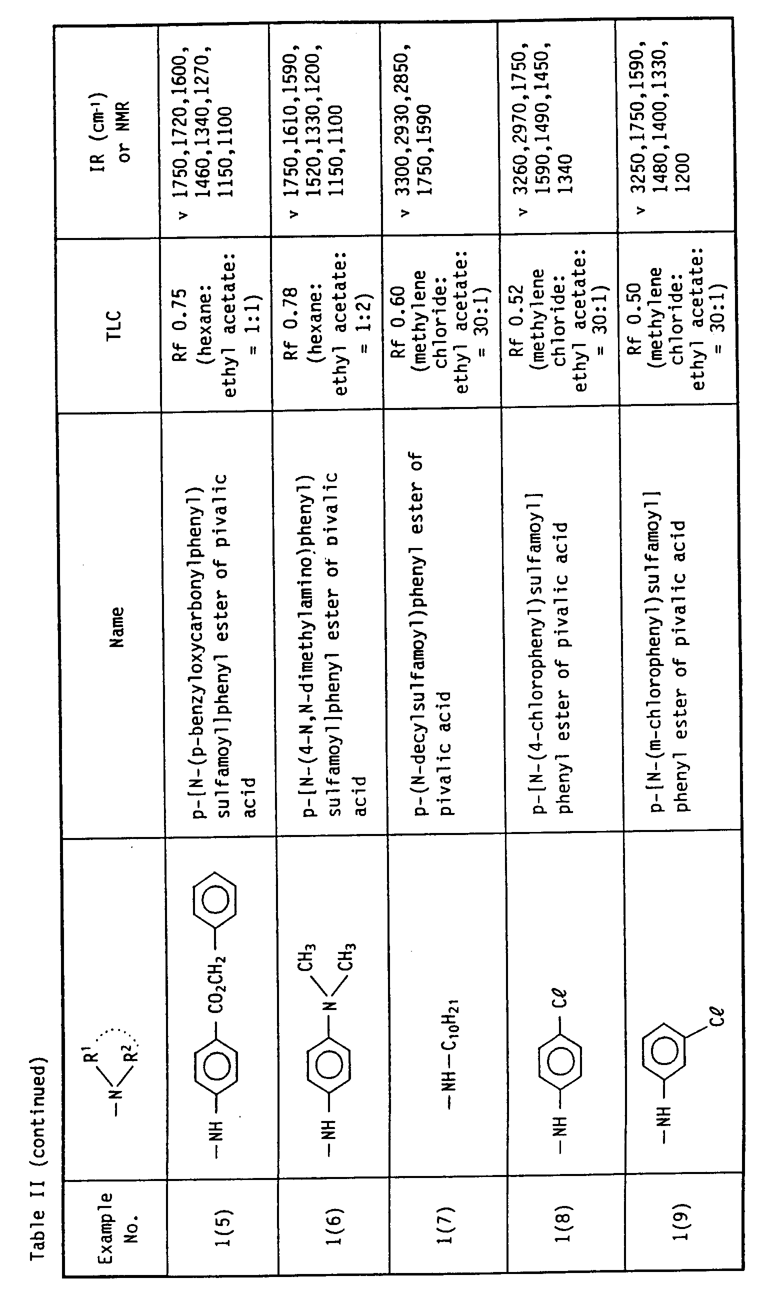

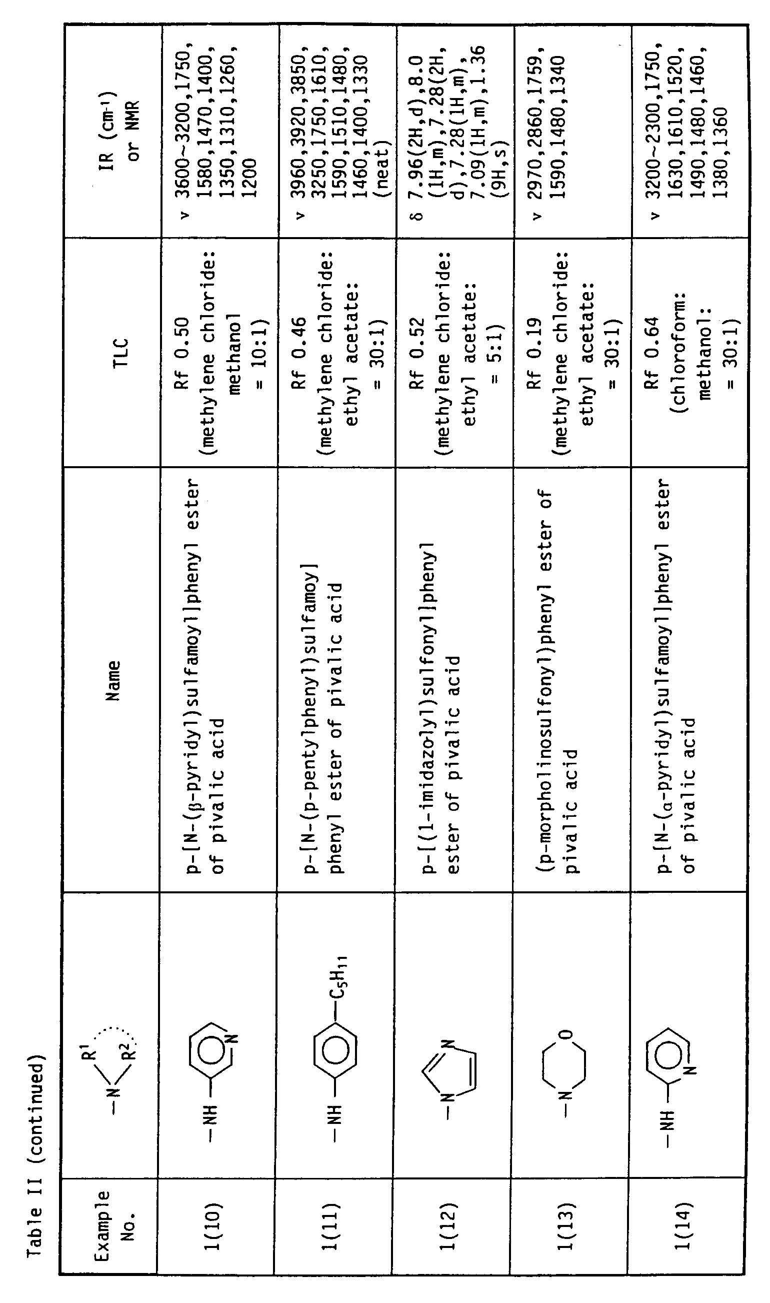

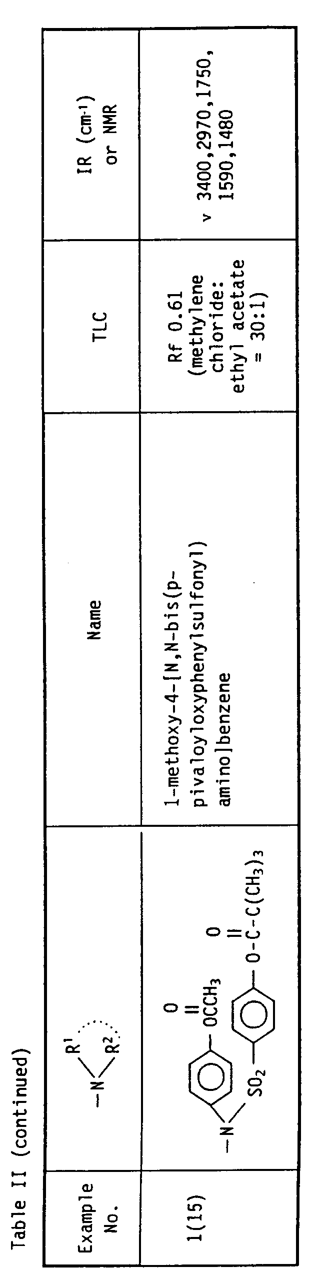

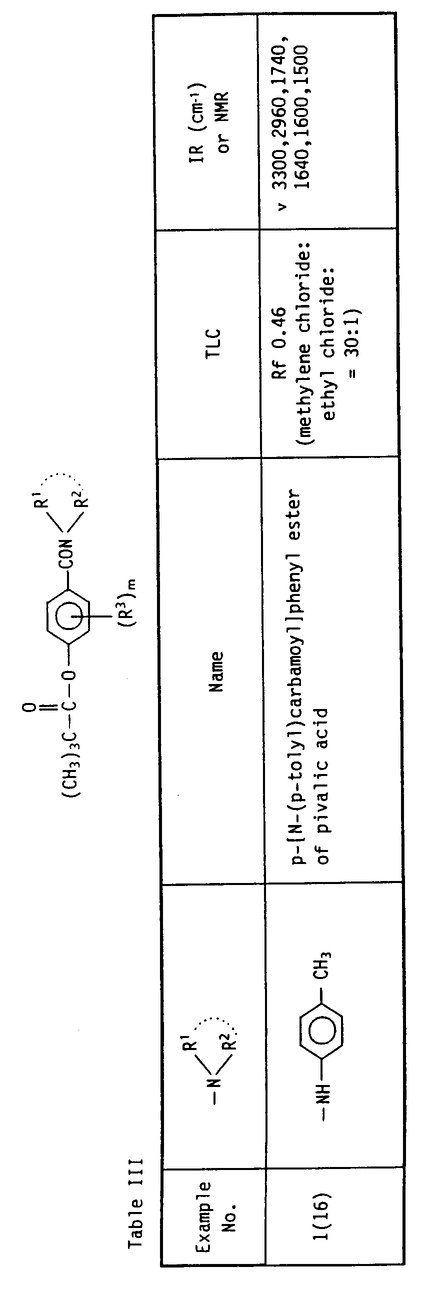

Reference Example 4

### sodium salt of p-pivaloyloxybenzenesulfonic acid

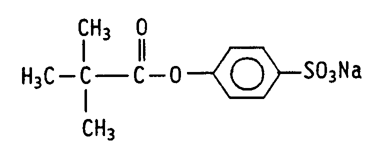

Pivaloyl chloride (2.4 g) was dissolved in the mixture of 4N-aqueous solution of sodium hydroxide (7.5 ml) of phenol 4-sulfonic acid (1.74 g) and tetrahydrofuran (5 ml), and the mixture was stirred for 10 minutes under cooling with ice. The mixture was reacted for one hour at room temperature.

The reaction solution was distilled off under reduced pressure, and the crystal was filtered off.

And obtained crystal was washed twice with small amount of ice-water, and dried to give the title compound (1.26 g) having the following physical data.

TLC : Rf 0.65 (ethyl acetate : acetic acid : water = 6 : 2 : 1).

### Reference Example 5

### p-pivaloyloxybenzenesulfonyl chloride

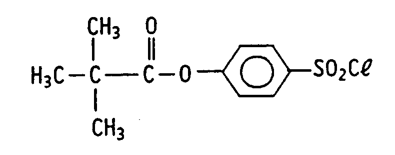

Thionyl chloride (2.1 ml) was added to dimethylformamide solution (33 ml) of the compound (2.8 g) of Reference Example 4, and the mixture was stirred for 30 minutes under cooling with ice, and stirred for 30 minutes at room temperature.

The reaction solution was extracted with ether-hexane (1:1), and the extract was washed twice with ice-water.

The solution was dried with magnesium sulfate to give the title compound (2.49 g) having the following physical data.

TLC : Rf 0.34 (hexane : ethyl acetate = 10 : 1).

### Example 2

### p-[N((trans-p-carboxycyclohexyl)methyl)sulfamoyl]phenyl ester of pivalic acid

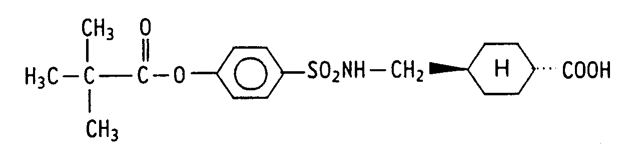

By using the sulfonyl chloride of Reference Example 5 and the corresponding amine and the same procedure as Reference Example 1, the title compound (110 mg) having the following physical data was obtained.

TLC : Rf 0.32 (chloroform : methanol : acetic acid = 100 : 5 : 1);

NMR : 7.9(2H,d), 7.25(2H,d), 4.4(1 H,m), 2.8(2H,m), 2.4-1.0(9H,m), 1.35(9H,s).

Hereinafter, by using sulfonyl chloride of Reference Example 5 and corresponding amine, and by the same procedure of Example 2, the desired compounds described in the following Table IV and V were obtained.

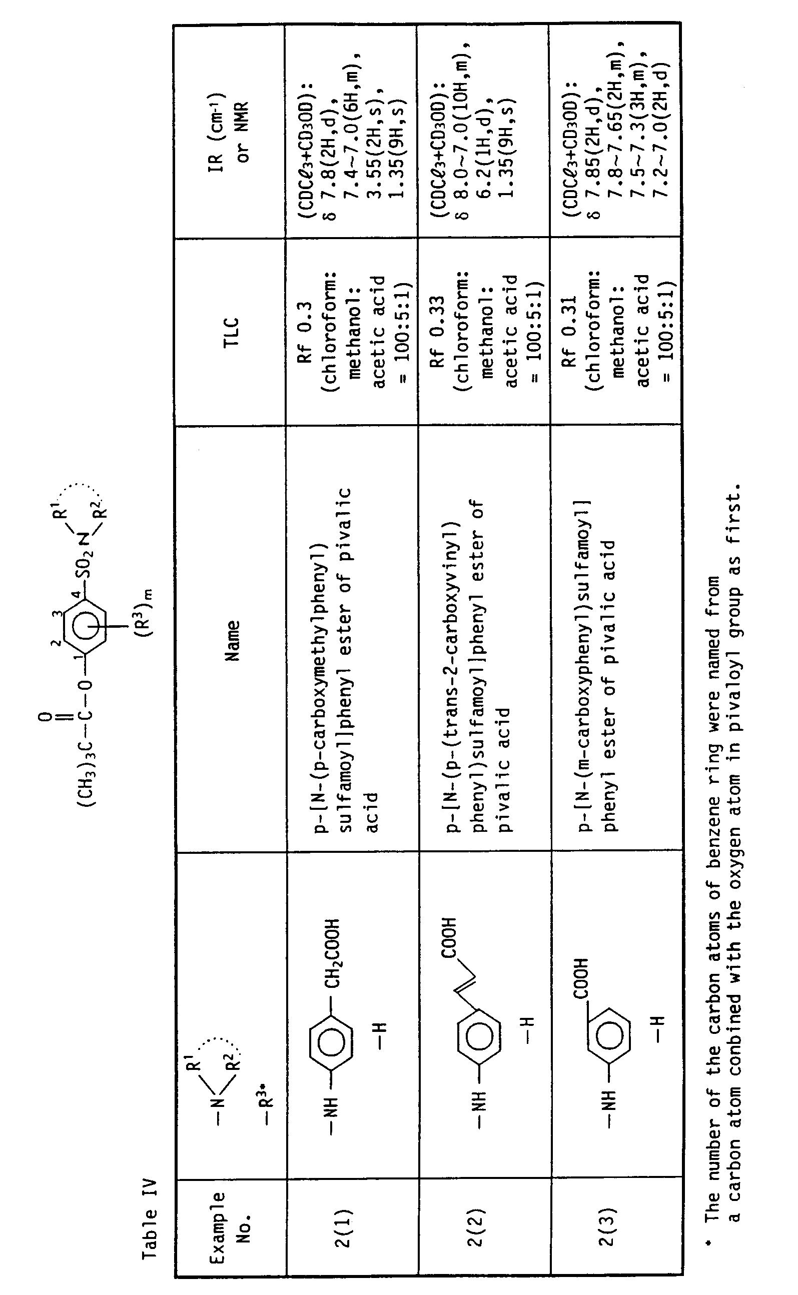

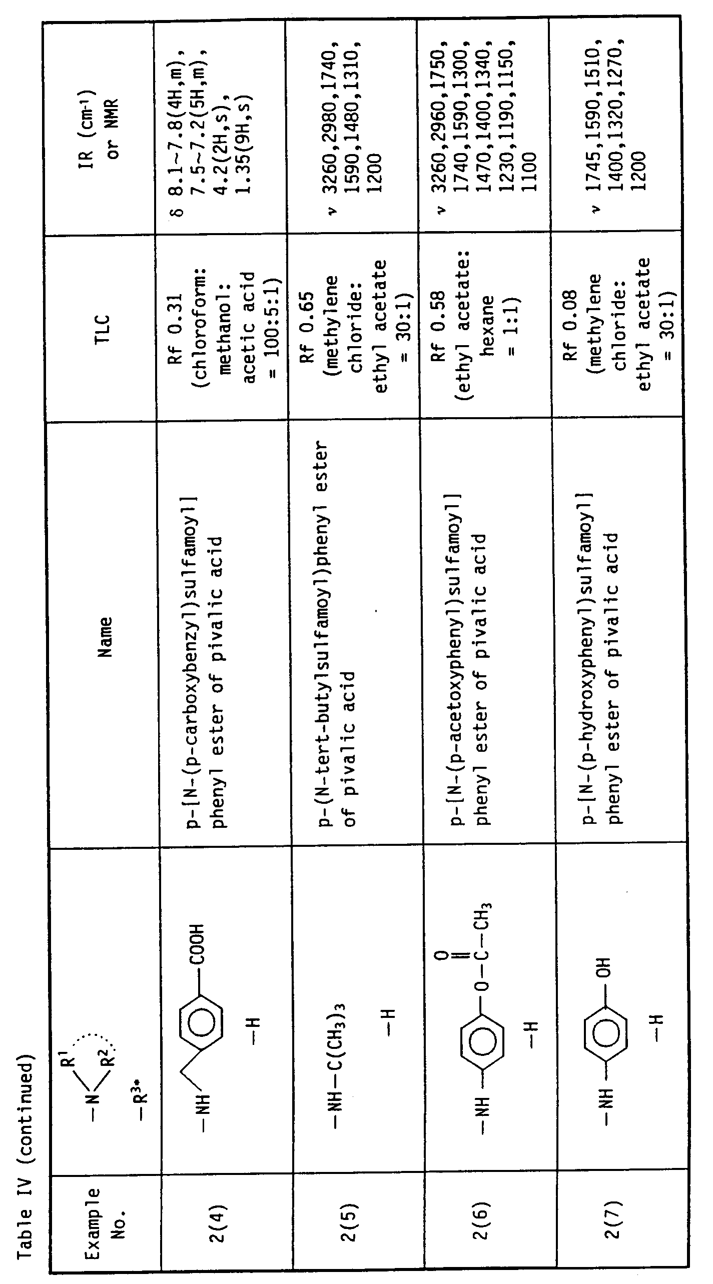

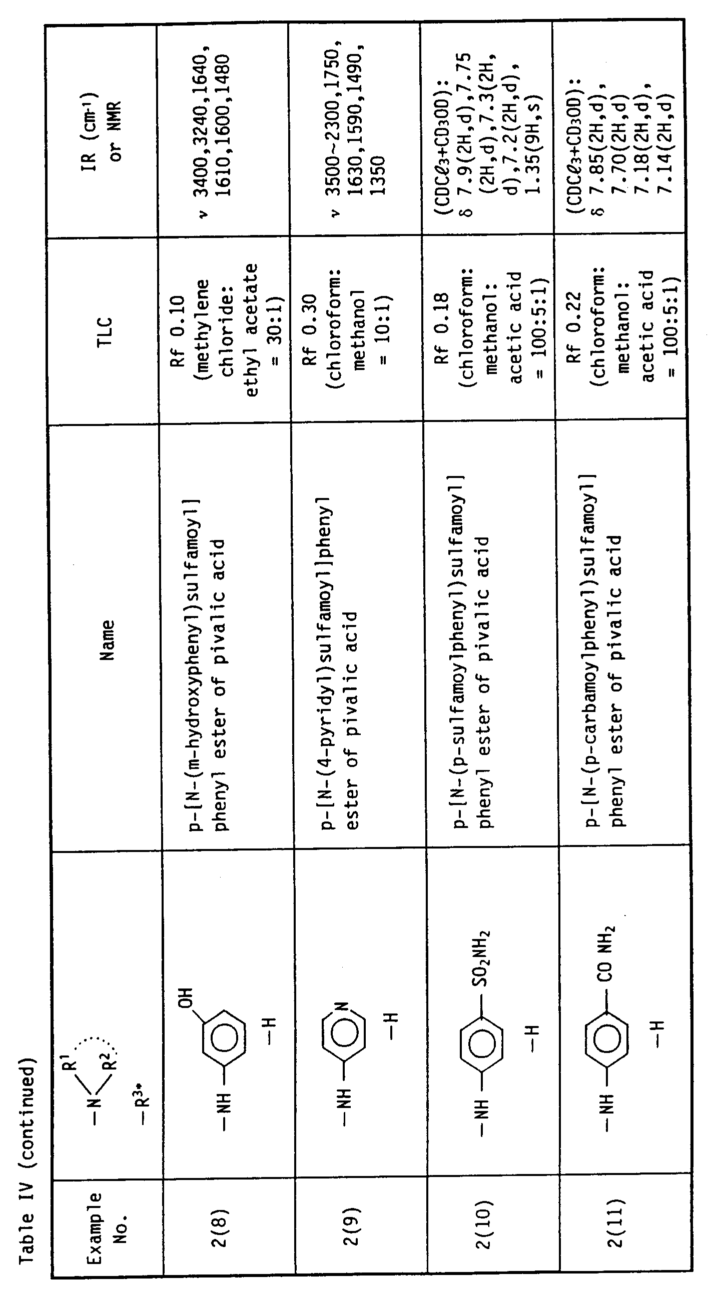

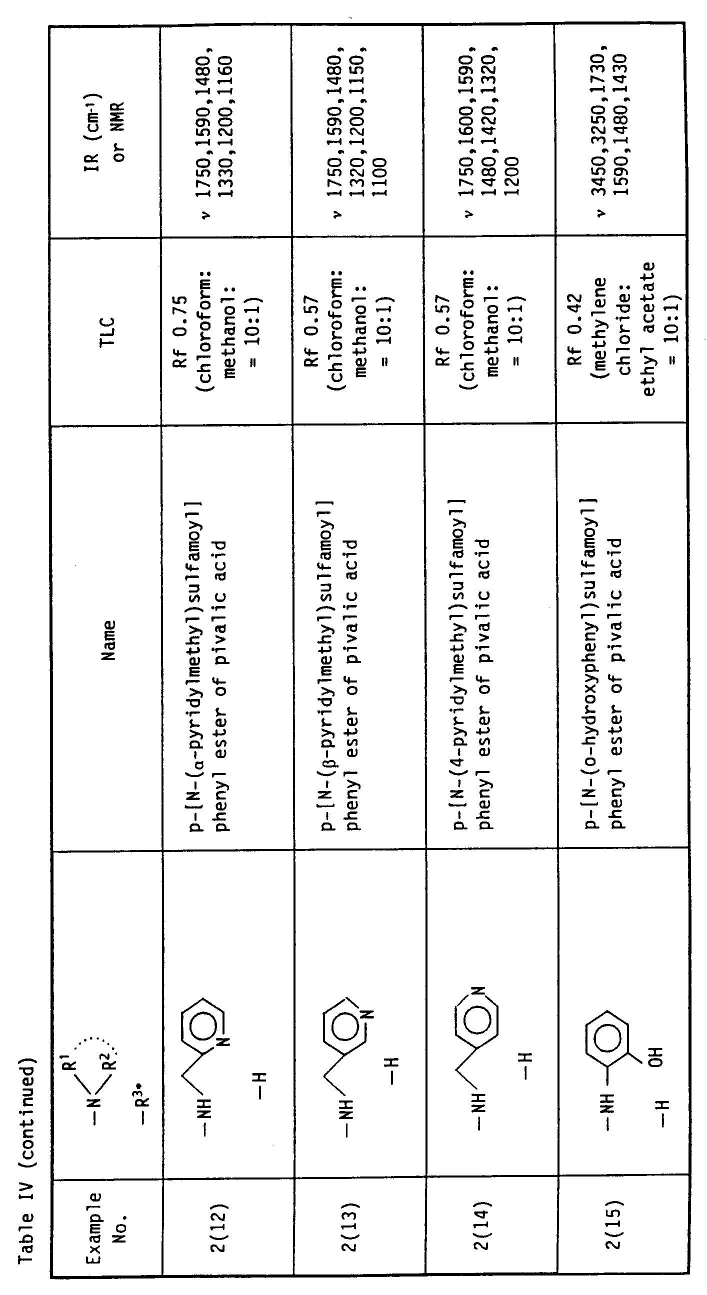

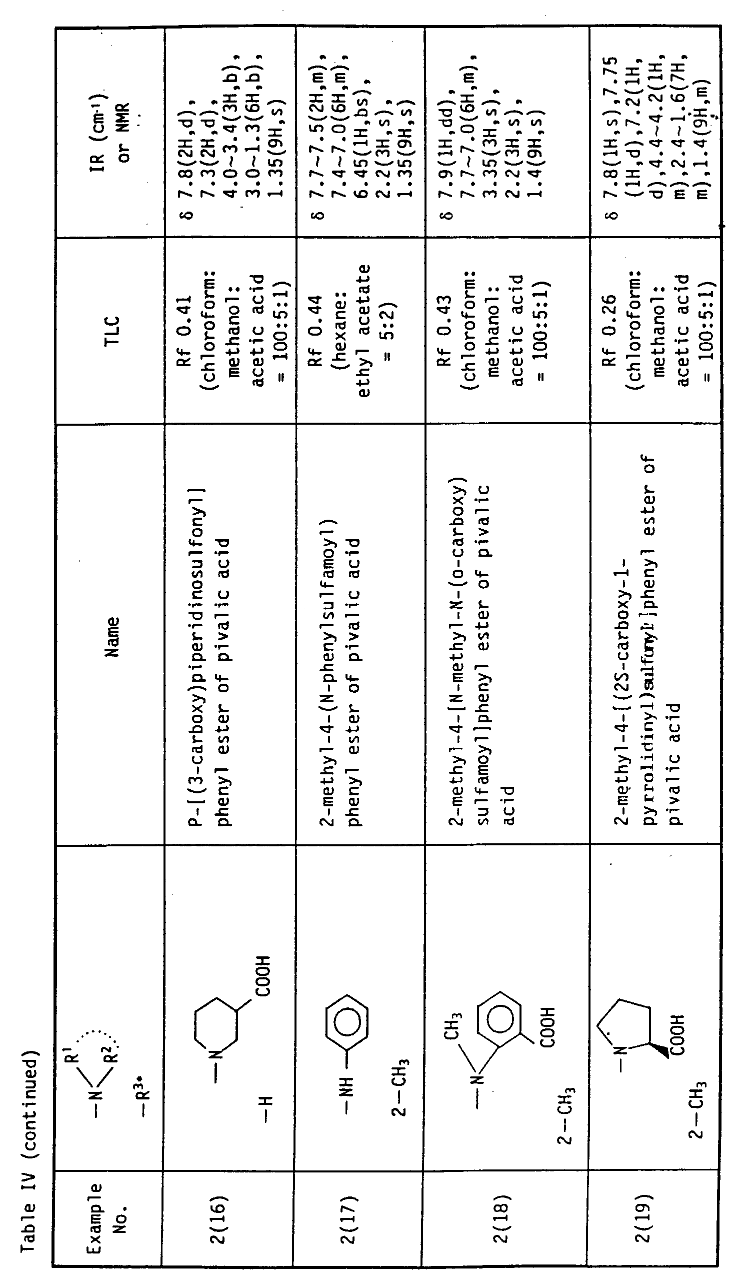

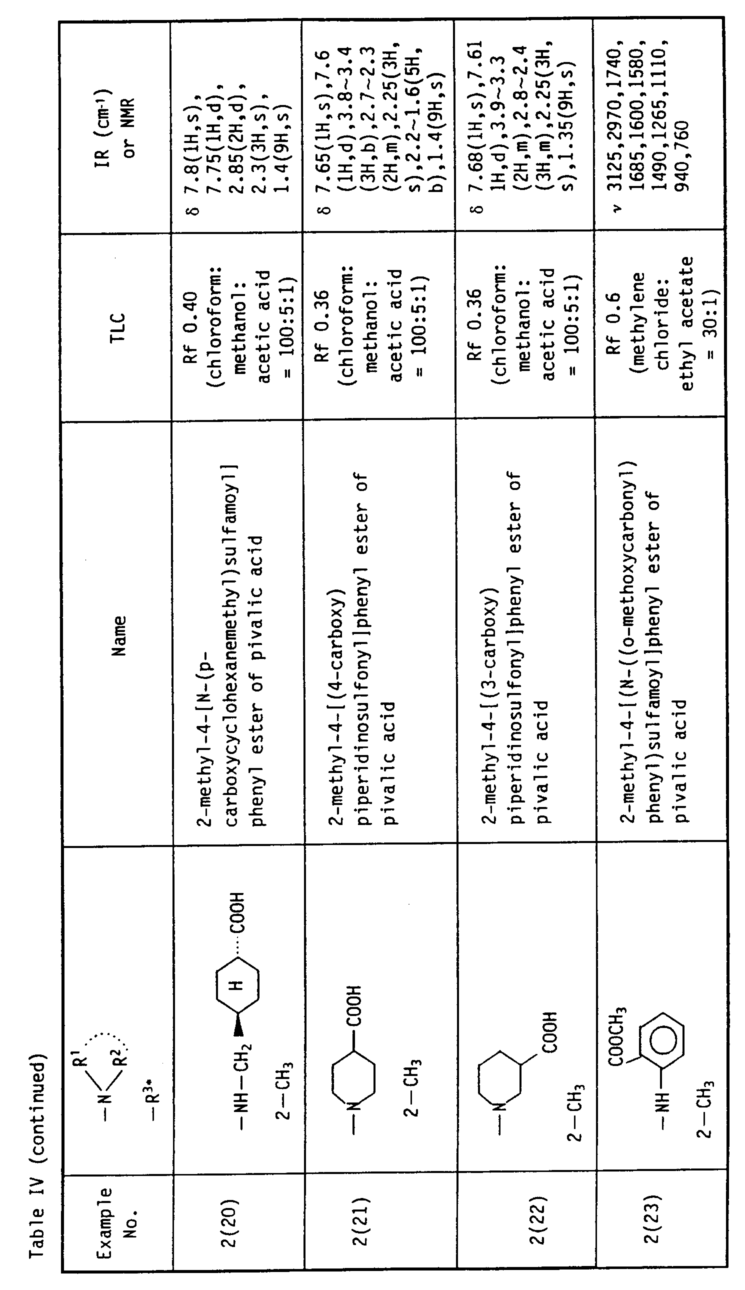

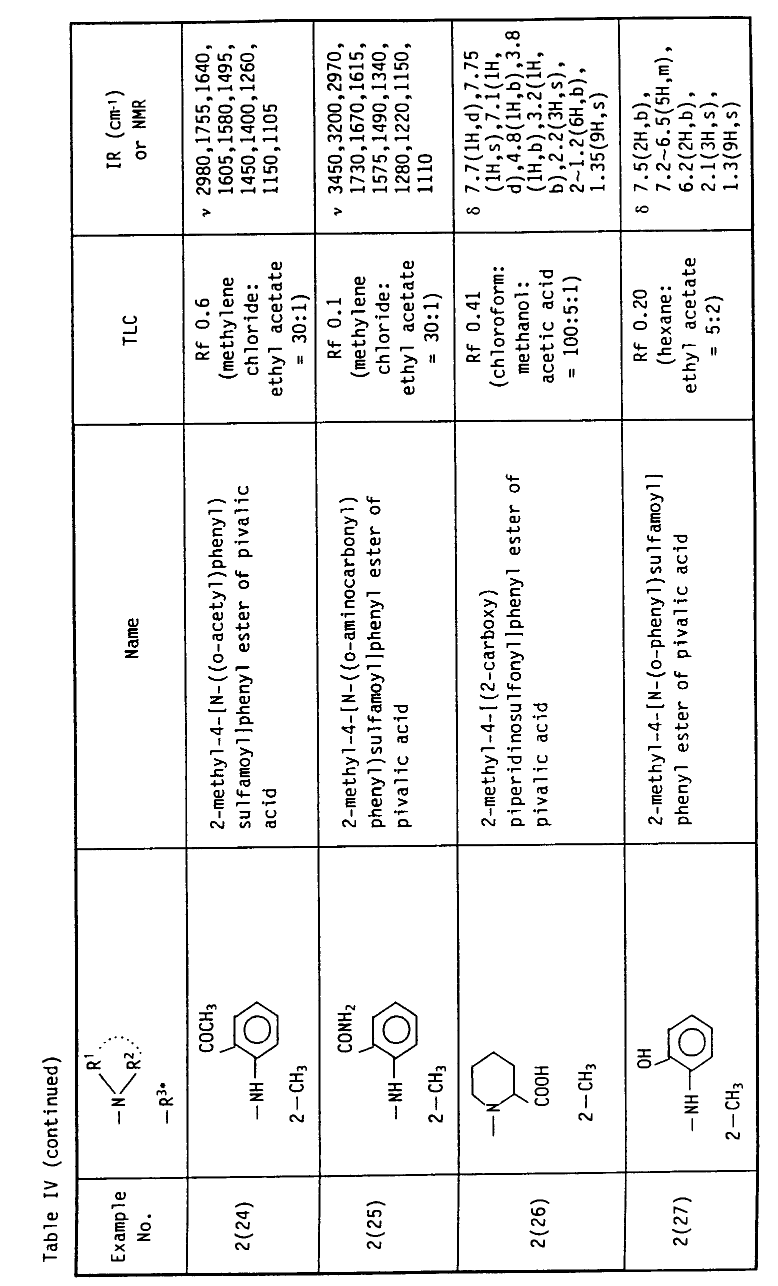

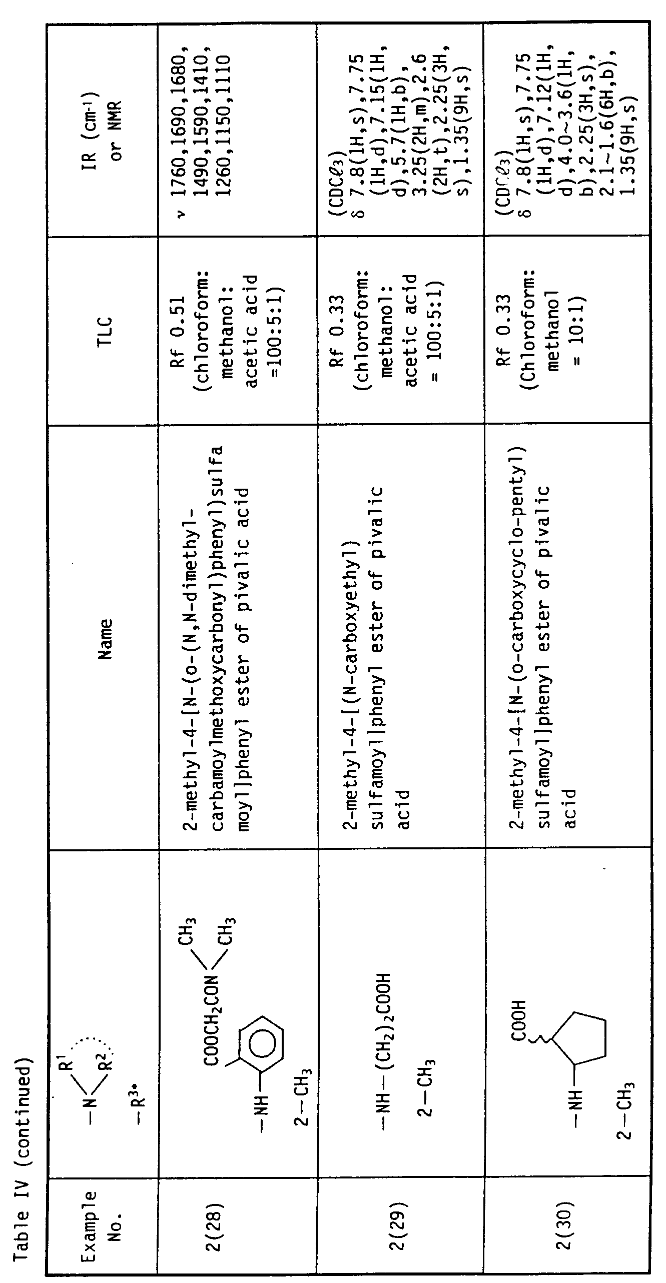

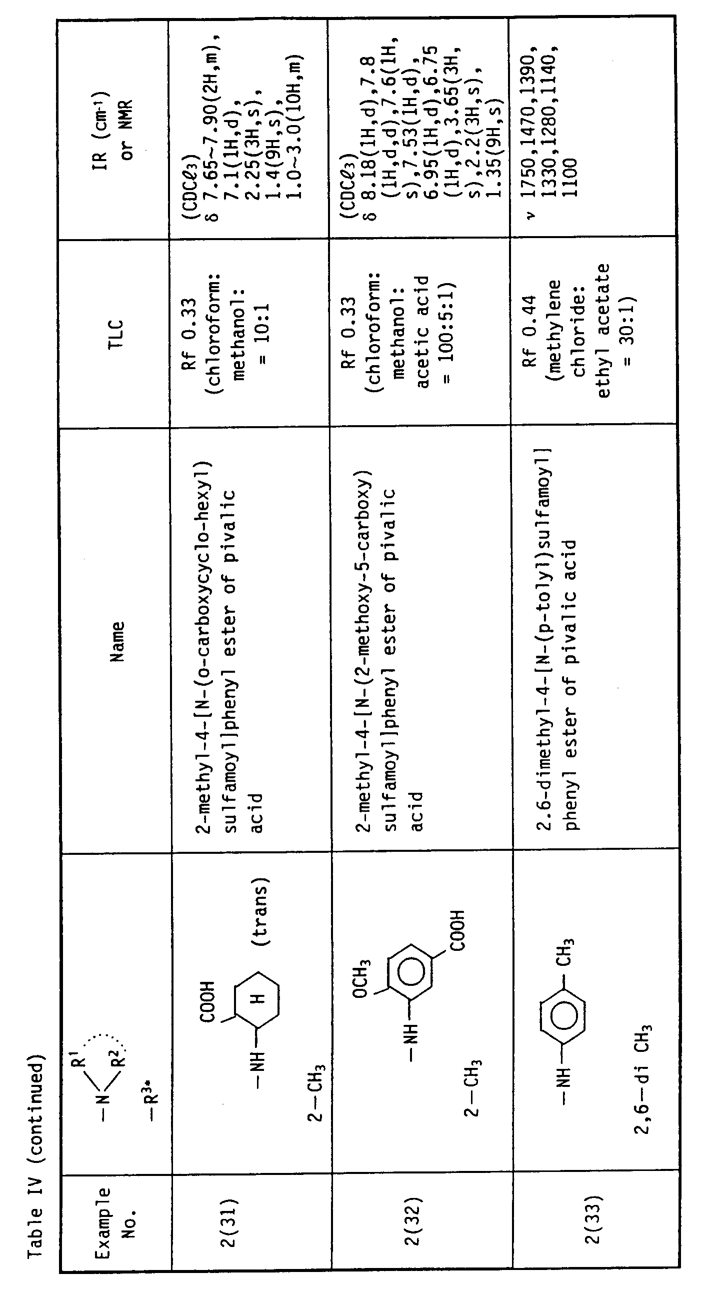

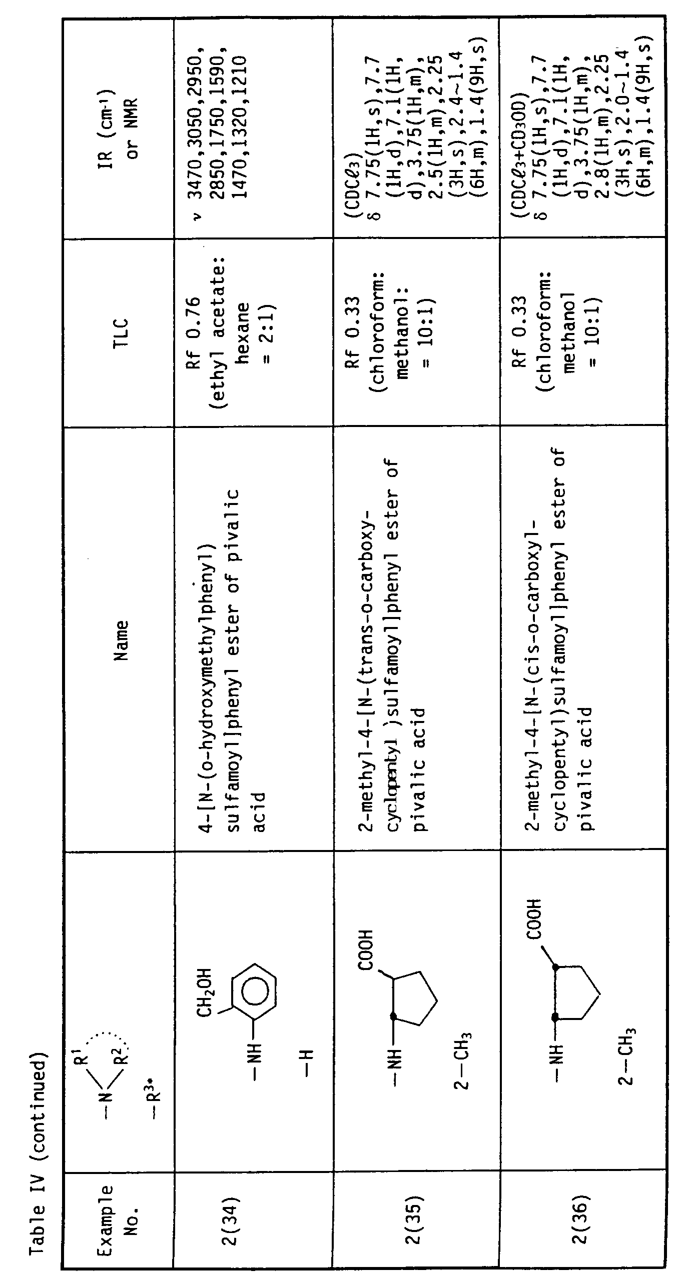

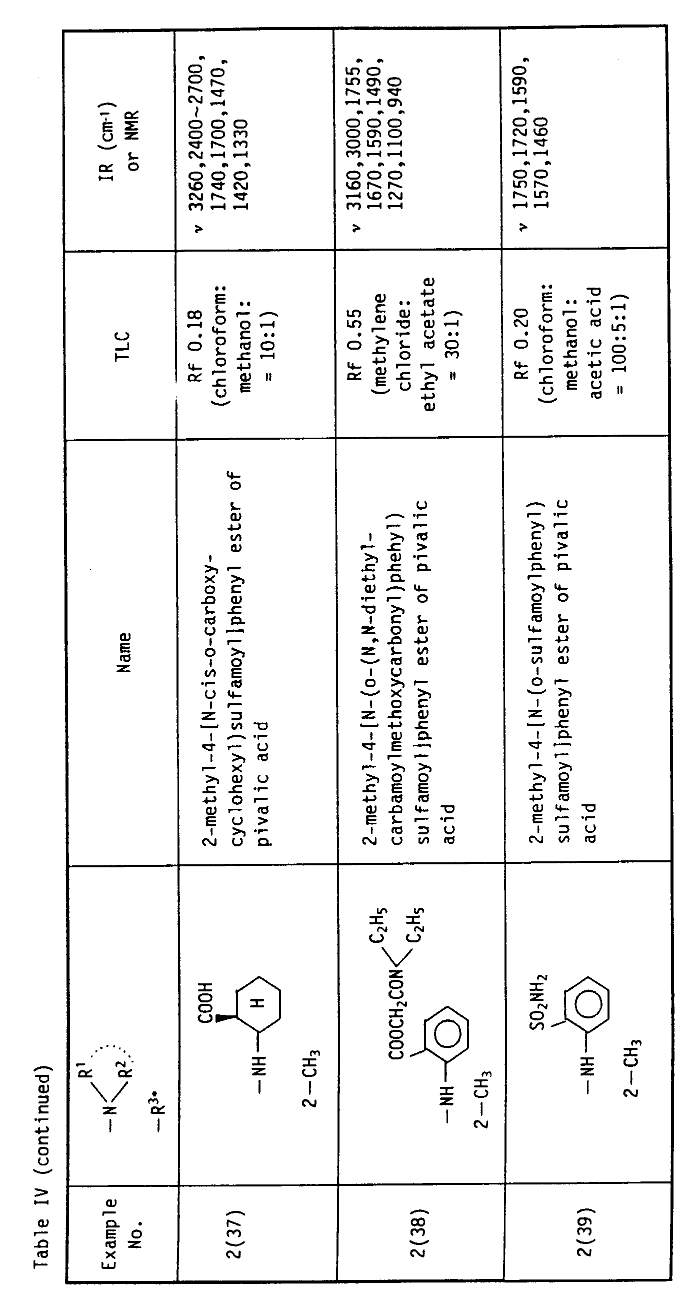

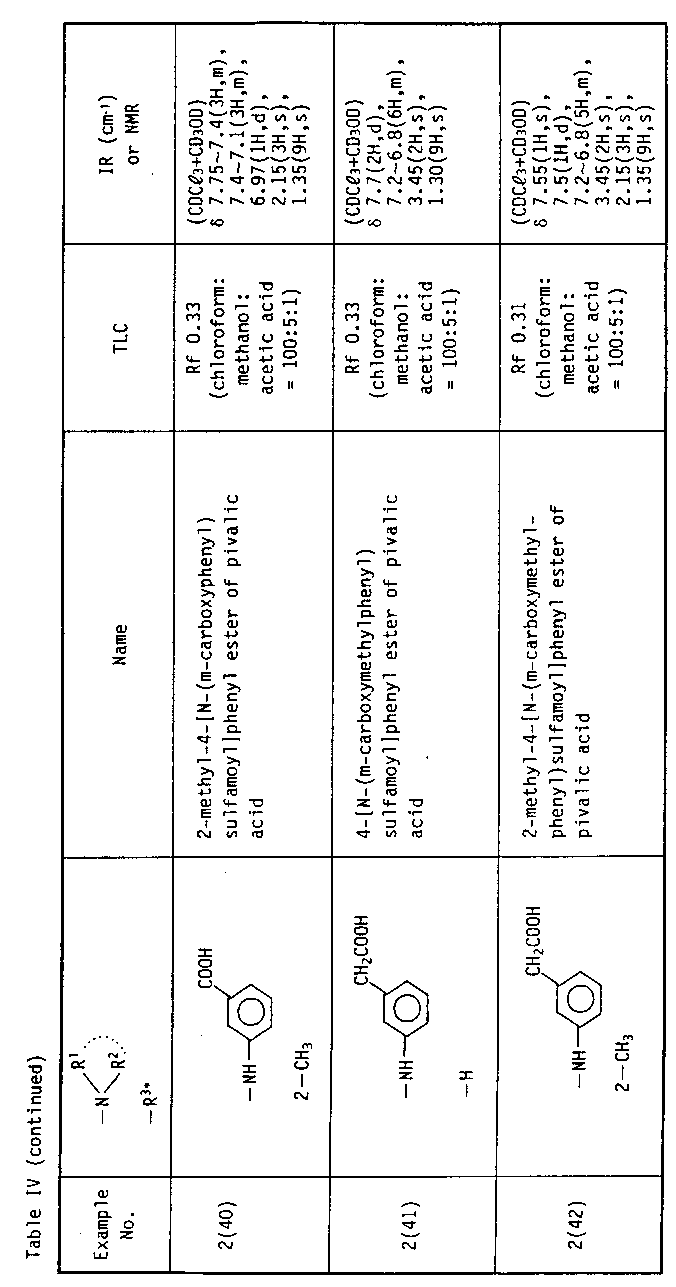

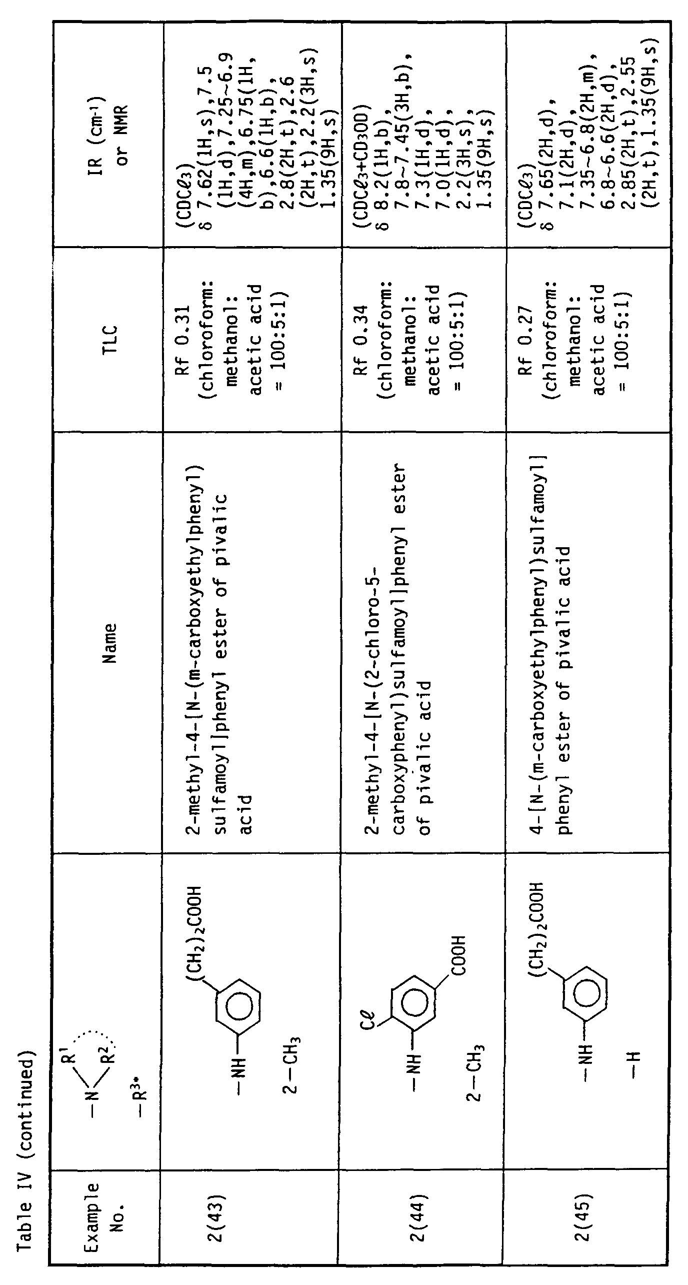

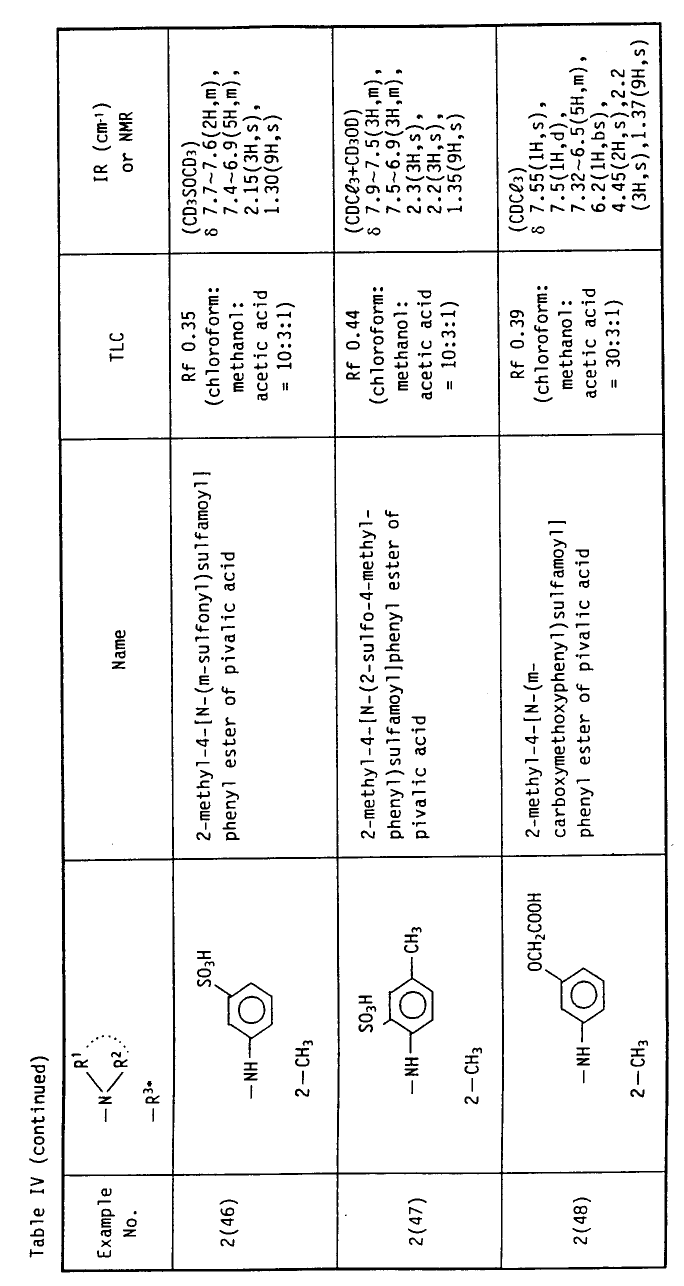

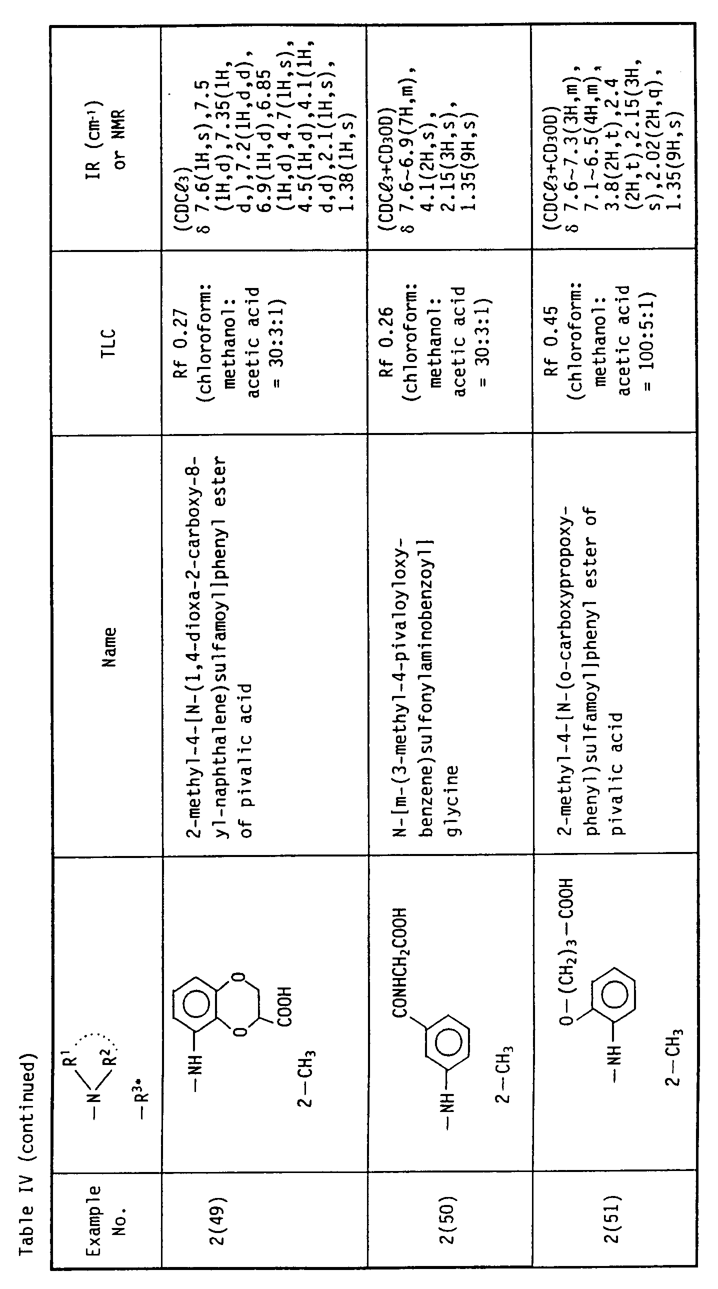

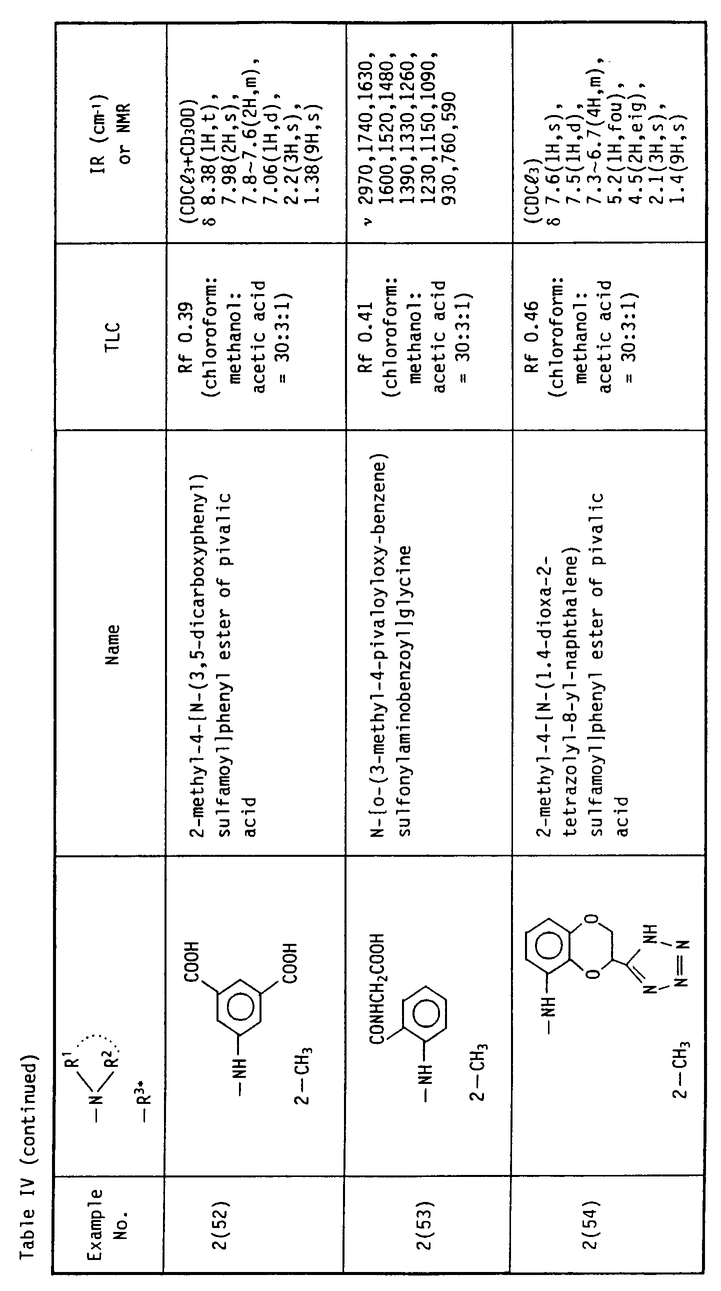

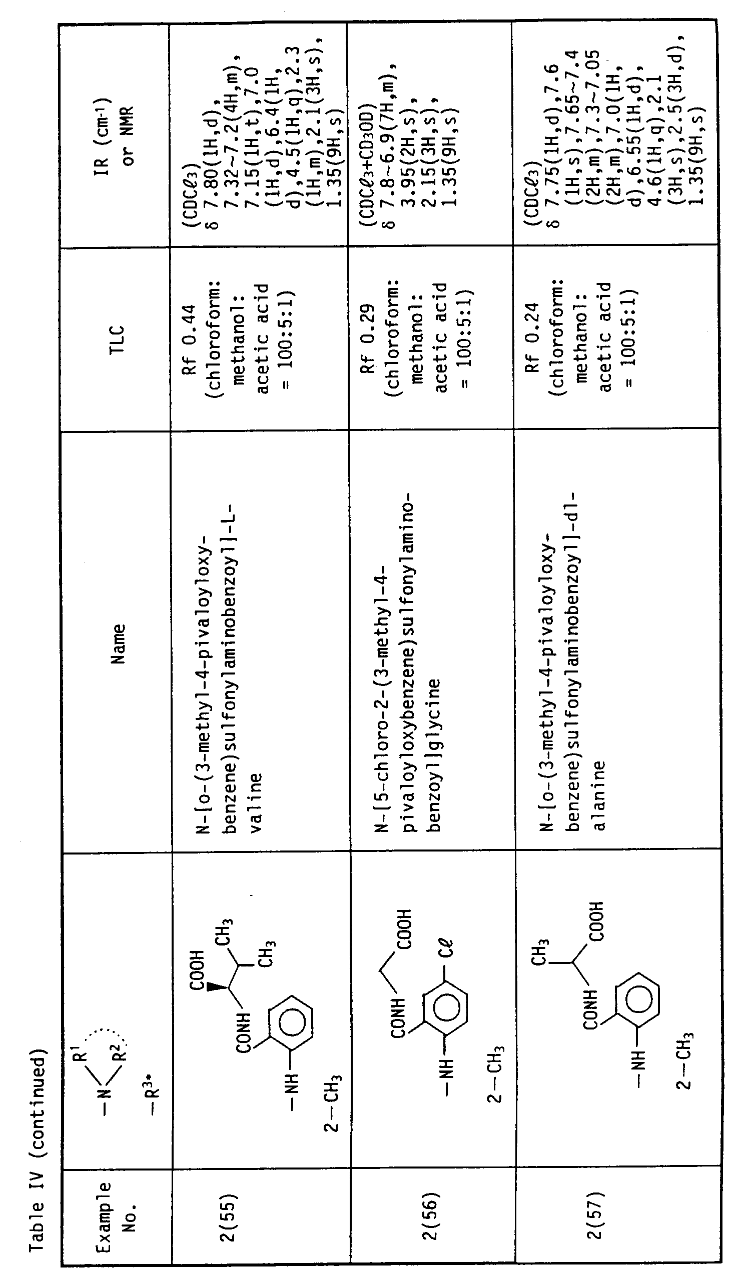

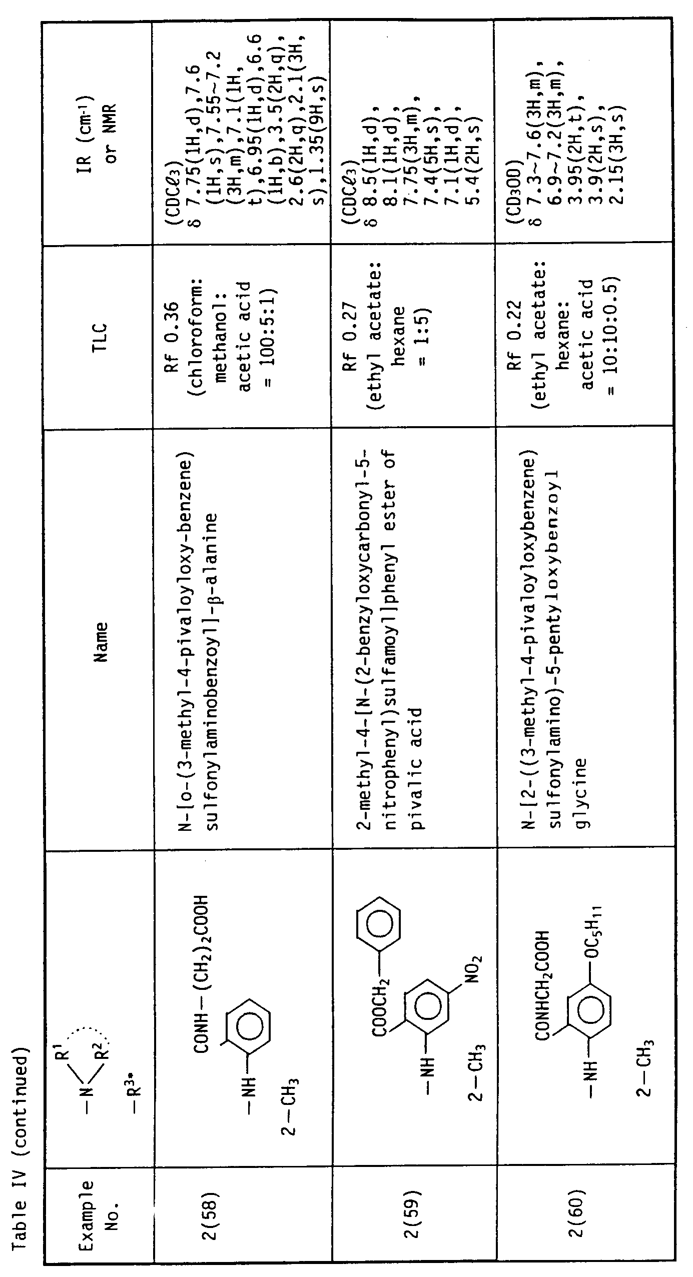

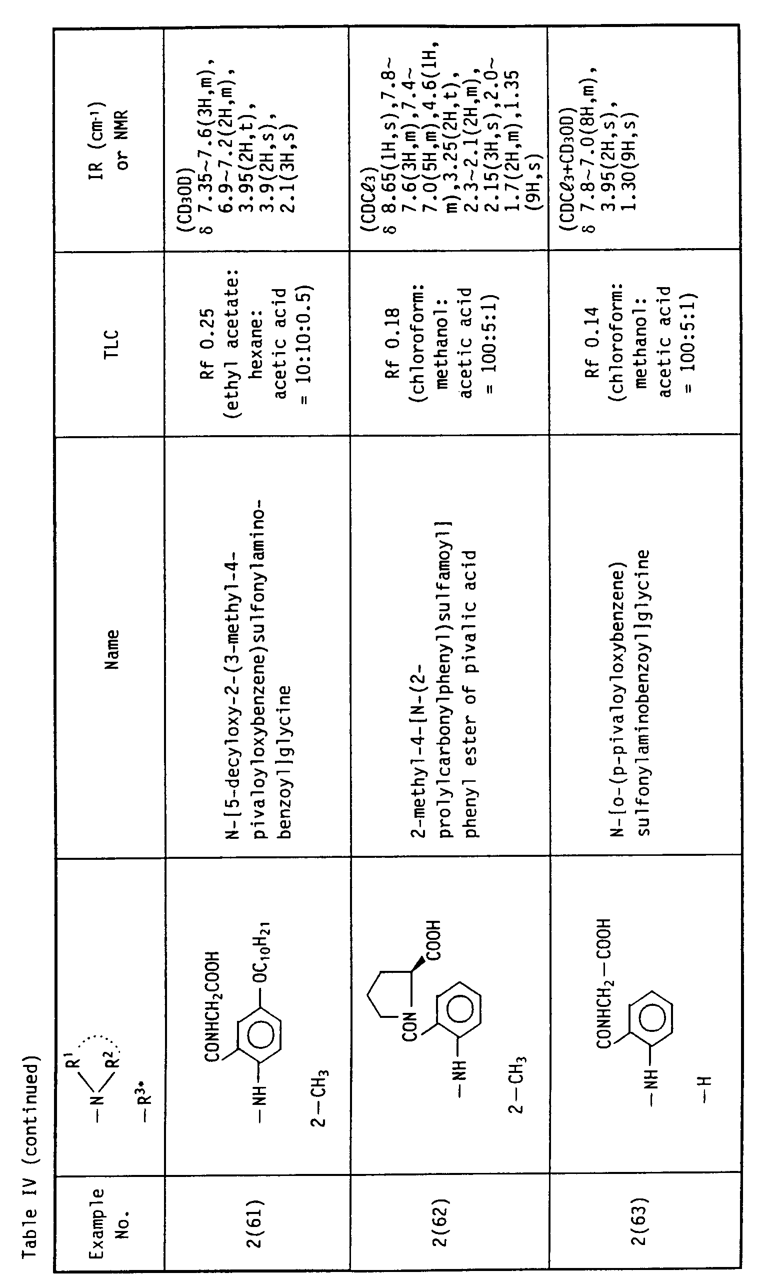

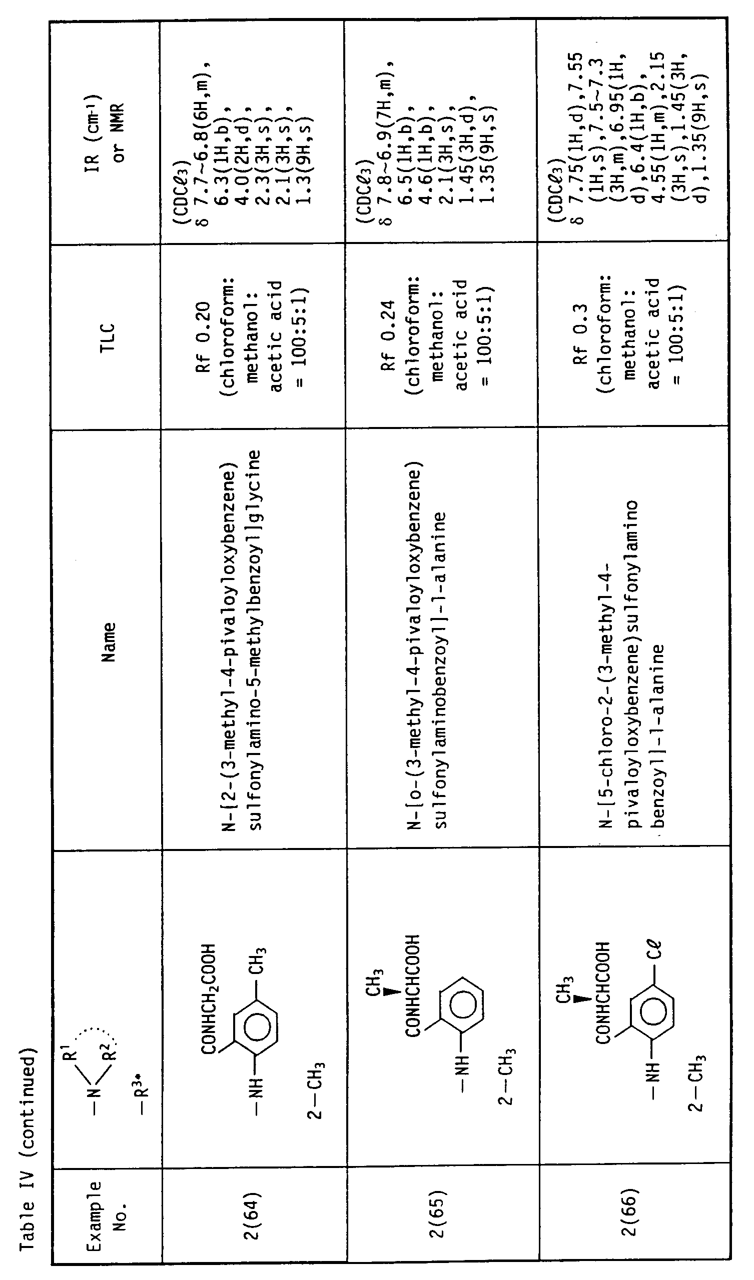

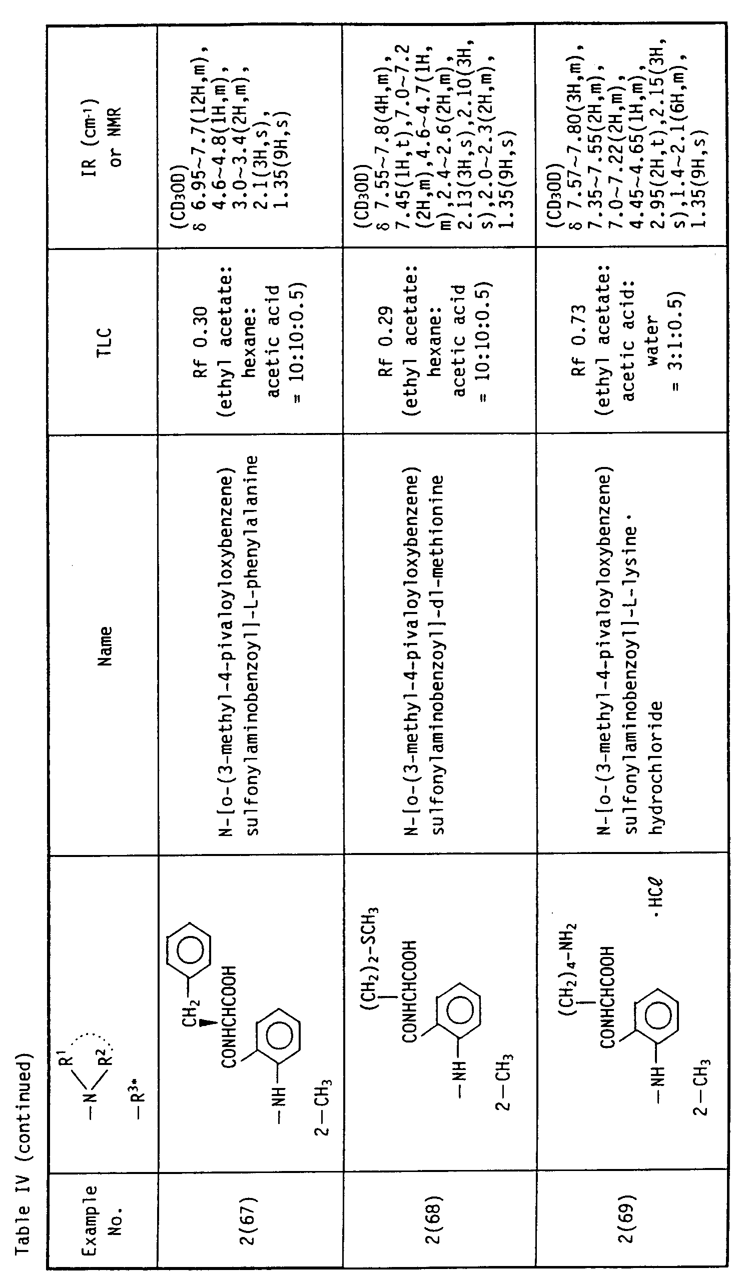

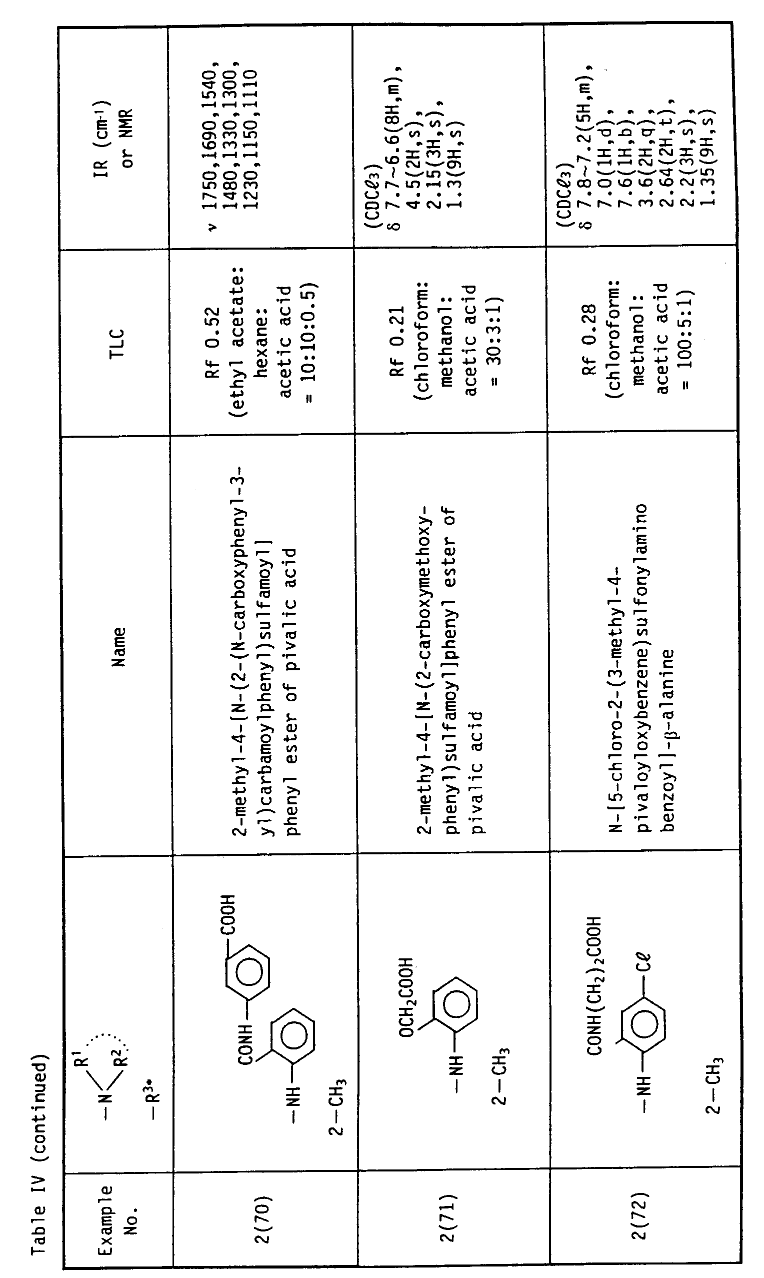

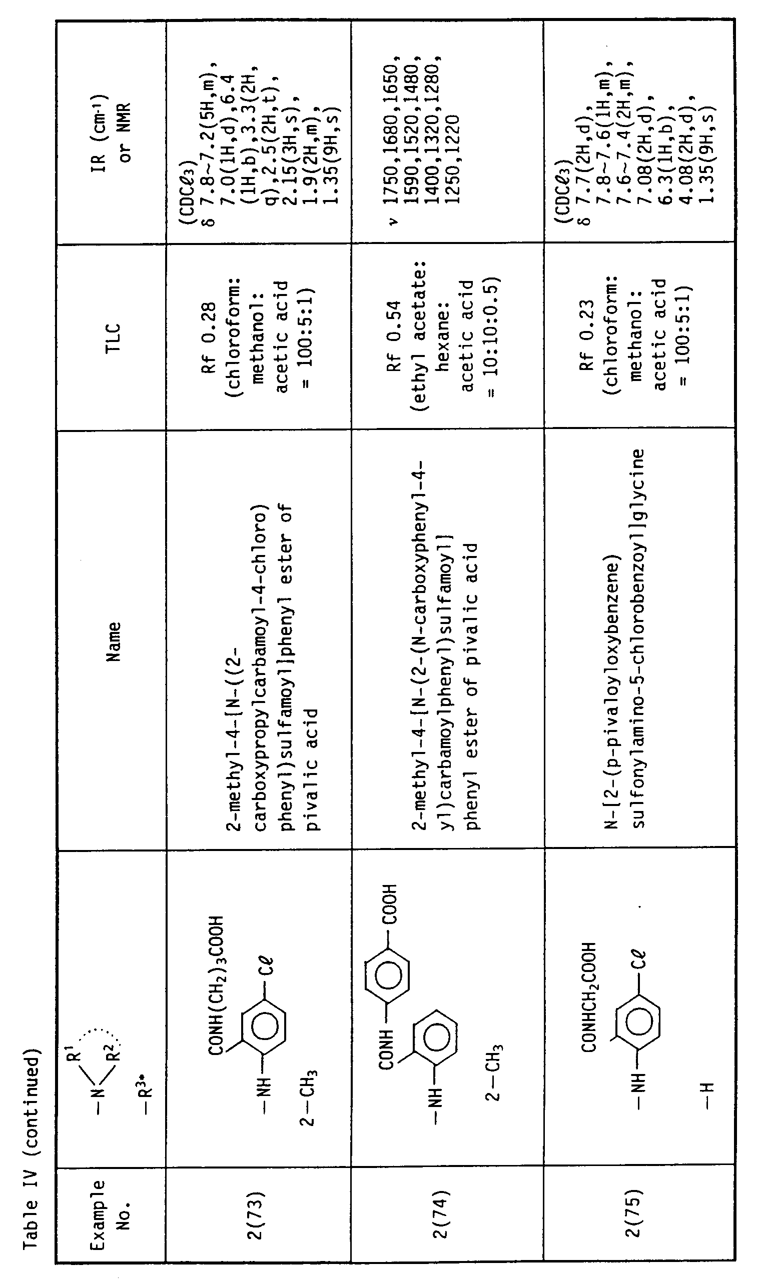

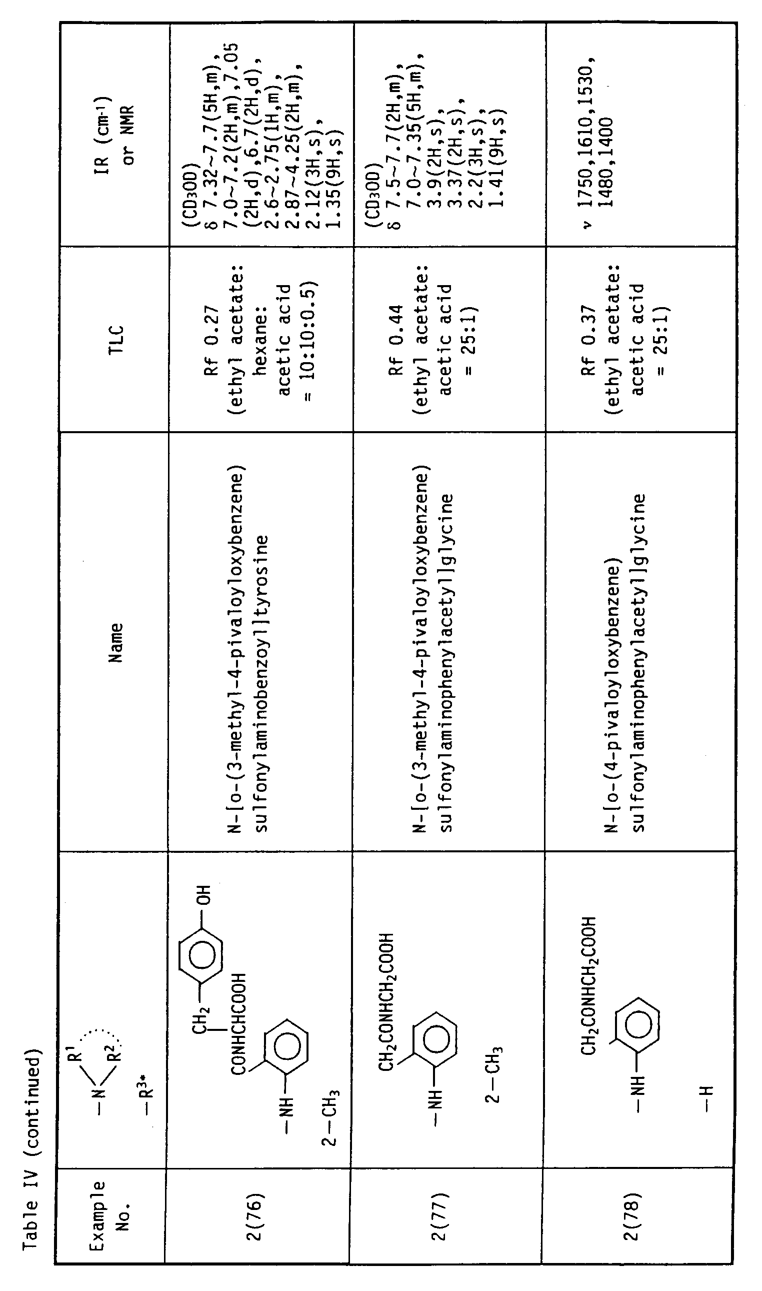

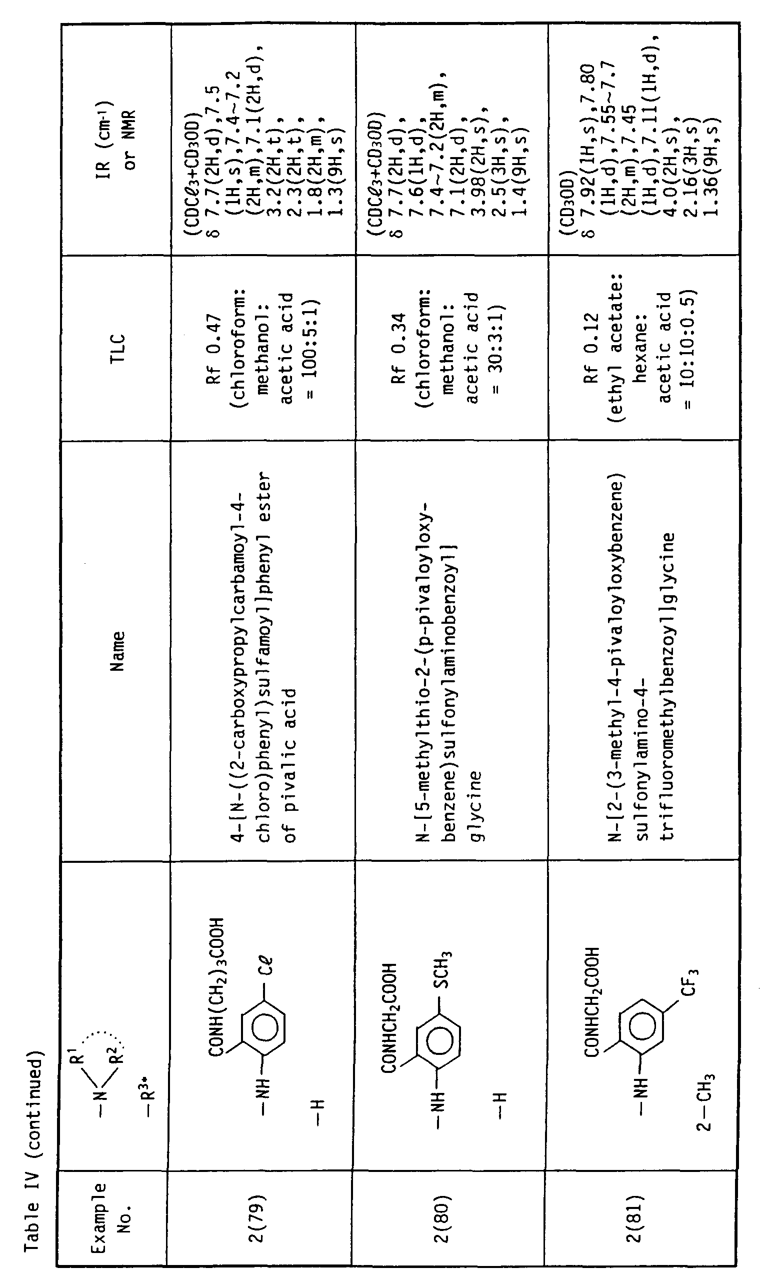

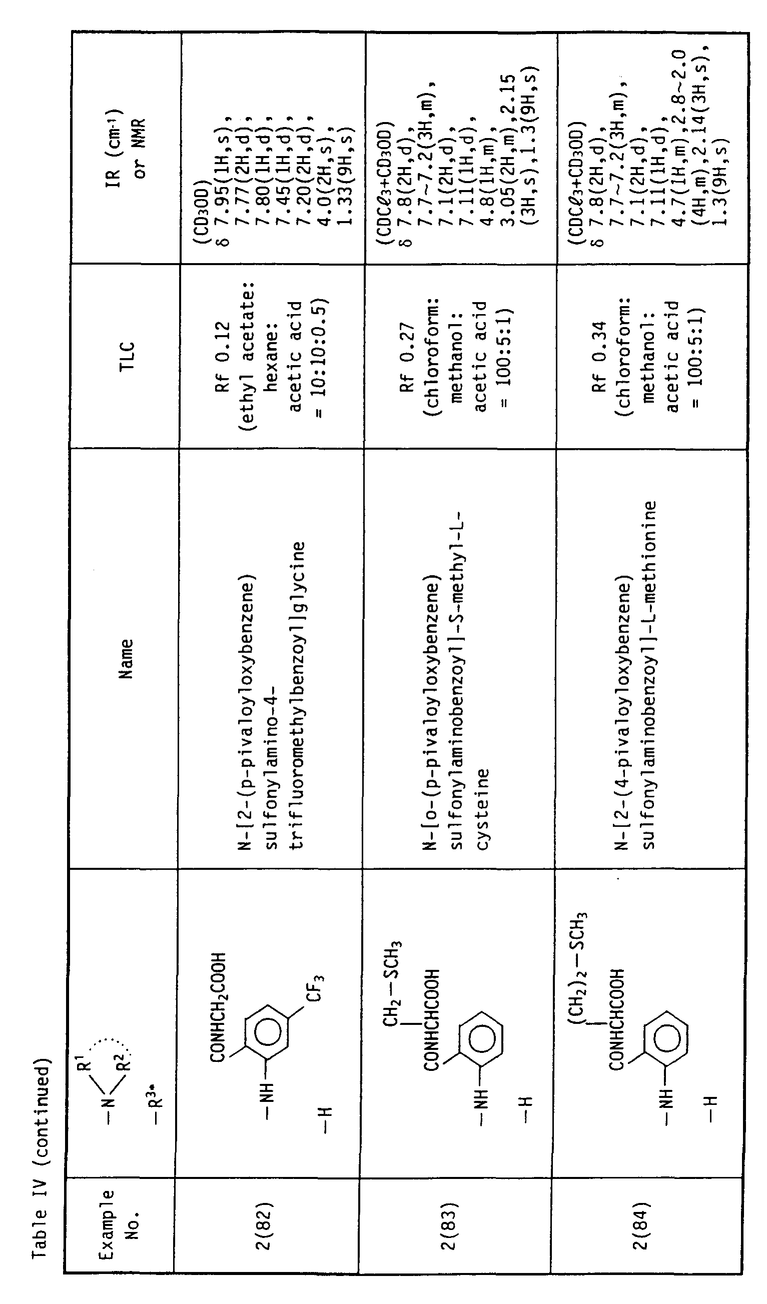

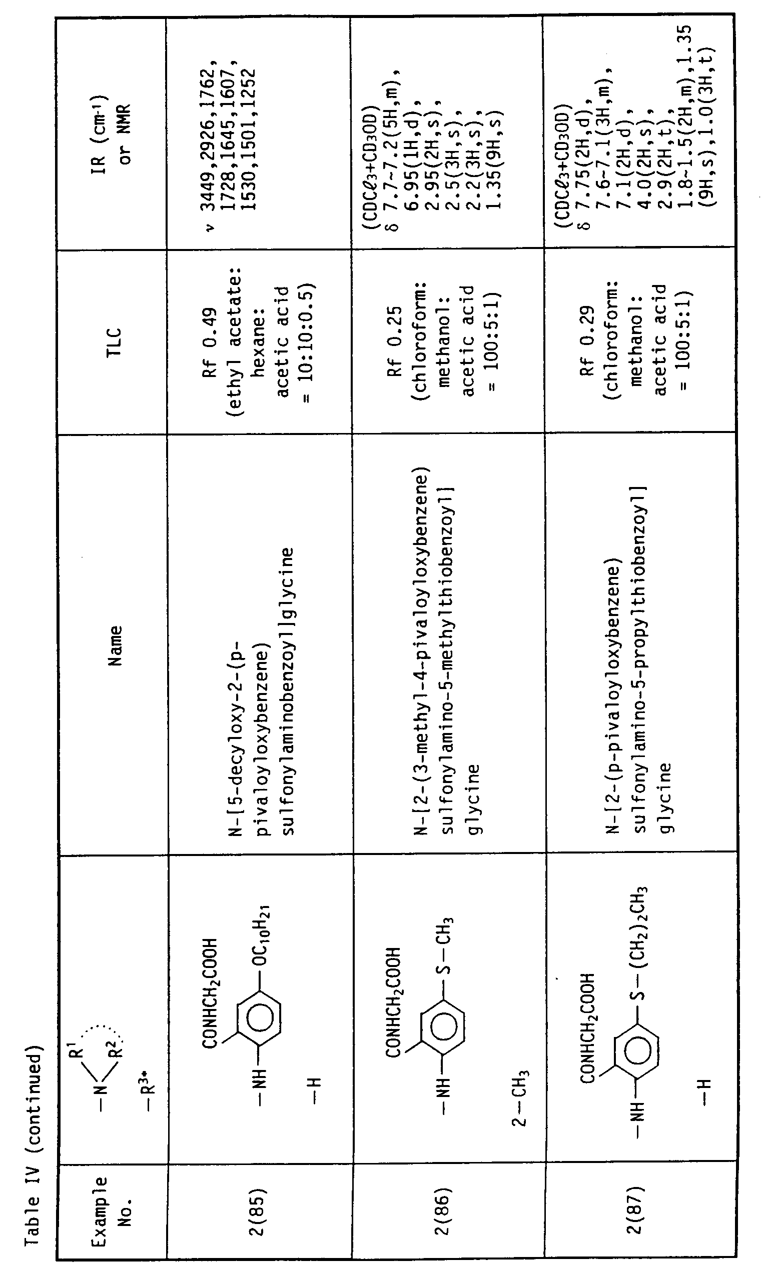

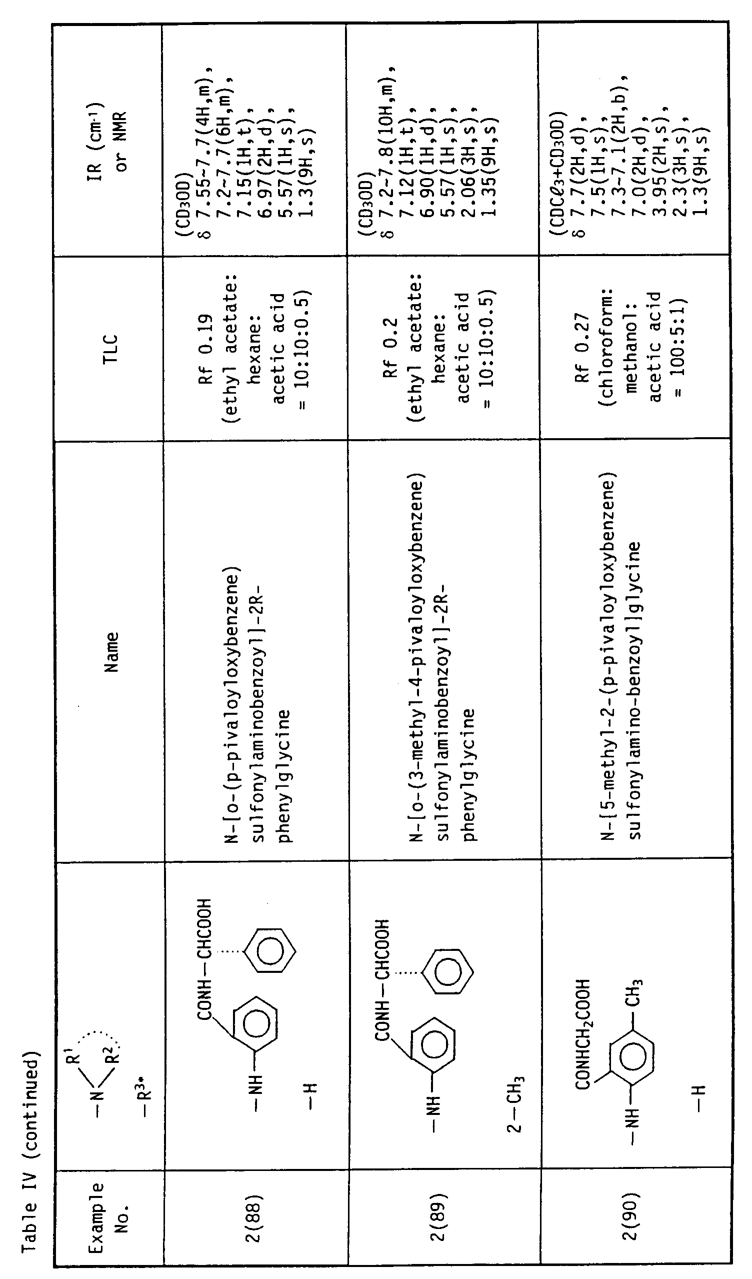

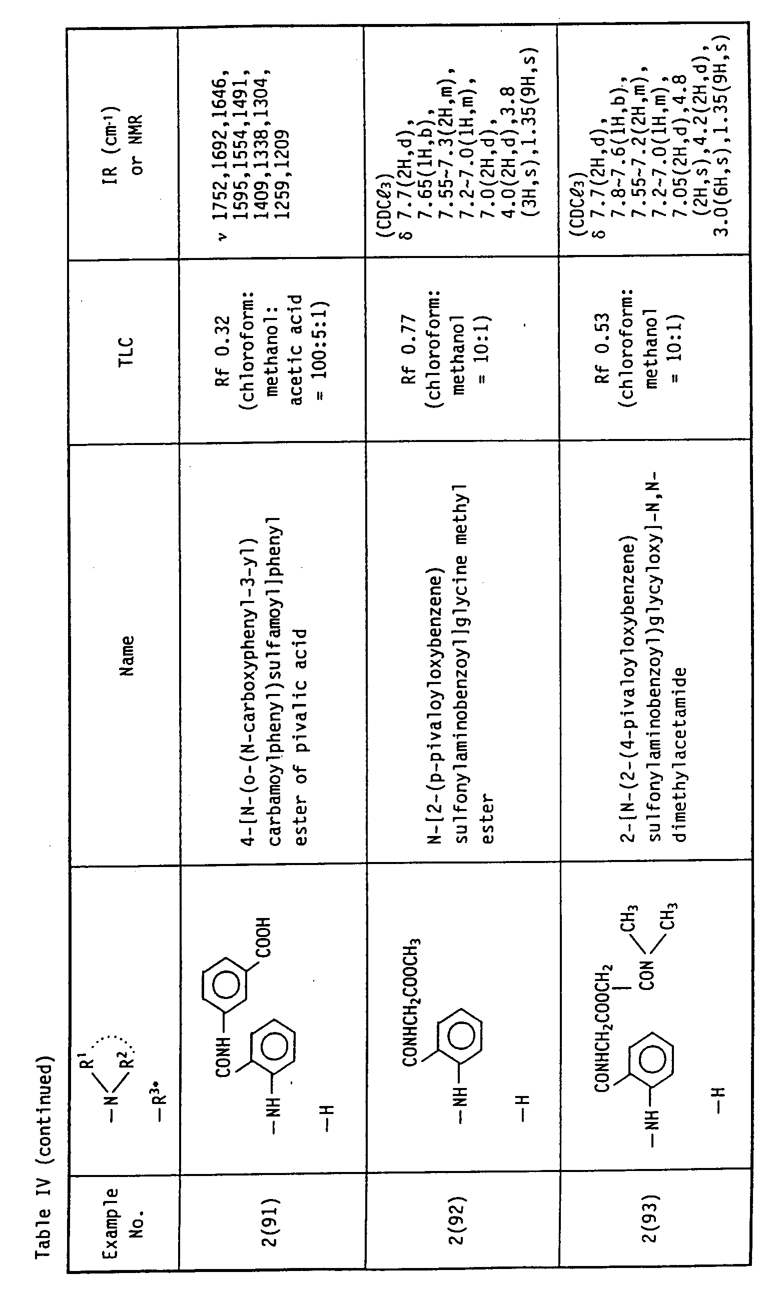

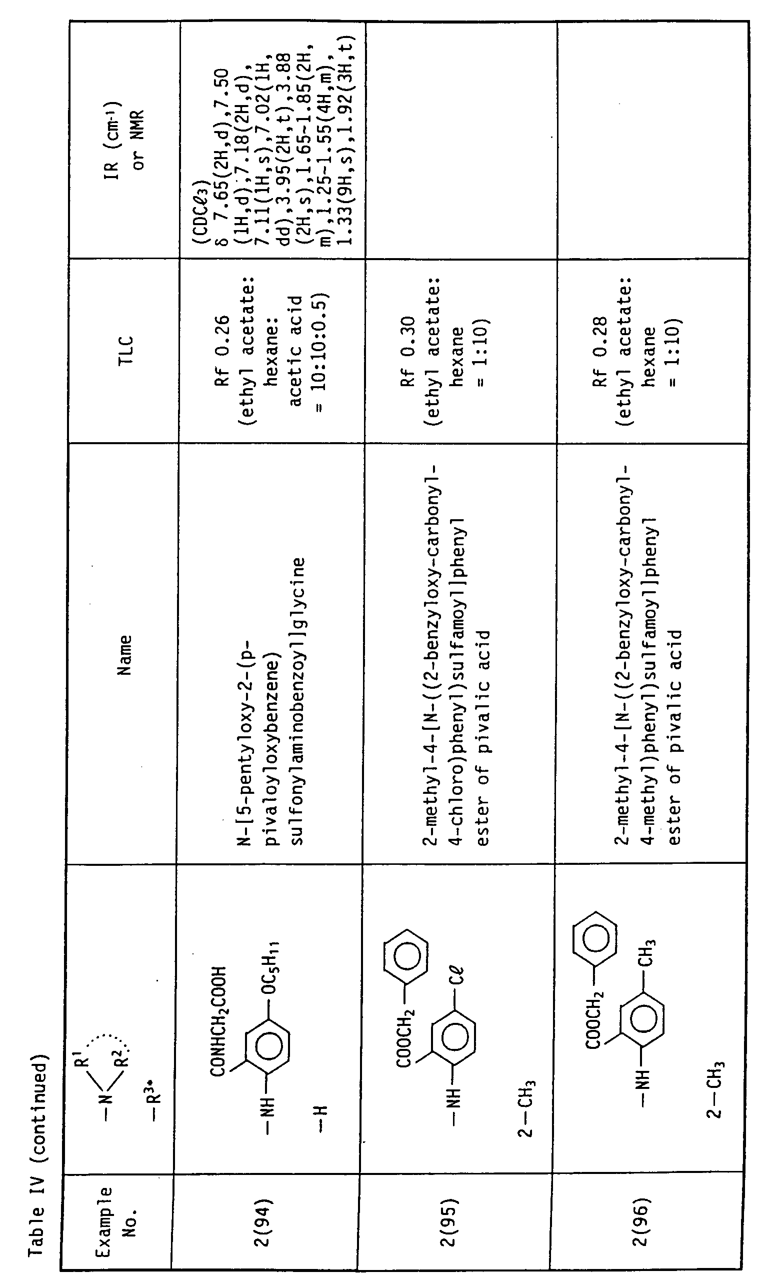

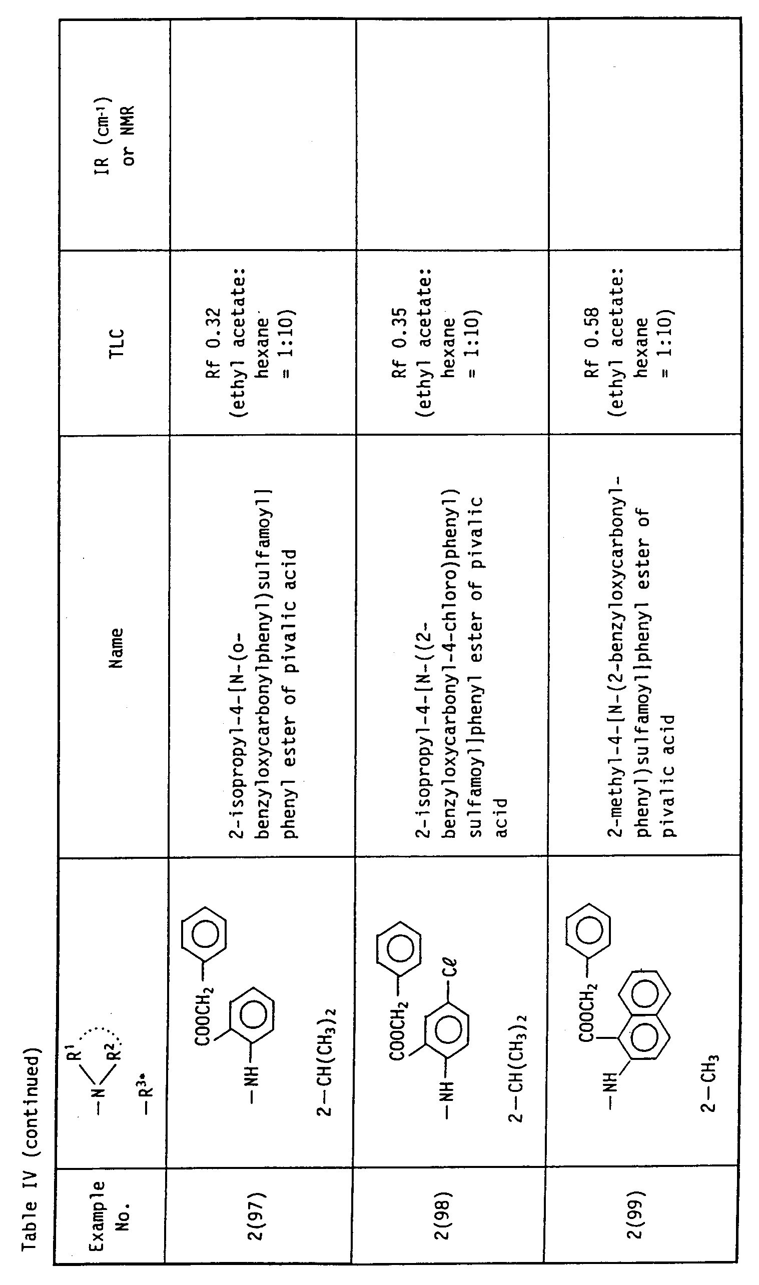

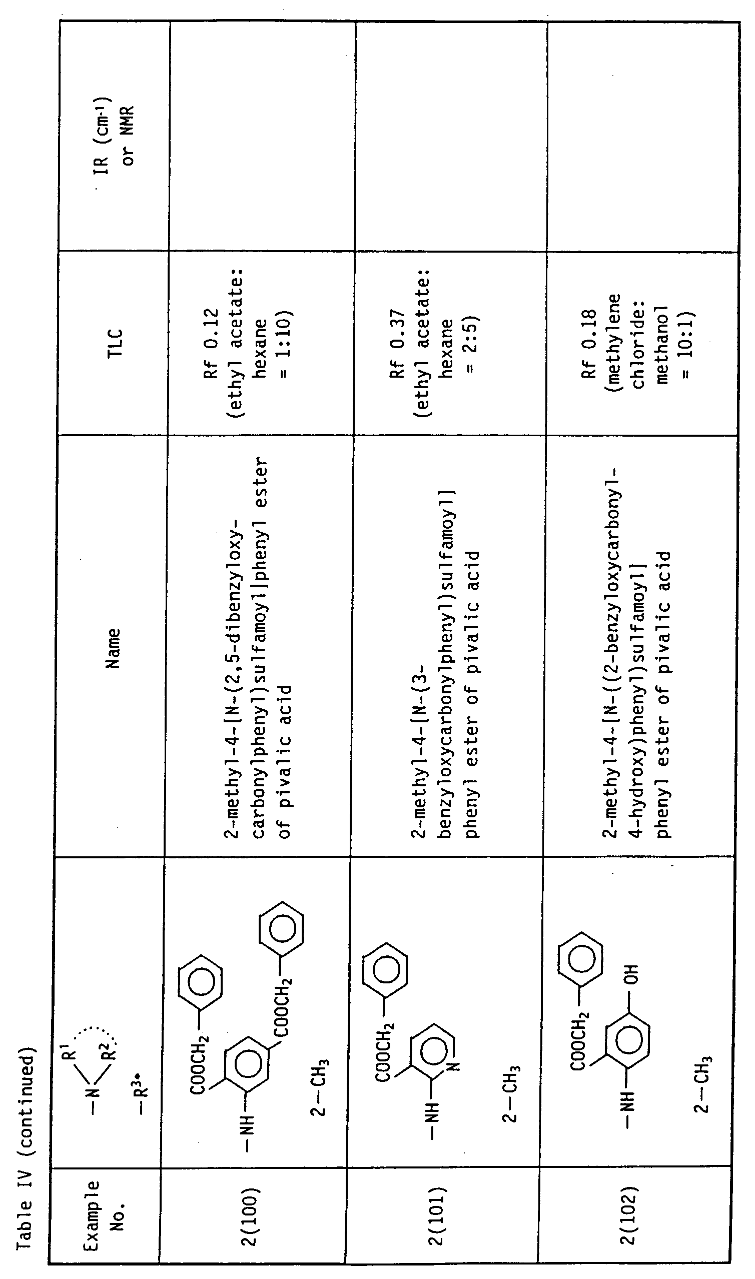

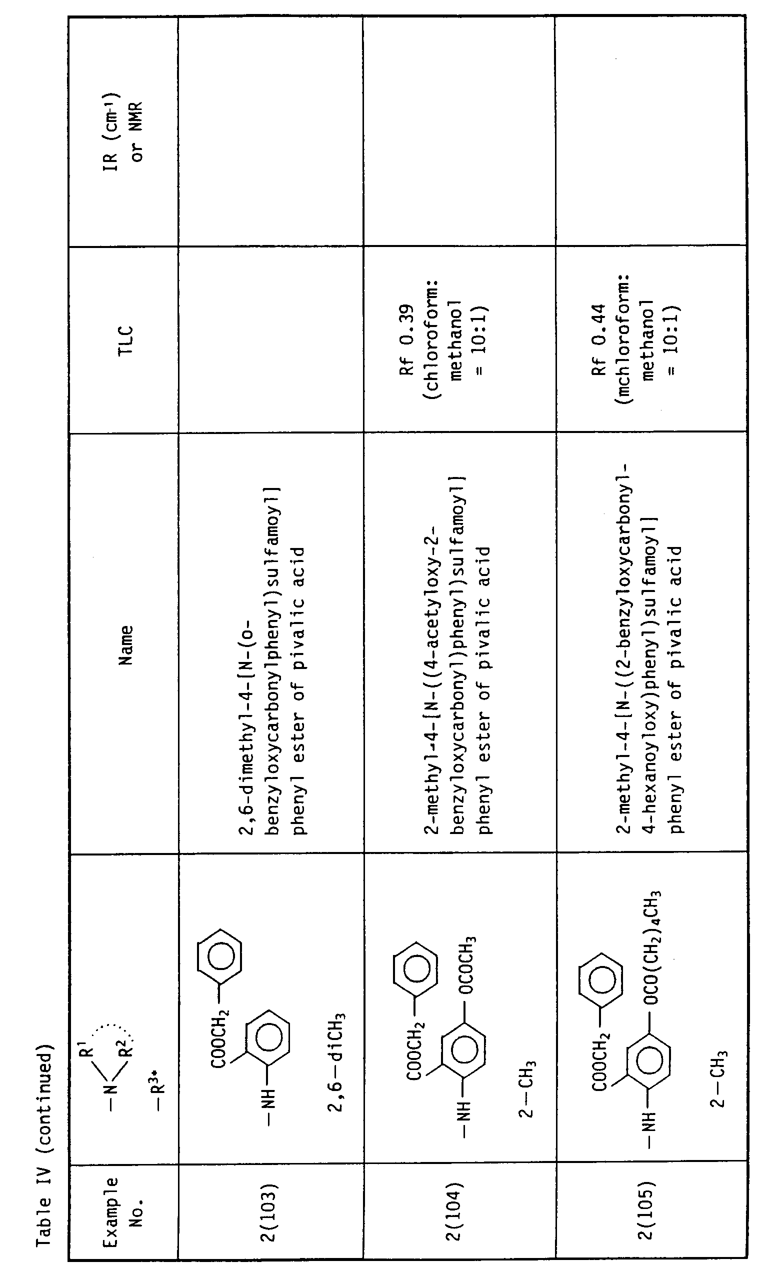

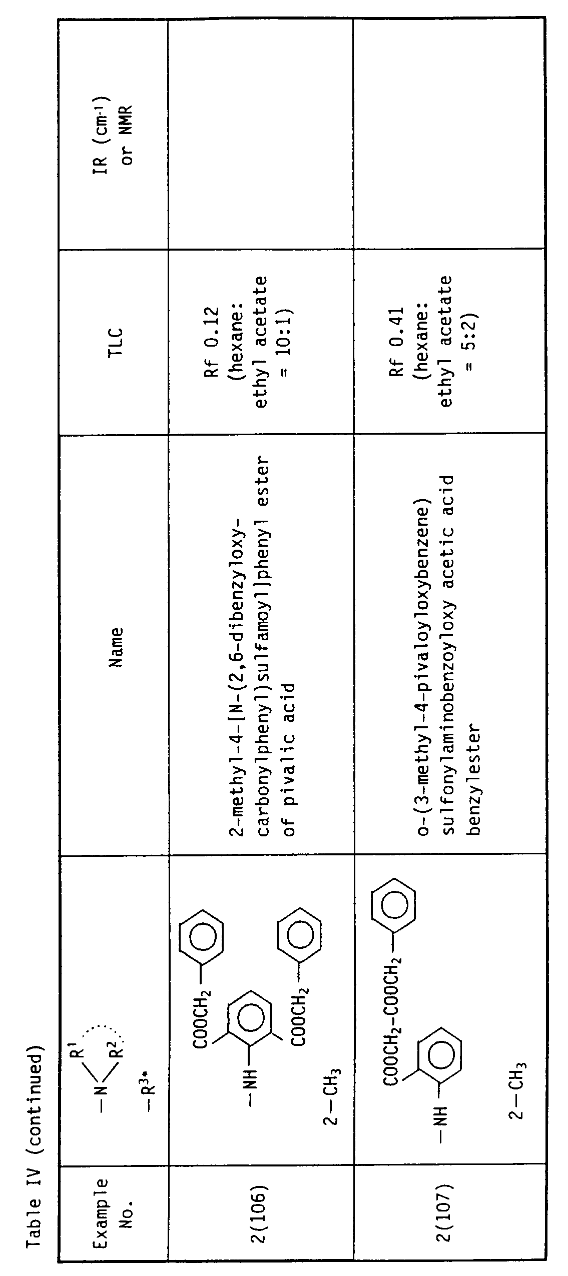

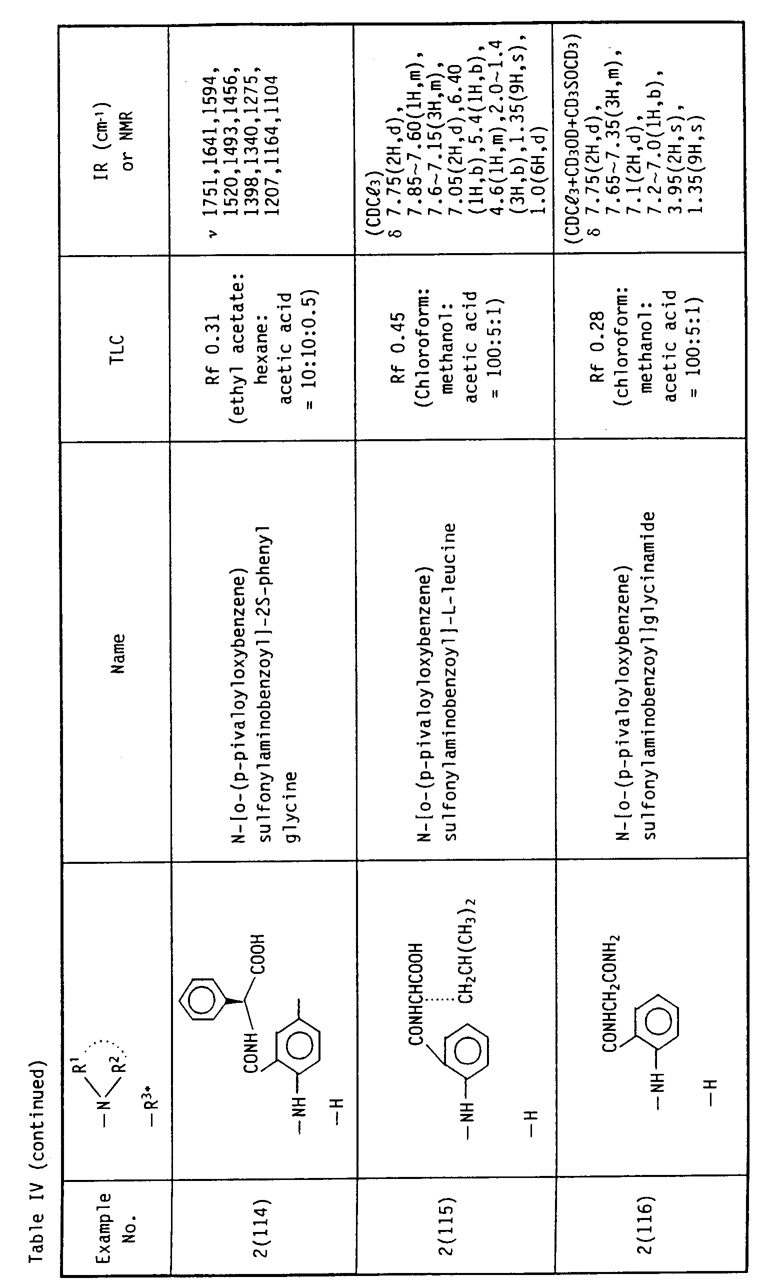

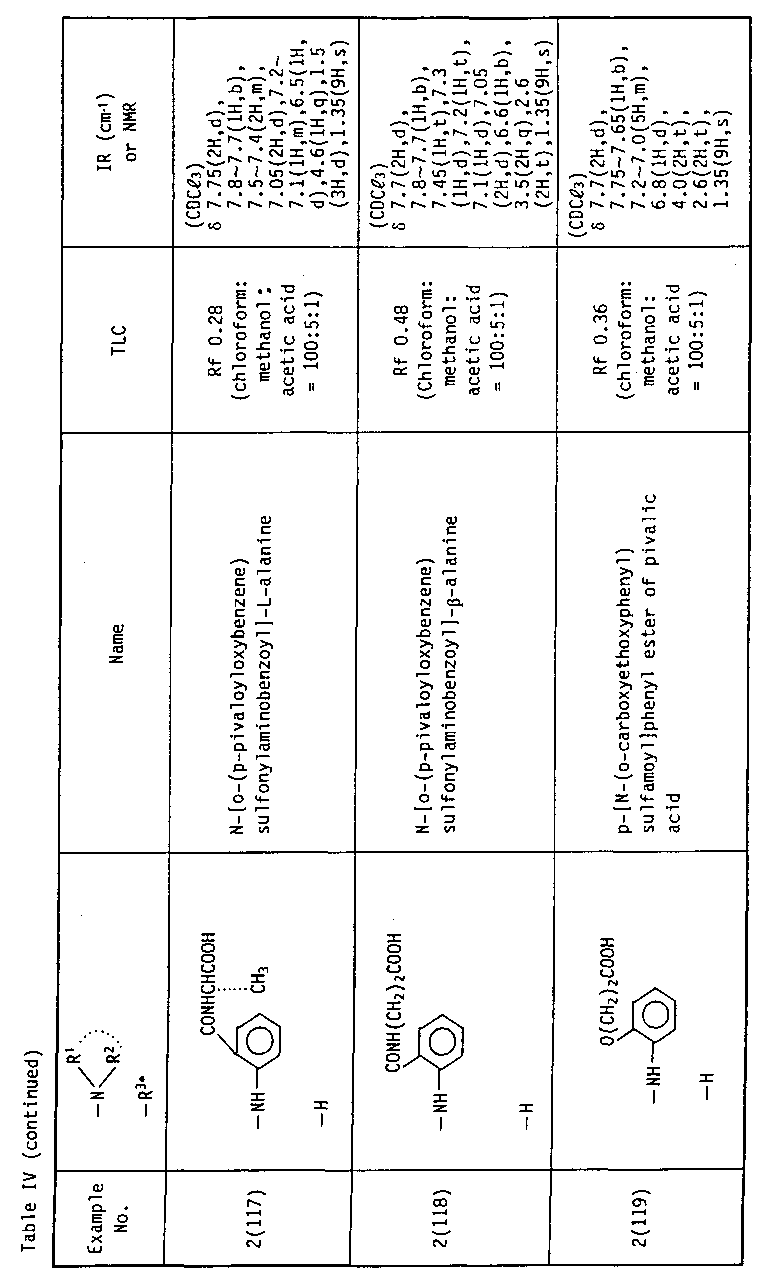

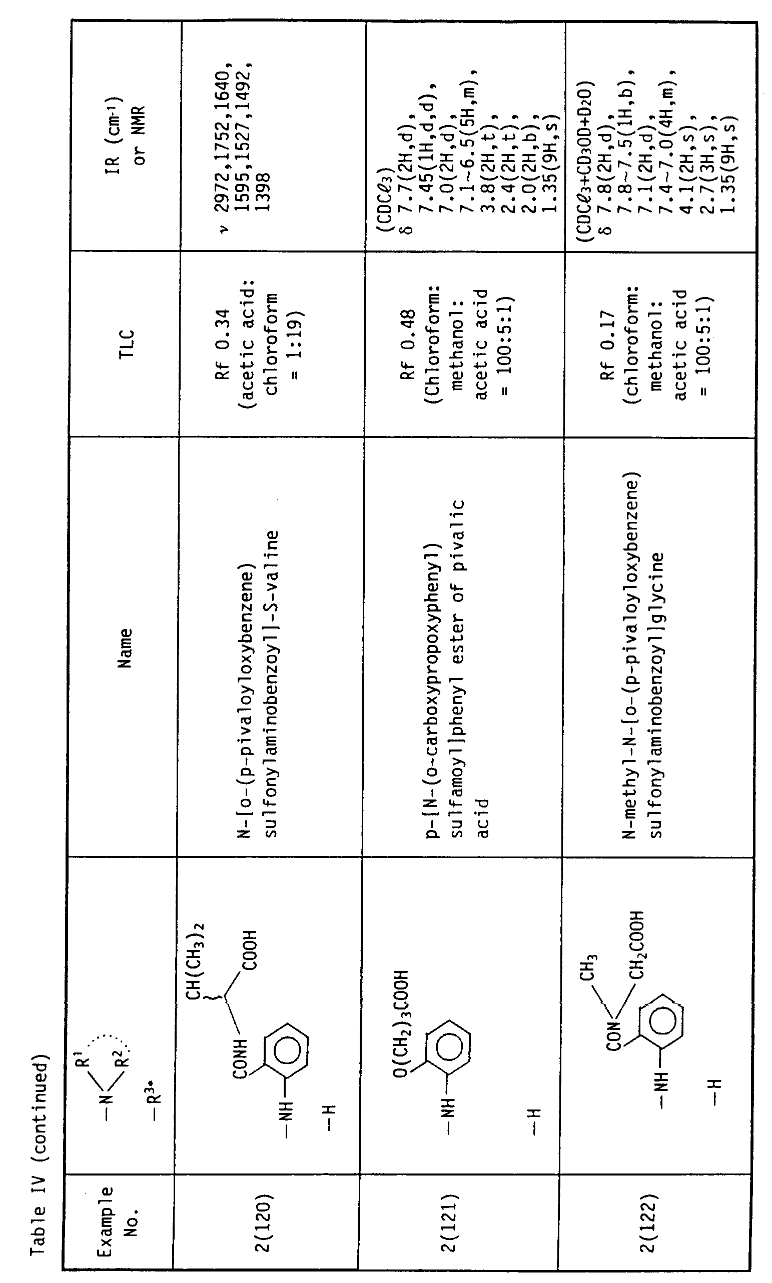

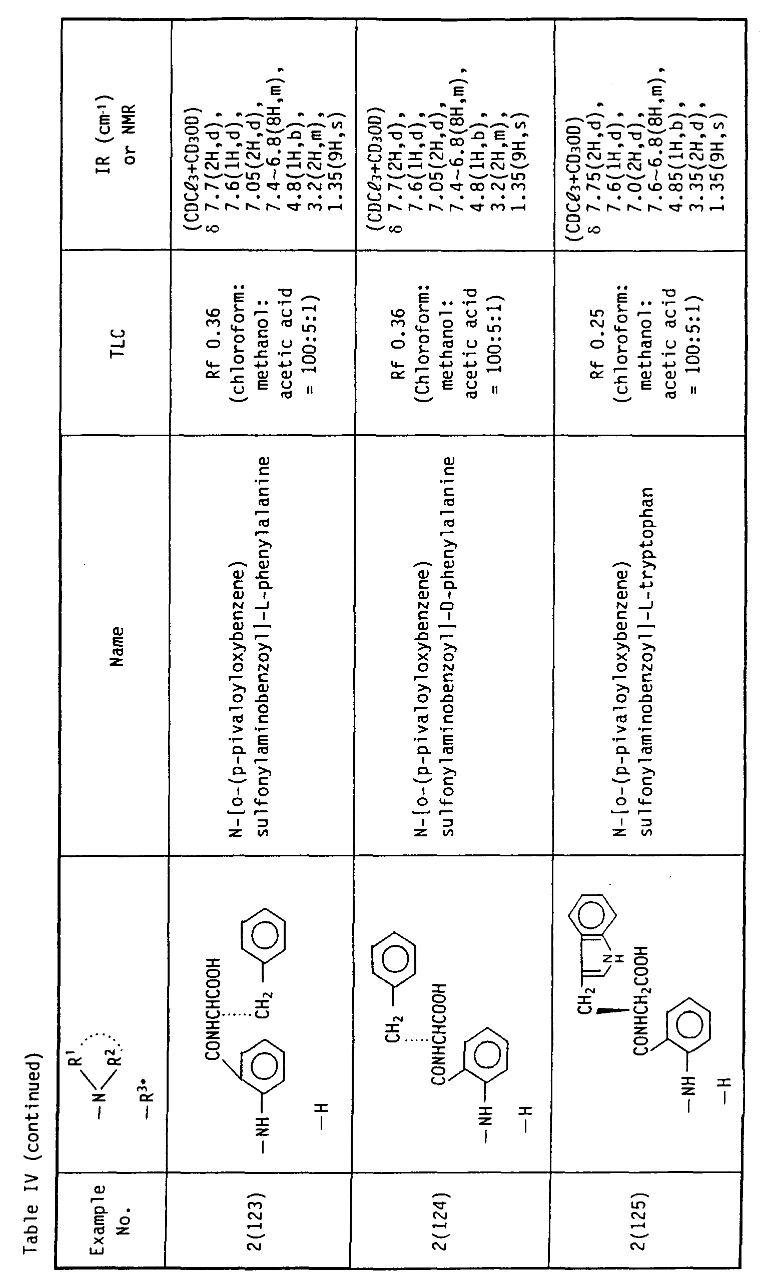

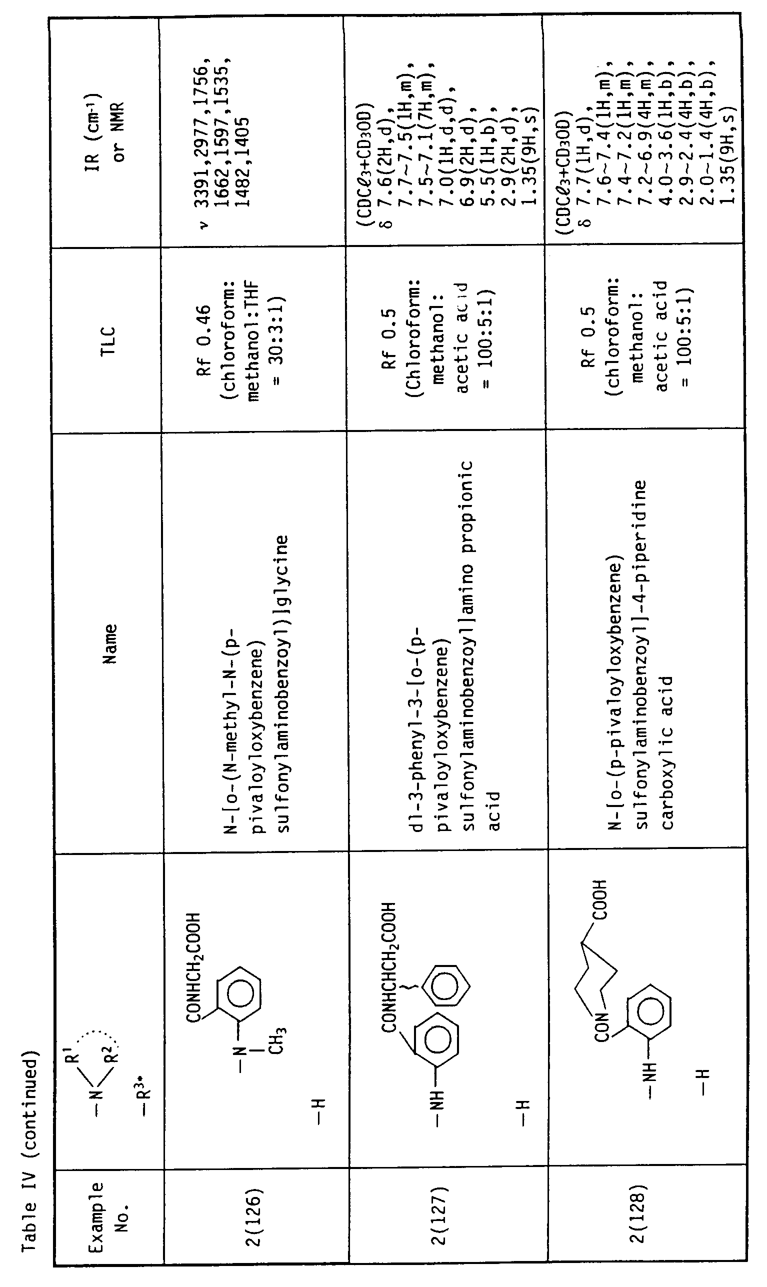

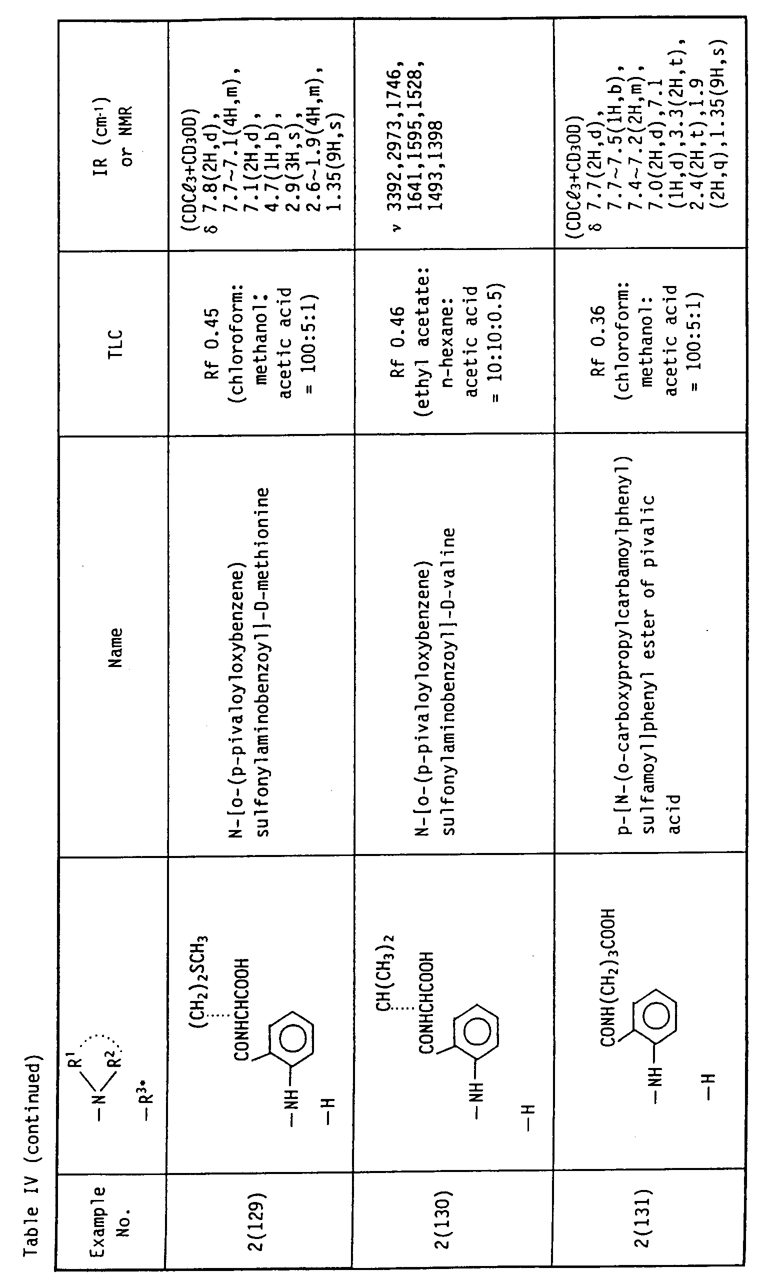

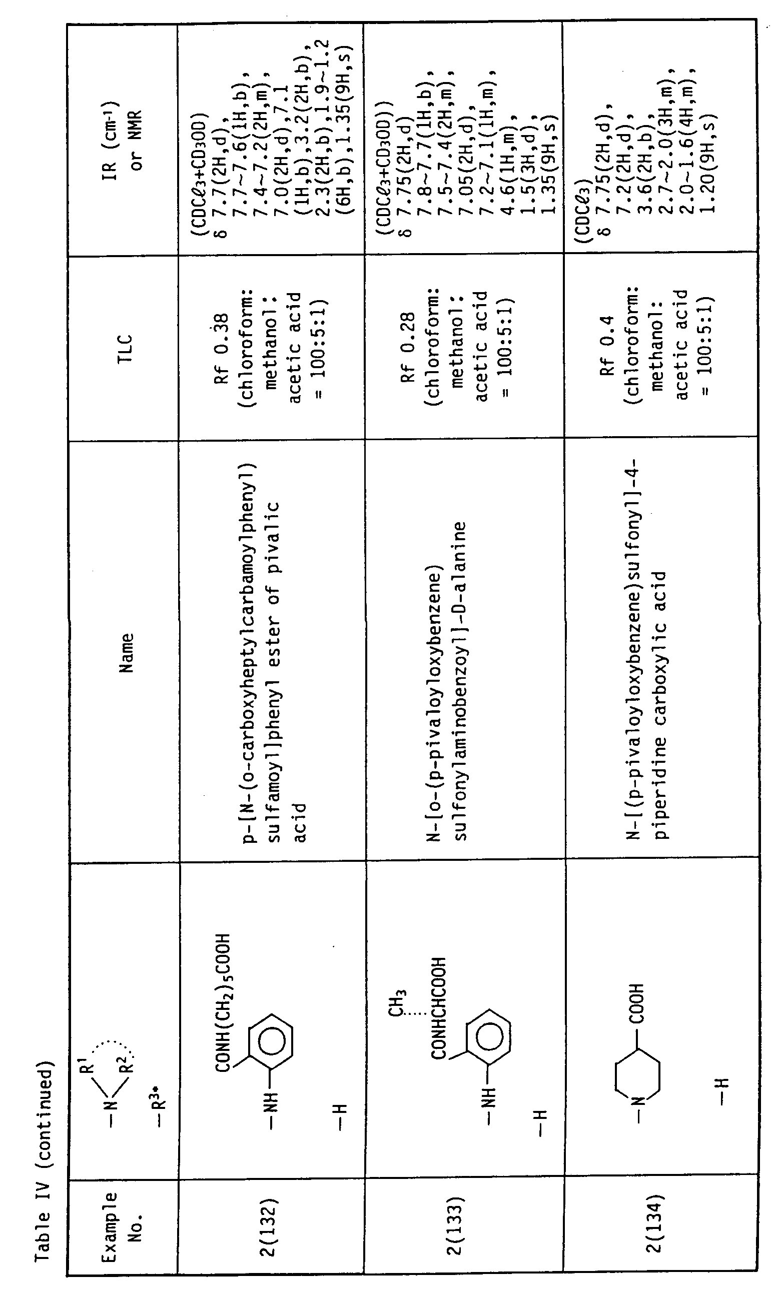

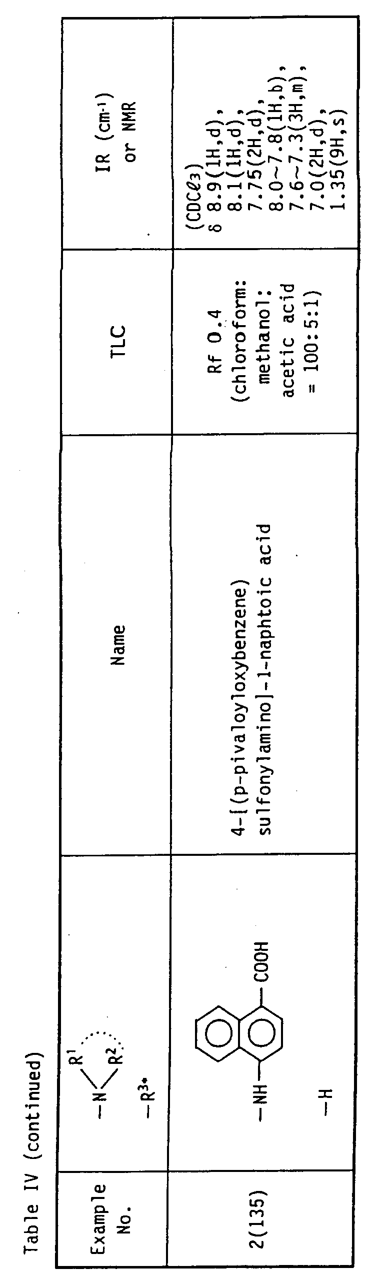

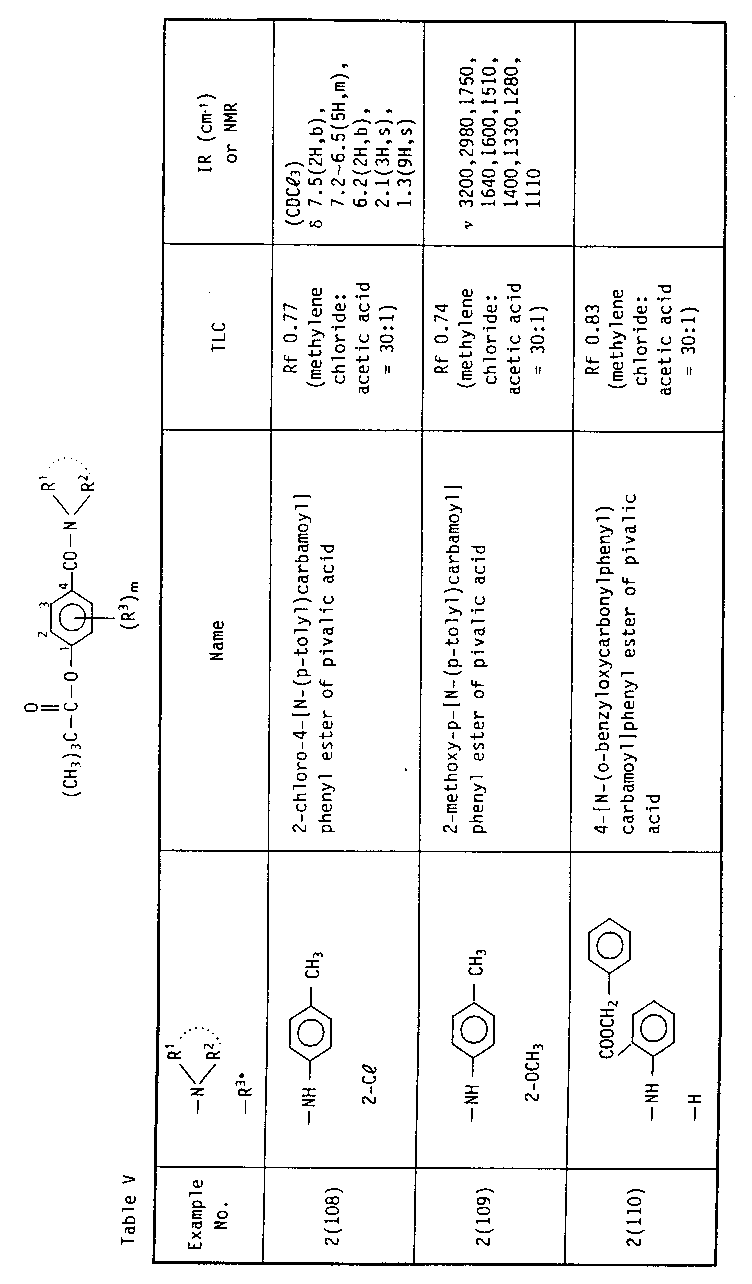

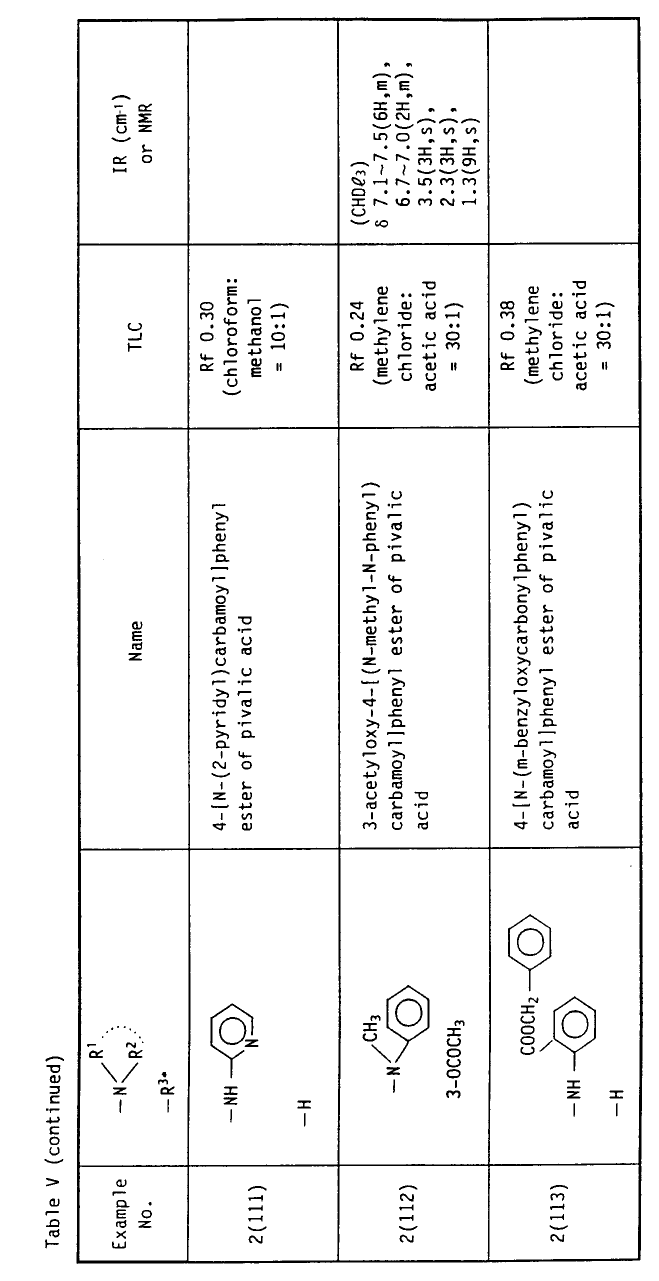

### Example 3

### p-[N-(p-(p-guanidinobenzoyloxy)phenyl)sulfamoyl]phenyl ester of pivalic acid (addition salt with acetic acid)

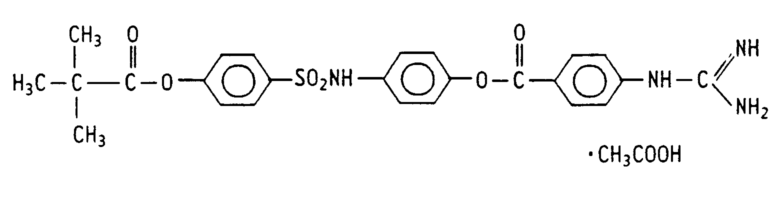

p-Guanidinobenzoyl chloride hydrochloride (800 mg) was added to pyridine solution (5 ml) of the compound of the present invention (500 mg) obtained in Example 2(7) under cooling with ice, and the mixture was stirred for 2 hours.

After reaction, ether was added to the reaction mixture and the supernatant was decanted. Saturated aqueous solution of sodium bicarbonate was added to the residue to obtain oily carbonate.

Further, the supernatant was decanted and the residue was purified by column chlomatography on silica-gel (ethyl acetate : acetic acid : water = 400 : 100 : 30) to give the title compound (532 mg) having the following physical data.
TLC : Rf 0.80 (ethyl acetate : acetic acid : water = 3 : 1 : 1
IR : ν3600~2300, 1750~1700, 1680, 1500, 1400.

### Example 4

### p-[N-(p-benzyloxycarbonylphenyl)sulfamoyl]phenyl ester of pivalic acid

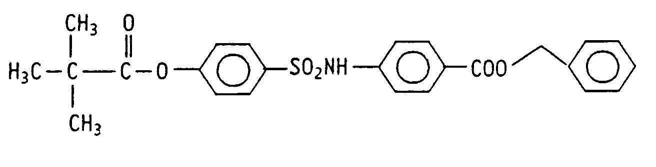

The title compound (210 mg, same as compound obtained in Example 1(5)) was obtained by the same procedure as Reference Example 1 - Example 1, by using a corresponding sulfonylchloride compound as starting material.

Further compounds of the present invention described in Tables VI and VII which follow, were obtained by the same procedure as Example 4, by using a corresponding amine and pivaloyl chloride.

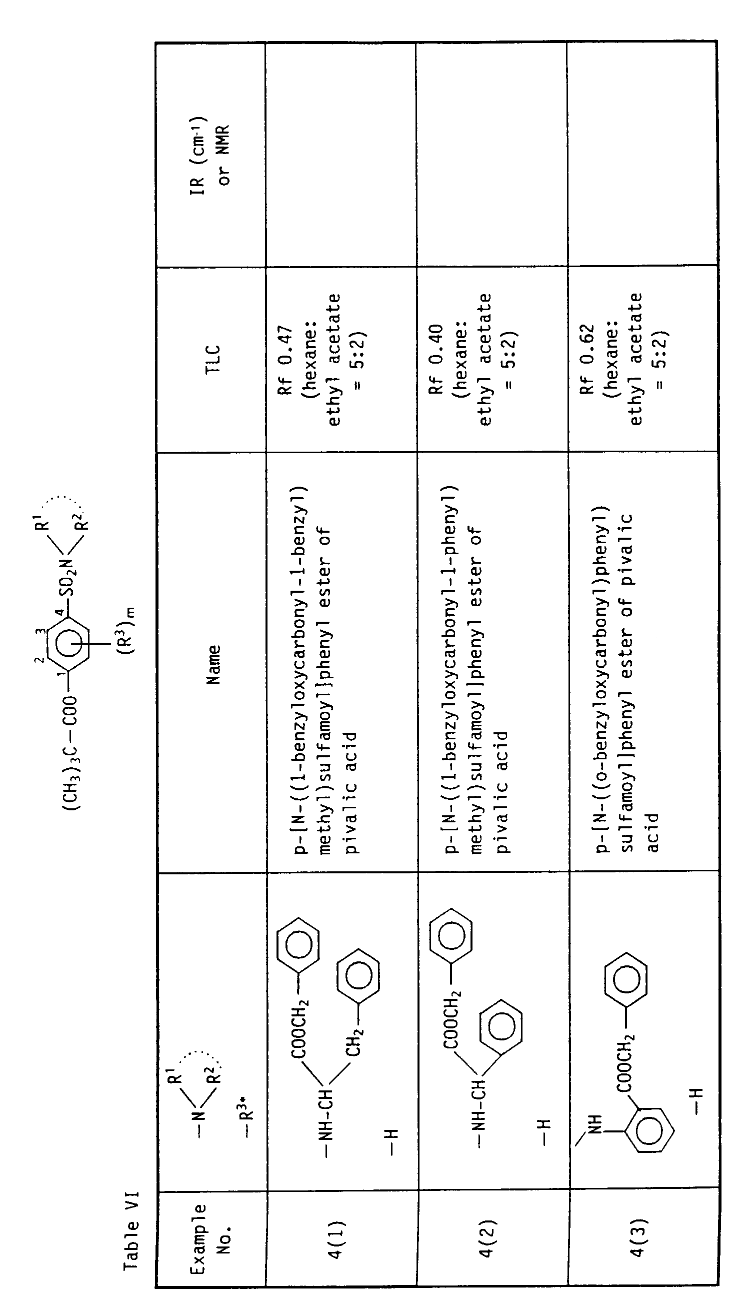

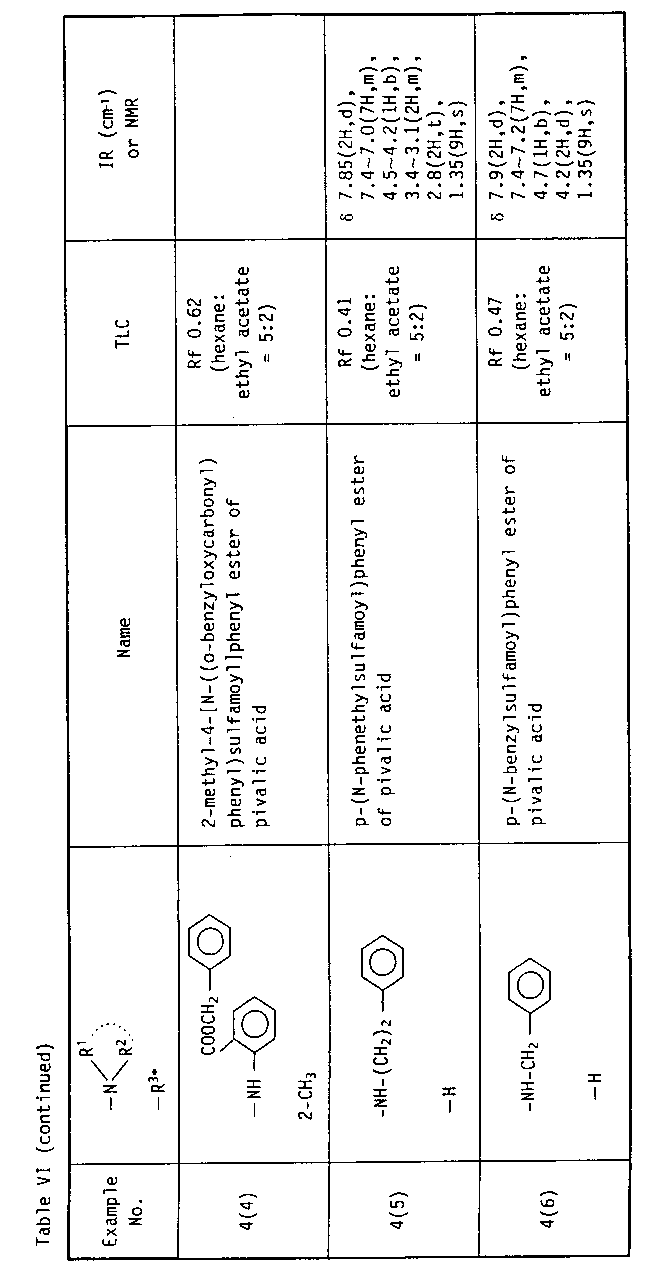

### Example 5

### p-[N-(p-carboxyphenyl)sulfamoyl]phenyl ester of pivalic acid

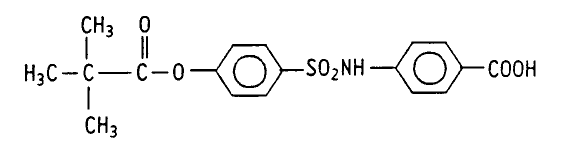

In an atmosphere of hydrogen gas, a mixture of a solution of the benzyl compound (190 mg) of Example 4, 10% Pd-carbon (30 mg), acetic acid (10 ml) and THF (4 ml) was stirred for 3 hours at room temperature.

The reaction solution was filtered off, and the filtrate was carried out azeotropic concentration by mixture of toluene-THF, and the azeotropic concentrate was recrystallized by mixture of ethyl acetate-hexane to give title compound (143 mg) having the following physical data.

TLC : Rf 0.56 (ethyl acetate : hexane = 1 : 1);

IR : ν2700~2400, 1750, 1680, 1600, 1340, 1290, 1200, 1160, 1110 cm-¹.

Hereinafter, by the same procedure of Example 5, using corresponding benzyl compound, compounds of the present invention described in the following Table VII were obtained.

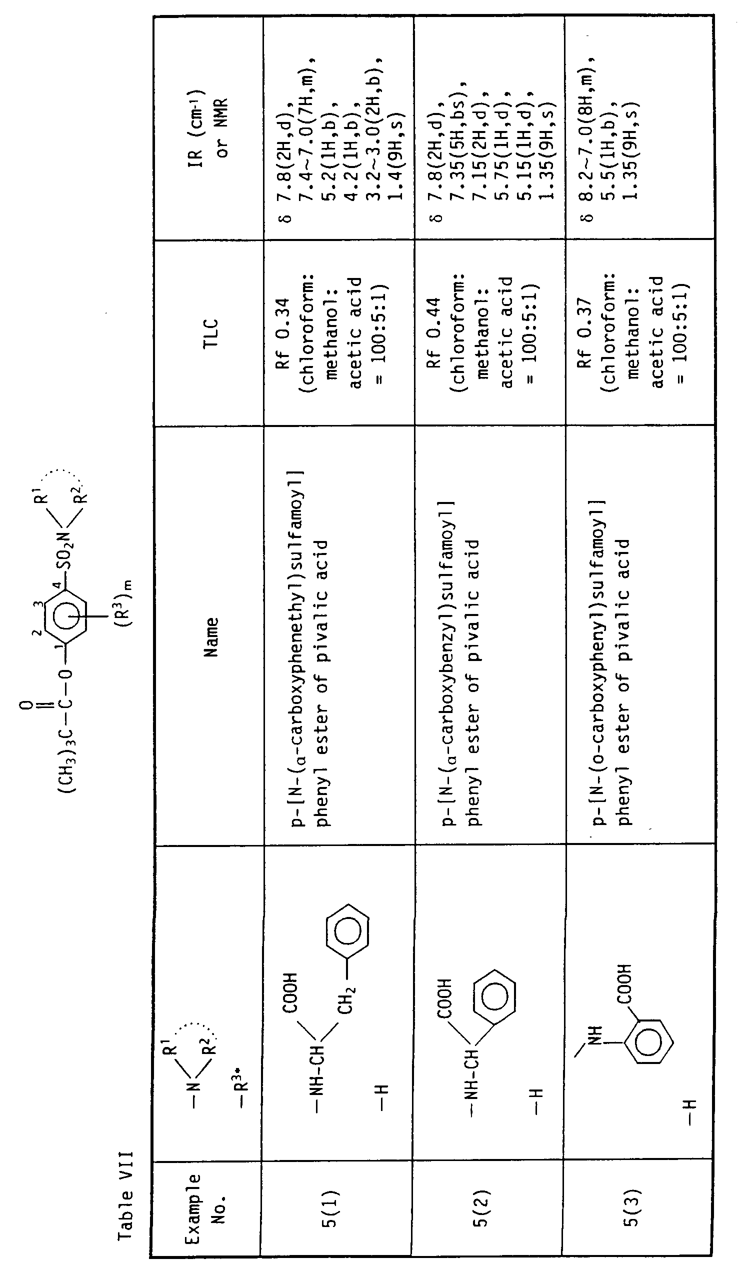

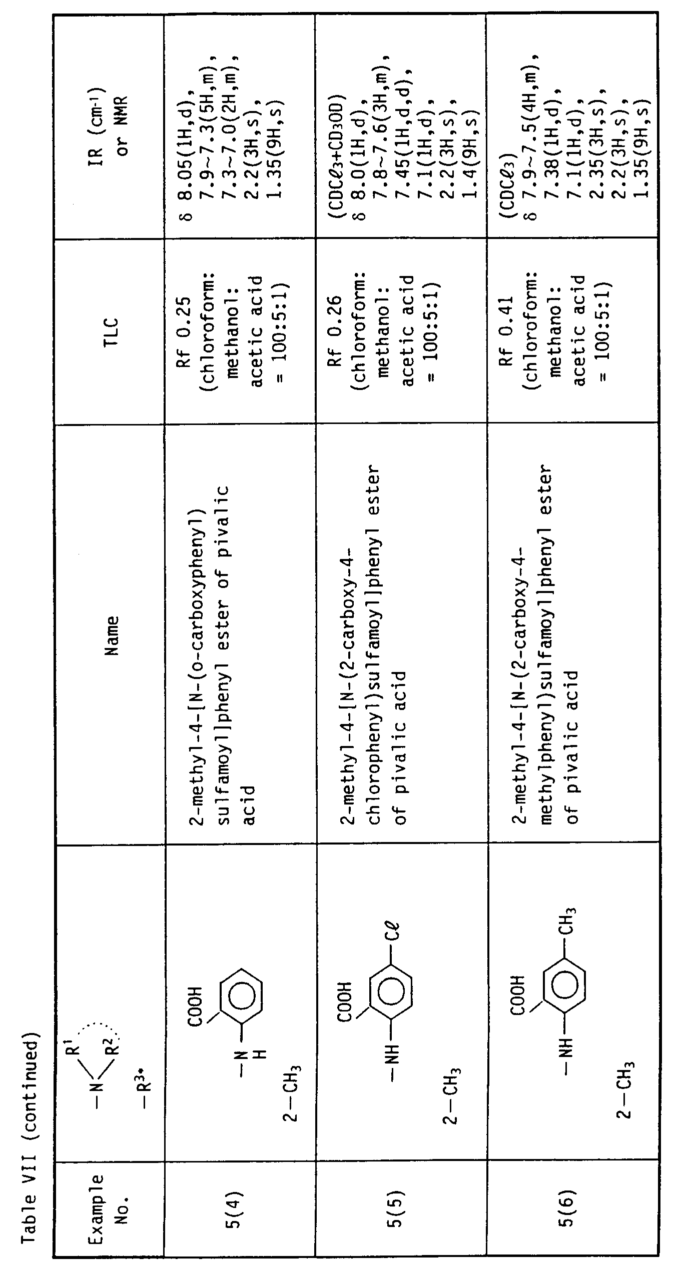

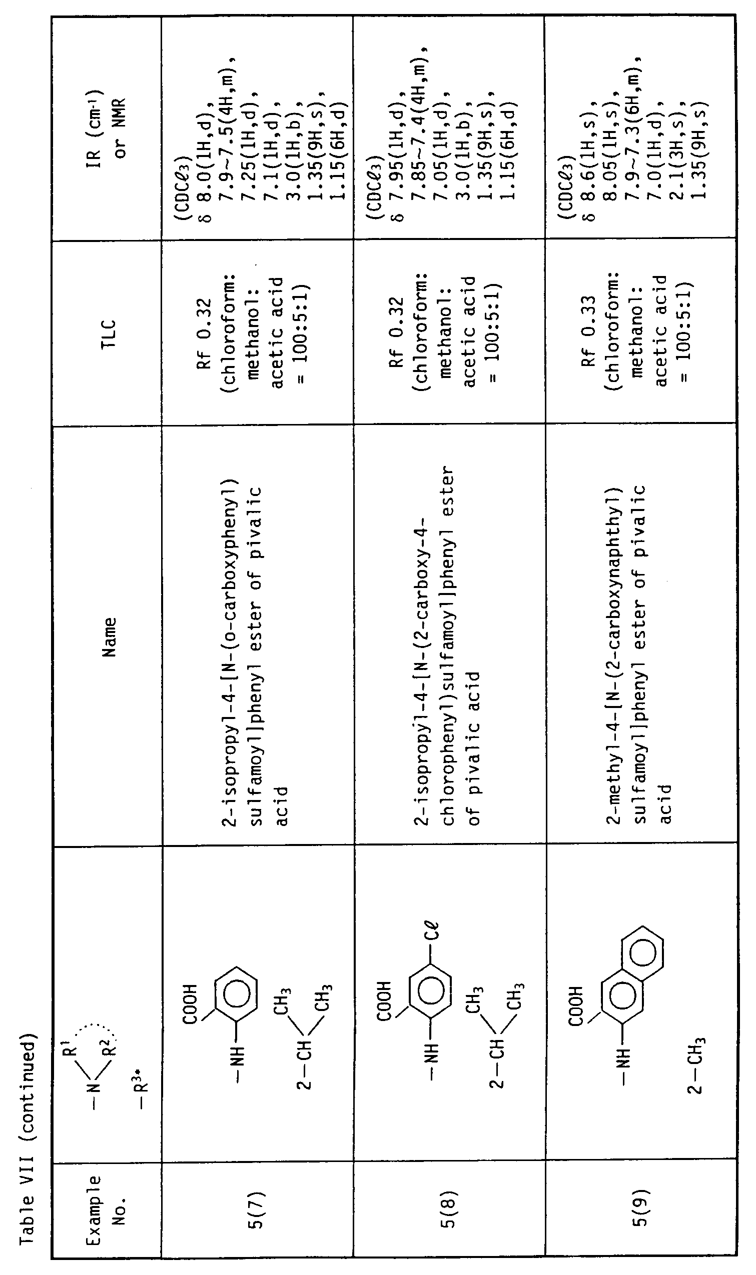

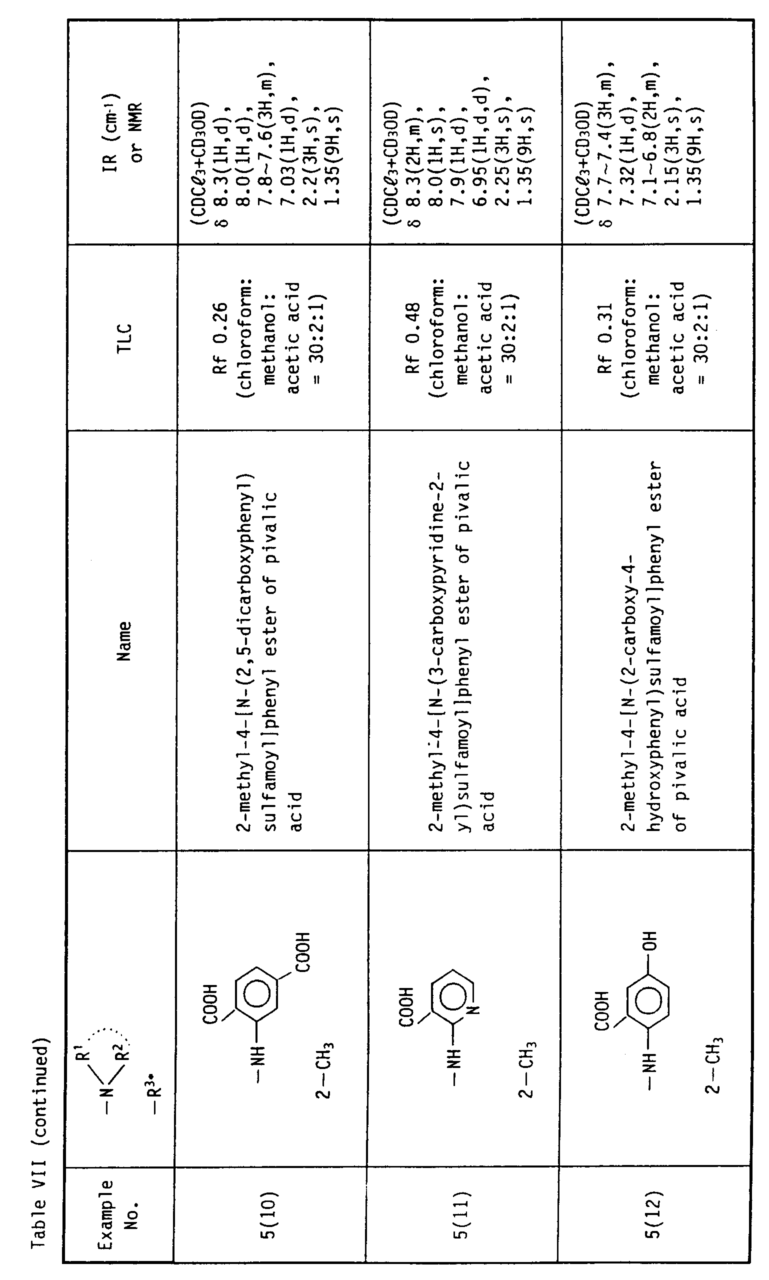

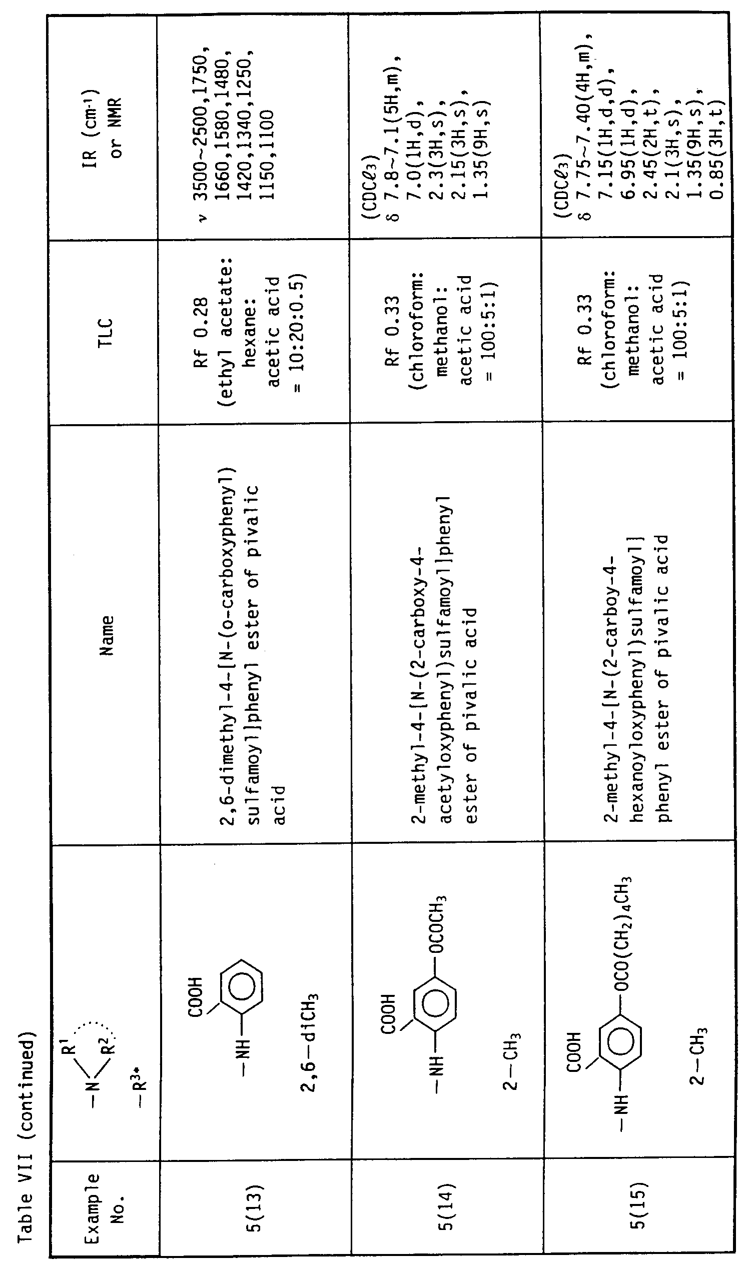

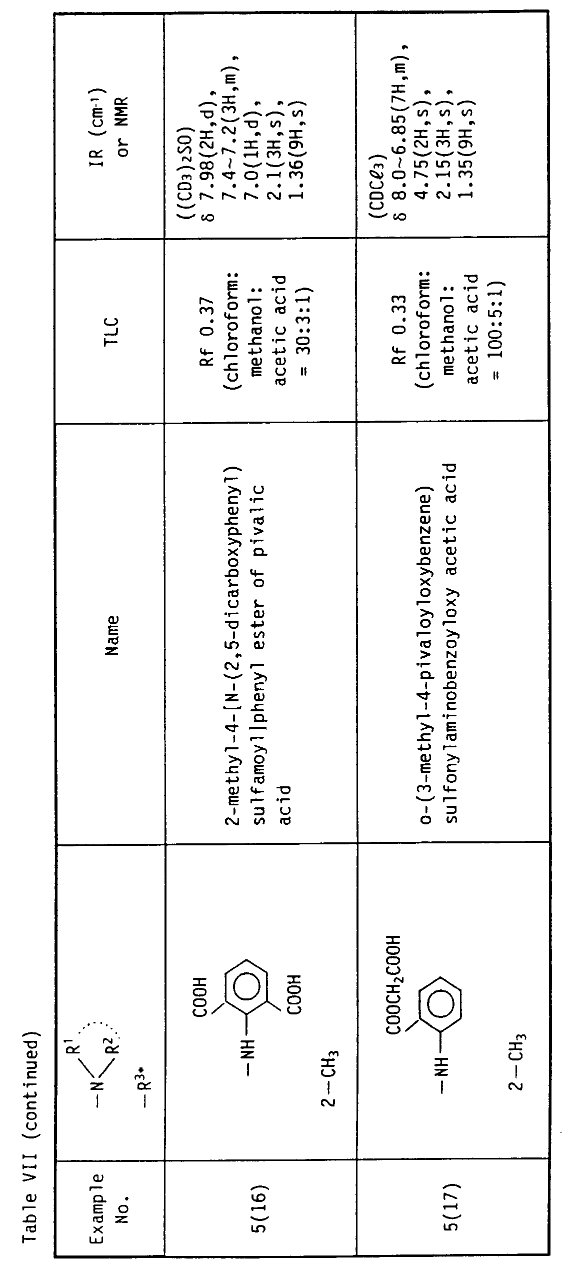

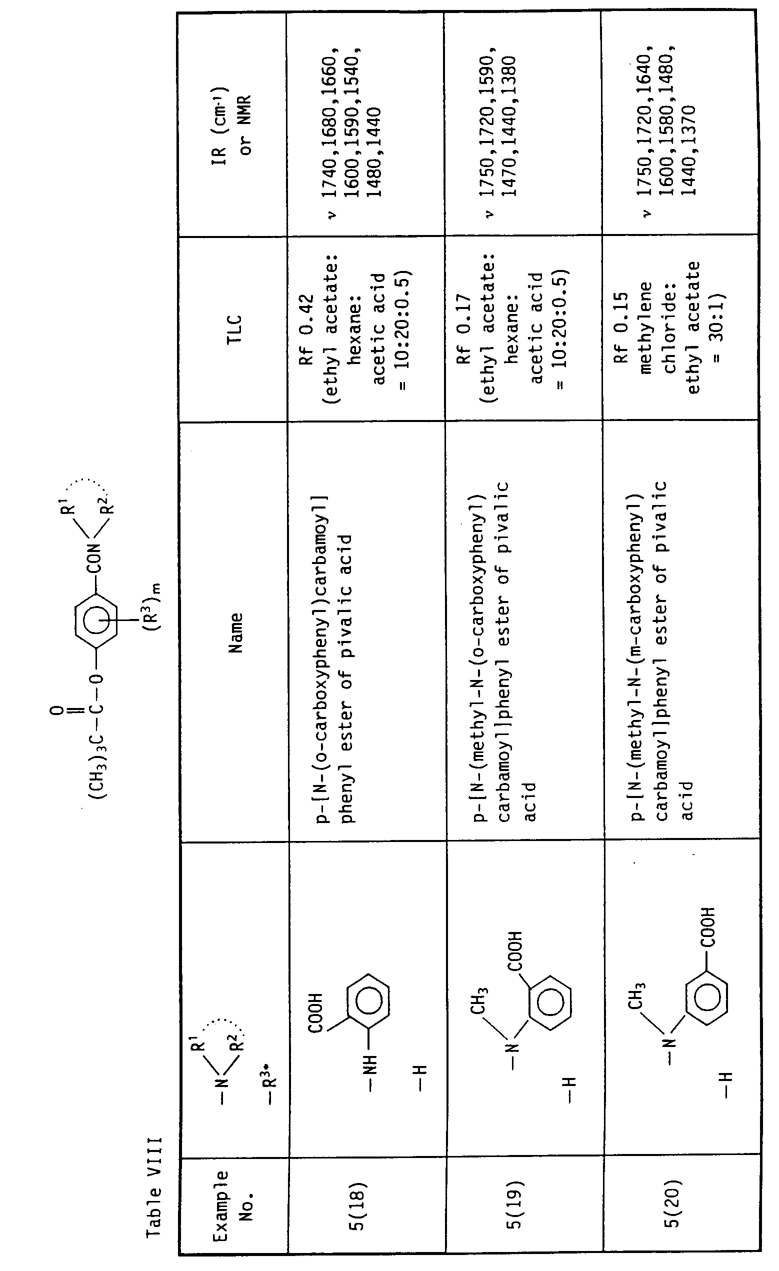

### Example 6

### p-[(N-methyl-N-phenyl)sulfamoyl]phenyl ester of pivalic acid

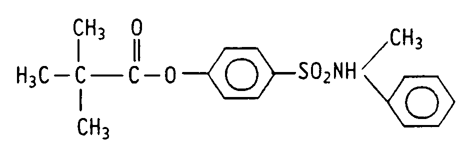

In an atmosphere of argon, THF solution (8 ml) of the compound of the present invention of Example 1 (1) (300 mg) was added to sodium hydride (37 mg) under cooling with ice, and the mixture was stirred for 2 hours.

Methyl iodide (66 µl) and hexamethylphosphoramide (HMPA) (1 ml) were added to the reaction solution, and the mixture was stirred for 30 minutes.

The reaction solution was extracted with ether, and the extract was washed with successive, water and saturated aqueous solution of sodium chloride.

And further, the solution was dried with sodium sulfate, and distilled off under reduced pressure to give the title compound (180 mg) having the following physical data.

TLC : Rf 0.61 (methylene chloride : ethyl acetate = 30 : 1);

IR : ν 1750, 1590, 1490, 1450, 1340 cm-¹.

Hereinafter, by the same procedure of Example 6, using corresponding derivative of pivalic acid, the compounds of the present invention described in Table VIII were obtained.

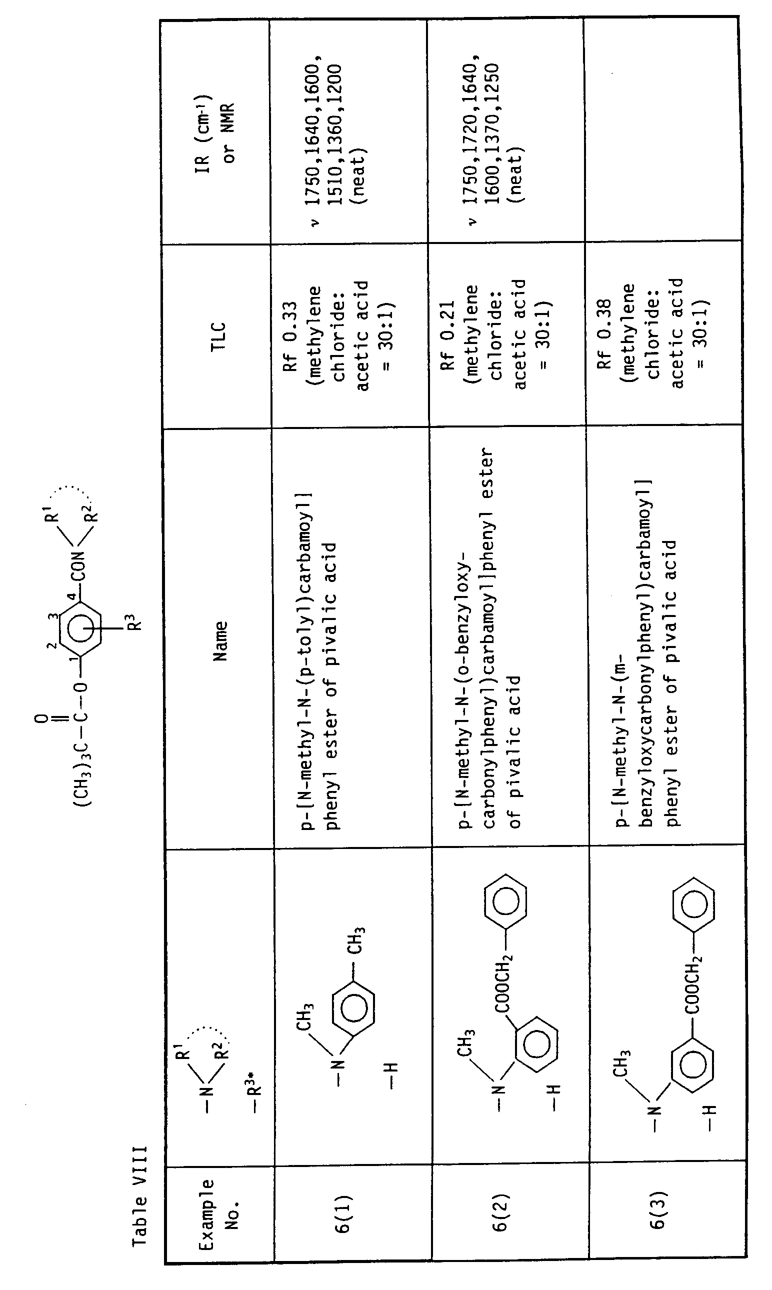

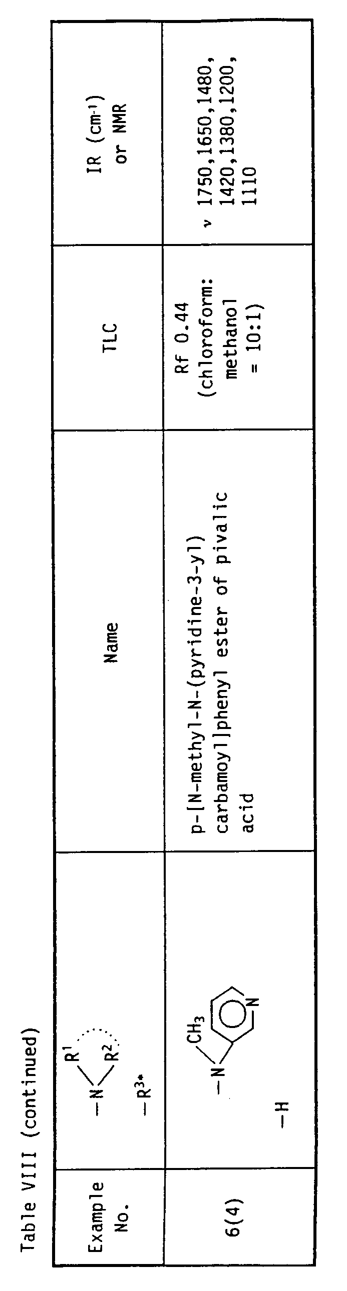

### Example 7

### 3-hydroxy-4-[(N-methyl-N-phenyl)carbamoyl phenyl ester of pivalic acid

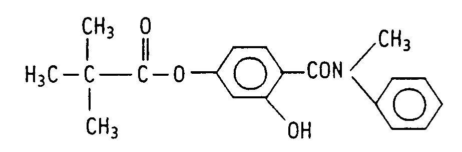

The mixture of methanol (5 ml) and triethylamine (0.3 ml) of the compound (83 mg) of the present invention obtained by procedure of Example 2 (112) was stirred for 3 hours at room temperature.

The reaction solution was extrated with ether, and the extract was washed with succesive, 1N-HCℓ, water, saturated an aqueous solution of sodium chloride.

The solution was dried with sodium sulfate, and distilled off under reduced pressure to give the title compound (68 mg) having the following physical data.

TLC : Rf 0.34 (methylene chloride : ethyl acetate = 30 :1);

NMR (CDCℓ3) : 6 7.0-7.4(6H, m), 6.65(1 H,d), 6.1(1H,2d), 3.5(3H,s), 1.3(9H,s).

### [Preparative Example]

The following components were admixed in conventional manner and the mixture punched out to obtain 100 tablets each containing 50 mg of active ingredient.

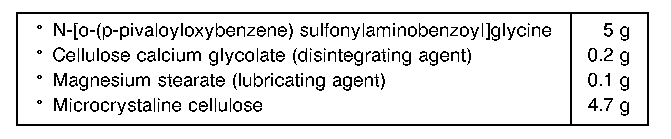

## Claims (Claims for the following Contracting State(s) : AT, BE, CH, DE, FR, GB, IT, LI, LU, NL, SE)

1. A pivalic acid ester derivative of the general formula:

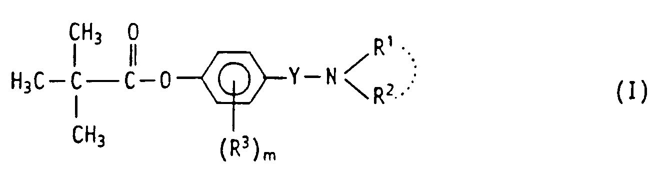

wherein Y represents a sulfonyl (-S0₂-) group or carbonyl 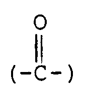 group,

(i) R¹ and R² each independently represents, a hydrogen atom; an alkyl group of up to 16 carbon atoms or an alkyl group of up to 16 carbon atoms substituted by a carboxy (-COOH) group; a group of the formula: 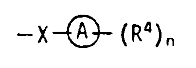 wherein X represents a single bond, a sulfonyl group, an alkylene group of up to 4 carbon atoms or an alkylene group of up to 4 carbon atoms substituted by a carboxy or benzyloxycarbonyl 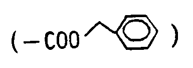 group,
Ⓐ represents a carbocyclic ring or heterocyclic ring,
n represents an integer of 1 to 5;
the groups R⁴ represent independently, a hydrogen atom or an alkyl group of up to 8 carbon atoms; an alkoxy group of up to 14 carbon atoms;
an alkylthio group of up to 6 carbon atoms;
a hydroxy group, halogen atom, nitro group or trihalomethyl group;
a group of the formula -NR⁴¹R⁴²
wherein R⁴¹ and R⁴² each represents, independently, a hydrogen atom or an alkyl group of up to 4 carbon atoms;
a tetrazole group;
a sulfonic acid (-SO₃H) group or
hydroxymethyl (-CH₂0H) group;
a group of the formula: ^{-S02} NR4 R⁴²
wherein R⁴¹ and R⁴² are as hereinbefore defined;
a group of the formula: -Z⁴¹-COOR⁴³
wherein Z⁴¹ represents a single bond, an alkylene group of up to 4 carbon atoms or an alkenylene group of from 2 to 4 carbon atoms and
R⁴³ represents a hydrogen atom, an alkyl group of up to 4 carbon atoms or a benzyl group;
a group of the formula -CONR⁴¹R⁴²
wherein R⁴¹ and R⁴² are as hereinbefore defined;
a group of the formula -COO-Z⁴²⁻COO_{R}⁴³
wherein Z⁴² represents an alkylene group of up to 4 carbon atoms, and
R⁴³ is as hereinbefore defined;
a group of the formula: -COO-Z⁴²⁻CONR⁴¹R⁴²
wherein Z⁴², R⁴¹ and R⁴² are as hereinbefore defined;
a group of the formula: -OCO-R⁴⁵
wherein R⁴⁵ represents an alkyl group of up to 8 carbon atoms or a p-guanidinophenyl group; a group of the formula: -CO-R⁴⁶
wherein R⁴⁶ represents an alkyl group of up to 4 carbon atoms;
a group of the formula: -O-Z⁴³-COOR⁴⁵⁰
wherein Z⁴³ represents an alkylene group of up to 6 carbon atoms, and
R⁴⁵⁰ represents a hydrogen atom, an alkyl group of up to 8 carbon atoms or a p-guanidinophenyl group;
a group of the formula: 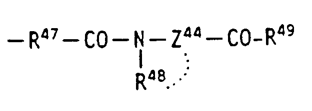 wherein 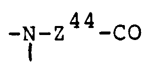 represents an amino acid residue,
R⁴⁷ represents a single-bond or an alkyl group of up to 4 carbon atom, R⁴⁸ represents a hydrogen atom or an alkyl group of up to 4 carbon atoms, and R⁴⁹ represents a hydroxy group, alkoxy group of up to 4 carbon atoms, amino group, amino group substituted by one or two alkyl groups of up to 4 carbon atoms, carbamoylmethoxy or carbamoylmethoxy group substituted by one or two alkyl groups of up to 4 carbon atoms at the nitrogen atom of the carbamoyl group, or wherein 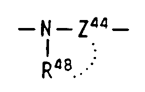 represents a heterocyclic ring containing 4 to 7 atoms in the ring including the nitrogen atom to which Z⁴⁴ and R⁴⁸ are attached and R⁴⁷ and R⁴⁹ each have the same meaning as described hereinbefore, or
(ii) R¹, R² and the nitrogen atom bonded to R¹ and R² together represent a heterocyclic ring containing at least one nitrogen atom and substituted by -COOH, or an unsubstituted heterocyclic ring containing at least one nitrogen atom, and R³ represents
(1) a hydrogen atom;
(2) a hydroxy group;
(3) an alkyl group of up to 6 carbon atoms;
(4) a halogen atom;
(5) an alkoxy group of up to 4 carbon atoms or
(6) an acyloxy group of 2 to 5 carbon atoms,
and m represents an integer of up to 4, and pharmaceutically acceptable salts thereof.

2. A compound according to claim 1, wherein Y is sulfonyl (-SO₂-).

3. A compound according to claim 1 or 2, wherein Ⓐ is a carbocyclic ring.

4. A compound according to claim 1 or 2, wherein Ⓐ is a phenyl group.

5. A compound according to any one of the preceding claims wherein one of R⁴ is an amino acid-residue.

6. A compound according to claim 5, wherein the amino acid-residue R⁴ is a glycine or alanine residue.

7. A compound according to claim 6, which is
N-[o-(p-pivaloyloxybenzene)sulfonylaminobenzoyl]glycine,
N-[2-(p-pivaloyloxybenzene)sulfonylamino-5-chlorobenzoyl]glycine,
N-[5-methylthio-2-(p-pivaloyloxybenzene)sulfonylaminobenzoyl] glycine,
N-[2-(p-pivaloyloxybenzene)sulfonylamino-5-propylthiobenzoyl] glycine,
N-[5-methyl-2-(p-pivaloyloxybenzene)sulfonylaminobenzoyl]glycine,
N-[o-(p-pivaloyloxybenzene)sulfonylaminobenzoyl]glycine methyl ester
N-[o-(3-methyl-4-pivaloyloxybenzene)sulfonylaminobenzoyl]-dl-alanine,
N-[o-(3-methyl-4-pivaloyloxybenzene)sulfonylaminobenzoyl]-β-alanine,
N-[o-(3-methyl-4-pivaloyloxybenzene)sulfonylaminobenzoyl]-ⁱ⁻alanine,
N-[5-chloro-2-(3-methyl-4-pivaloyloxybenzene)sulfonylaminobenzoyl]-1-alanine or
N-[5-chloro-2-(3-methyl-4-pivaloyloxybenzene)sulfonylaminobenzoyl]-β-alanine.

8. A process for the preparation of a pivalic acid derivative of the general formula: 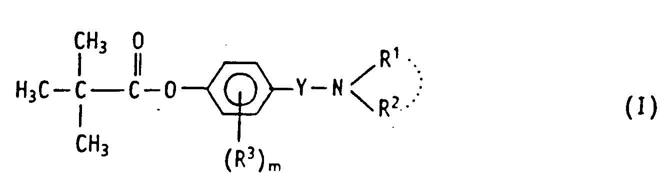 wherein R¹, R², R³ and m are as defined in claim 1, which comprises esterifying a compound of the general formula: 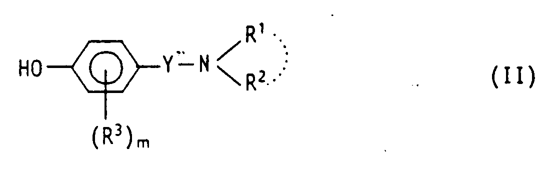 wherein R¹, R², R³ and m are as defined in claim 1, forming an amide bond by reacting a compound of the general formula: 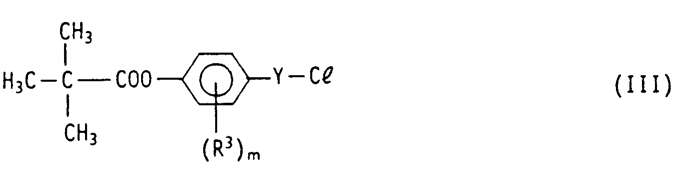 wherein R³, Y and m are as defined in claim 1, with an amine of formula HNR¹R², wherein R¹ and R² are as defined in claim 1, removing by hydrogenolysis a benzyl group from a compound of the general formula: 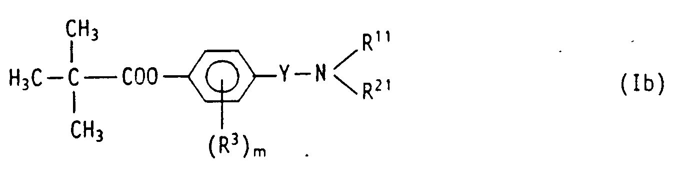 wherein R¹¹ and R²¹ are as defined in claim 1 for R¹ and R² provided that at least one of R¹¹ and R²¹ represents a group containing a benzyloxycarbonyl group, and the other symbols are as defined in claim 1, by N-alkylation of a compound of the general formula: 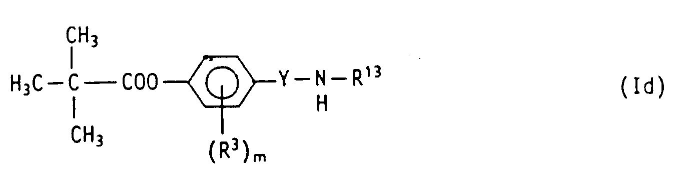 wherein R¹³ is as defined in claim 1 for R¹ and R² except for hydrogen and R³, Y and m are as defined in claim 1, or by deacylation of a compound of the general formula: 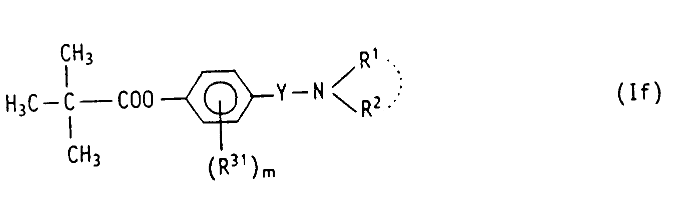 wherein R³¹ represents an acyloxy group, and R', R², Y and m are as defined in claim 1.

9. A pharmaceutical composition which comprises, as active ingredient, a pivalic acid ester derivative of the general formula (I) defined in claim 1, or a pharmaceutically acceptable salt thereof, in association with a pharmaceutically acceptable carrier or coating.

10. A compound as claimed in claim 1, for use in the prevention and/or treatment of pulmonary emphysema, atherosclerosis or rheumatoid arthritis.

## Claims (Claims for the following Contracting State(s) : ES, GR)

1. A process for the preparation of a pivalic acid ester derivative of the general formula: 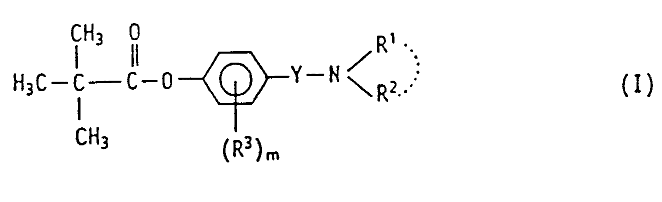 wherein Y represents a sulfonyl (-S0₂-) group or carbonyl 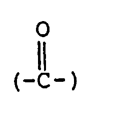 group,
(i) R¹ and R² each independently represents, a hydrogen atom;
an alkyl group of up to 16 carbon atoms or an alkyl group of up to 16 carbon atoms substituted by a carboxy (-COOH) group;
a group of the formula: 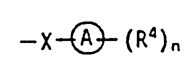 wherein X represents a single bond, a sulfonyl group, an alkylene group of up to 4 carbon atoms or an alkylene group of up to 4 carbon atoms substituted by a carboxy or benzyloxycarbonyl 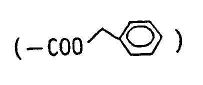 group,
@ represents a carbocyclic ring or heterocyclic ring,
n represents an integer of 1 to 5;
the groups R⁴ represent independently, a hydrogen atom or an alkyl group of up to 8 carbon atoms;
an alkoxy group of up to 14 carbon atoms;
an alkylthio group of up to 6 carbon atoms;
a hydroxy group, halogen atom, nitro group or trihalomethyl group;
a group of the formula -NR^{4l}_{R}⁴2
wherein R⁴¹ and R⁴² each represents, independently, a hydrogen atom or an alkyl group of up to 4 carbon atoms;
a tetrazole group;
a sulfonic acid (-SO₃H) group or
hydroxymethyl (-CH₂0H) group;
a group of the formula: ^{-S02} NR4 R42
wherein R⁴¹ and R⁴² are as hereinbefore defined;
a group of the formula: -Z⁴¹-COOR⁴³
wherein Z⁴¹ represents a single bond, an alkylene group of up to 4 carbon atoms or an alkenylene group of from 2 to 4 carbon atoms and
R⁴³ represents a hydrogen atom, an alkyl group of up to 4 carbon atoms or a benzyl group;
a group of the formula -CONR⁴¹R⁴²
wherein R⁴¹ and R⁴² are as hereinbefore defined;
a group of the formula -COO-Z⁴²⁻COO_{R}⁴³
wherein Z⁴² represents an alkylene group of up to 4 carbon atoms, and
R⁴³ is as hereinbefore defined;
a group of the formula: -COO-Z⁴²-CONR⁴¹R⁴²
wherein Z⁴², R⁴¹ and R⁴² are as hereinbefore defined;
a group of the formula: -OCO-R⁴⁵
wherein R⁴⁵ represents an alkyl group of up to 8 carbon atoms or a p-guanidinophenyl group;
a group of the formula: -CO-R⁴⁶
wherein R⁴⁶ represents an alkyl group of up to 4 carbon atoms;
a group of the formula: -O-Z⁴³-COOR^{45o}
wherein Z⁴³ represents an alkylene group of up to 6 carbon atoms, and
R⁴⁵⁰ represents a hydrogen atom, an alkyl group of up to 8 carbon atoms or a p-guanidinophenyl group;
a group of the formula: 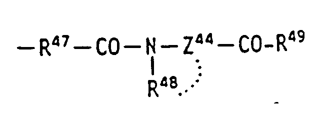 wherein 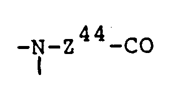 represents an amino acid residue,
R⁴⁷ represents a single-bond or an alkyl group of up to 4 carbon atoms, R⁴⁸ represents a hydrogen atom or an alkyl group of up to 4 carbon atoms, and R⁴⁹ represents a hydroxy group, alkoxy group of up to 4 carbon atoms, amino group, amino group substituted by one or two alkyl groups of up to 4 carbon atoms, carbamoylmethoxy or carbamoylmethoxy group substituted by one or two alkyl groups of up to 4 carbon atoms at the nitrogen atom of the carbamoyl group, or wherein 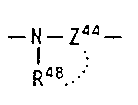 represents a heterocyclic ring containing 4 to 7 atoms in the ring including the nitrogen atom to which Z⁴⁴ and R⁴⁸ are attached and R⁴⁷ and R⁴⁹ each have the same meaning as described hereinbefore, or
(ii) R¹, R² and the nitrogen atom bonded to R¹ and R² together represent a heterocyclic ring containing at least one nitrogen atom and substituted by -COOH, or an unsubstituted heterocyclic ring containing at least one nitrogen atom,
and R³ represents
(1) a hydrogen atom;
(2) a hydroxy group;
(3) an alkyl group of up to 6 carbon atoms;
(4) a halogen atom;
(5) an alkoxy group of up to 4 carbon atoms or
(6) an acyloxy group of 2 to 5 carbon atoms,
and m represents an integer of up to 4, and pharmaceutically acceptable salts thereof, which process comprises esterifying a compound of the general formula: 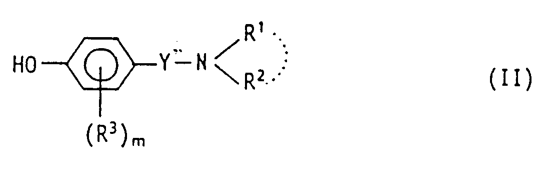
wherein R¹, R², R³ and m are as hereinbefore defined, forming an amide bond by reacting a compound of the general formula: 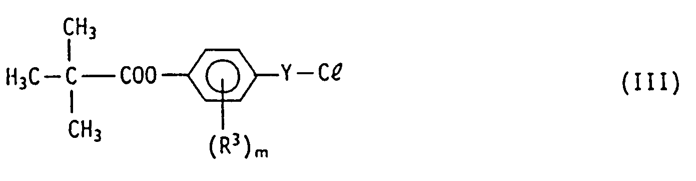 wherein R³, Y and m are as hereinbefore defined, with an amine of formula HNR¹R², wherein R¹ and R² are as hereinbefore defined, removing by hydrogenolysis a benzyl group from a compound of the general formula: 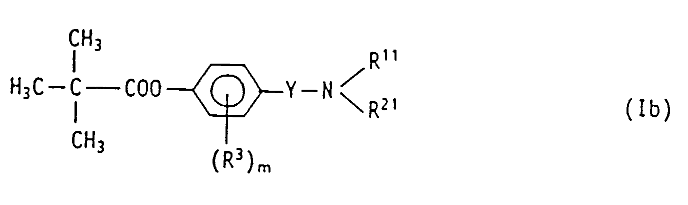 wherein R¹¹ and R²¹ are as hereinbefore defined for R¹ and R² provided that at least one of R¹¹ and R²¹ represents a group containing a benzyloxycarbonyl group, and the other symbols are as hereinbefore defined, by N-alkylation of a compound of the general formula: 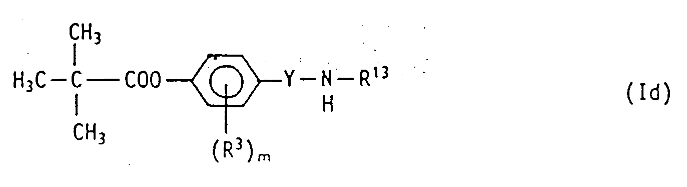 wherein R¹³ is as hereinbefore defined for R¹ and R² except for hydrogen and R³, Y and m are as hereinbefore defined, or by deacylation of a compound of the general formula: 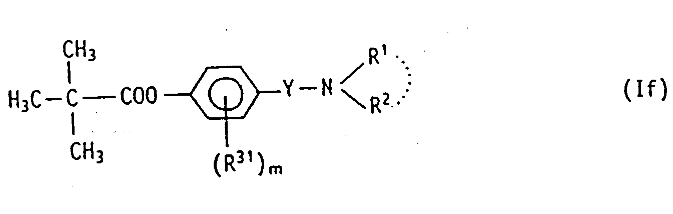 wherein R³¹ represents an acyloxy group, and R¹, R², Y and m are as hereinbefore defined, and optionally converting a pivalic acid ester derivative thus obtained into a pharmaceutically acceptable salt thereof.

2. A process according to claim 1, wherein Y is sulfonyl (-SO₂-).

3. A process according to claim 1 or 2, wherein Ⓐ is a carbocylic ring.

4. A process according to claim 1 or 2, wherein Ⓐ is a phenyl group.

5. A process according to any one of the preceding claims, wherein one of R⁴ is an amino acid-residue.

6. A process according to claim 5, wherein the amino acid-residue R⁴ is a glycine or alanine residue.

7. A process according to claim 6, for the preparation of a compound which is
N-[o-(p-pivaloyloxybenzene)sulfonylaminobenzoyl]glycine,
N-[2-(p-pivaloyloxybenzene)sulfonylamino-5-chlorobenzoyl]-glycine,
N-[5-methylthio-2-(p-pivaloyloxybenzene)sulfonylaminobenzoyl] glycine,
N-[2-(p-pivaloyloxybenzene)sulfonylamino-5-propylthiobenzoyl] glycine,
N-[5-methyl-2-(p-pivaloyloxybenzene)sulfonylaminobenzoyl]glycine,
N-[o-(p-pivaloyloxybenzene)sulfonylaminobenzoyl]glycine methyl ester
N-[o-(3-methyl-4-pivaloyloxybenzene)sulfonylaminobenzoyl]-dl-alanine,
N-[o-(3-methyl-4-pivaloyloxybenzene)sulfonylaminobenzoyl]-β-alanine,
N-[o-(3-methyl-4-pivaloyloxybenzene)sulfonylaminobenzoyl]-ⁱ⁻alanine,
N-[5-chloro-2-(3-methyl-4-pivaloyloxybenzene)sulfonylaminobenzoyl]-1-alanine or
N-[5-chloro-2-(3-methyl-4-pivaloyloxybenzene)sulfonylaminobenzoyl]-β-alanine.

8. A process according to any one of the preceding claims followed by the formulation, as active ingredient, of a pivalic acid ester derivative of the general formula (I), or a pharmaceutically acceptable salt thereof, thus obtained with a pharmaceutically acceptable carrier or coating.

## Patentansprüche (Patentansprüche für folgende(n) Vertragsstaat(en) : AT, BE, CH, DE, FR, GB, IT, LI, LU, NL, SE)

1. Pivalinsäureesterderivat der allgemeinen Formel: 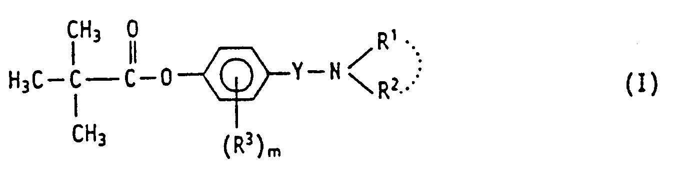 worin bedeuten:
Y eine Sulfonyl (-S0₂-)-Gruppe oder Carbonyl 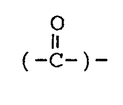 Gruppe;
(i) R¹ und R² jeweils unabhängig voneinander ein Wasserstoffatom; eine Alkylgruppe mit bis zu 16 Kohlenstoffatomen oder eine durch eine Carboxy (-COOH)-Gruppe substituierte Alkylgruppe mit bis zu 16 Kohlenstoffatomen; eine Gruppe der Formel: 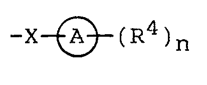 mit X gleich einer Einfachbindung, einer Sulfonylgruppe einer Alkylengruppe mit bis zu 4 Kohlenstoffatomen oder einer durch eine Carboxy- oder Benzyloxycarbonyl 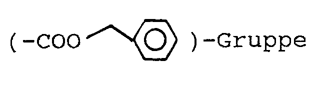 substituierten Alkylengruppe mit bis zu 4 Kohlenstofatomen, Ⓐ gleich einem carbocyclischen Ring oder heterocyclischen Ring, n gleich einer ganzen Zahl von 1 bis 5 und den Gruppen R⁴ jeweils unabhängig gleich einem Wasserstoffatom oder einer Alkylgruppe mit bis zu 8 Kohlenstoffatomen, einer Alkoxygruppe mit bis zu 14 Kohlenstoffatomen, einer Alkylthiogruppe mit bis zu 6 Kohlenstoffatomen, einer Hydroxygruppe, einem Halogenatom, einer Nitrogruppe oder einer Trihalogenmethylgruppe, einer Gruppe der Formel -NR⁴'R⁴², worin R⁴¹ und R⁴² jeweils unabhängig voneinander für ein Wasserstoffatom oder eine Alkylgruppe mit bis zu 4 Kohlenstoffatomen stehen, einer Tetrazolgruppe, einer Sulfonsäure (-S0₃H)-Gruppe oder Hydroxymethyl (-CH₂0H)-Gruppe, einer Gruppe der Formel: -SO₂NR⁴¹R⁴², worin R⁴¹ und R⁴² die zuvor angegebene Bedeutung besitzen, einer Gruppe der Formel:-Z⁴¹⁻COOR⁴³, worin Z⁴¹ eine Einfachbindung, eine Alkylengruppe mit bis zu 4 Kohlenstoffatomen oder eine Alkenylengruppe mit 2 bis 4 Kohlenstoffatomen darstellt und R⁴³ ein Wasserstoffatom, eine Alkylgruppe mit bis zu 4 Kohlenstoffatomen oder eine Benzylgruppe bedeu- tₑt, einer Gruppe der Formel -CONR⁴¹R⁴², worin R⁴¹ und R⁴² die zuvor angegebene Bedeutung besitzen, einer Gruppe der Formel -COO-Z⁴²⁻COOR⁴³, worin Z⁴² eine Alkylengruppe mit bis zu 4 Kohlenstoffatomen darstellt und R⁴³ die zuvor angegebene Bedeutung besitzt, einer Gruppe der Formel: -COO-Z⁴²⁻CONR⁴¹R⁴², worin Z⁴², R⁴¹ und R⁴² die zuvor angegebene Bedeutung besitzen, einer Gruppe der Formel: -OCO-R⁴⁵, worin R⁴⁵ eine Alkylgruppe mit bis zu 8 Kohlenstoffatomen oder eine p-Guanidinophenylgruppe darstellt, einer Gruppe der Formel -CO-R⁴⁶, worin R⁴⁶ eine Alkylgruppe mit bis zu 4 Kohlenstoffatomen bedeutet, einer Gruppe der Formel: -O-Z⁴³⁻COOR⁴⁵°, worin Z⁴³ eine Alkylengruppe mit bis zu 6 Kohlenstoffatomen darstellt und R^{45o} für ein Wasserstoffatom, eine Alkylgruppe mit bis zu 8 Kohlenstoffatomen oder eine p-Guanidinophenylgruppe steht, einer Gruppe der Formel 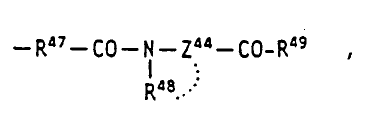 worin 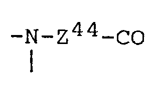 einen Aminosäurerest darstellt, R⁴⁷ für eine Einfachbindung oder eine Alkylgruppe mit bis zu 4 Kohlenstoffatomen steht, R⁴⁸ ein Wasserstoffatom oder eine Alkylgruppe mit bis zu 4 Kohlenstoffatomen darstellt und R⁴⁹ einer Hydroxygruppe, Alkoxygruppe mit bis zu 4 Kohlenstoffatomen, Aminogruppe, ein- oder zweifach alkyl (mit bis zu 4 Kohlenstoffatomen)-substituierten Aminogruppe, einer Carbamoylmethoxygruppe oder einer am Stickstoffatom der Carbamoylgruppe ein- oder zweifach alkyl (mit bis zu 4 Kohlenstoffatomen)-substituierten Carbamoylmethoxygruppe entspricht oder worin 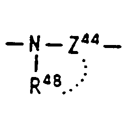 einen heterocyclischen Ring mit 4 bis 7 Atomen im Ring einschließlich des Stickstoffatoms, an dem Z⁴⁴ und R⁴⁸ hängen,und R⁴⁷ und R⁴⁹ die zuvor angegebene Bedeutung besitzen, oder
(ii) R¹, R² und das an R¹ und R² gebundene Stickstoffatom zusammen einen mindestens ein Stickstoffatom enthaltenden und durch -COOH substituierten heterocyclischen Ring oder einenmindestens ein Stickstoffatom enthaltenden unsubstituierten heterocyclischen Ring; R³
(1) ein Wasserstoffatom,
(2) eine Hydroxygruppe,
(3) eine Alkylgruppe mit bis zu 6 Kohlenstoffatomen,
(4) ein Halogenatom,
(5) eine Alkoxygruppe mit bis zu 4 Kohlenstoffatomen oder
(6) eine Acyloxygruppe mit 2 bis 5 Kohlenstoffatomen, und
m eine ganze Zahl von bis zu 4, oder ein pharmazeutisch akzeptables Salz desselben.

2. Verbindung nach Anspruch 1, worin Y für Sulfonyl (-SO₂-) steht.

3. Verbindung nach Anspruch 1 oder 2, worin Ⓐ für einen carbocyclischen Ring steht.

4. Verbindung nach Anspruch 1 oder 2, worin Ⓐ für eine Phenylgruppe steht.

5. Verbindung nach einem der vorhergehenden Ansprüche, worin einer der Reste R⁴ für einen Aminosäurerest steht.

6. Verbindung nach Anspruch 5, worin der Aminosäurerest R⁴ aus einem Glycin- oder Alaninrest besteht.

7. Verbindung nach Anspruch 6, nämlich
N-[o-(p-Pivaloyloxybenzol)-sulfonylaminobenzoyl]-glycin,
N-[2-(p-Pivaloyloxybenzol)-sulfonylamino-5-chlorbenzoyl]-glycin,
N-[5-Methylthio-2-(p-pivaloyloxybenzol)-sulfonylaminobenzoyl]-glycin,
N-[2-(p-Pivaloyloxybenzol)-sulfonylamino-5-propylthiobenzoyl]-glycin,
N-[5-Methyl-2-(p-pivaloyloxybenzol)-sulfonylaminobenzoyl]-glycin,
N-[o-(p-Pivaloyloxybenzol)-sulfonylaminobenzoyl]-glycinmethylester,
N-[o-(3-Methyl-4-pivaloyloxybenzol)-sulfonylaminobenzoyl]-dl-alanin,
N-[o-(3-Methyl-4-pivaloyloxybenzol)-sulfonylaminobenzoyl]-β-alanin,
N-[o-(3-Methyl-4-pivaloyloxybenzol)-sulfonylaminobenzoyl]-1-alanin,
N-[5-Chlor-2-(3-methyl-4-pivaloyloxybenzol)-sulfonylaminobenzoyl]-1-alanin oder
N-[5-Chlor-2-(3-methyl-4-pivaloyloxybenzol)-sulfonylaminobenzoyl]-β-alanin.

8. Verfahren zur Herstellung eines Pivalinsäurederivats der allgemeinen Formel: 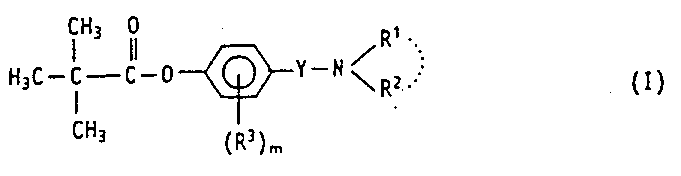 worin R¹, R², R³ und m die in Anspruch 1 angegebene Bedeutung besitzen, durch Verestern einer Verbindung der allgemeinen Formel: 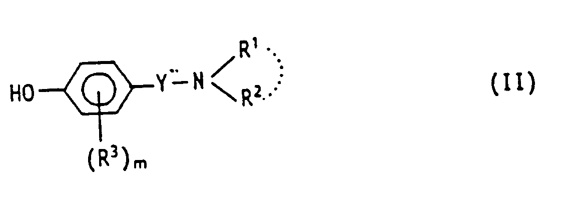 worin R¹, R², R³ und m die in Anspruch 1 angegebene Bedeutung besitzen, Ausbilden einer Amidbindung durch Umsetzen einer Verbindung der allgemeinen Formel: 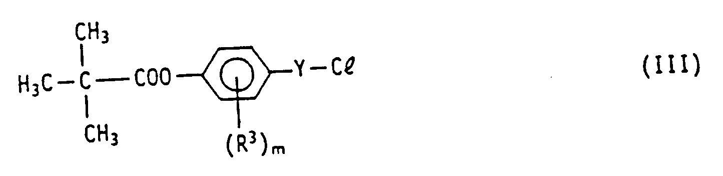 worin R³ Y und m die in Anspruch 1 angegebene Bedeutung besitzen, mit einem Amin der Formel HNR¹R², worin R¹ und R² die in Anspruch 1 angegebene Bedeutung besitzen, hydrogenolytische Entfernung einer Benzylgruppe aus einer Verbindung der allgemeinen Formel: 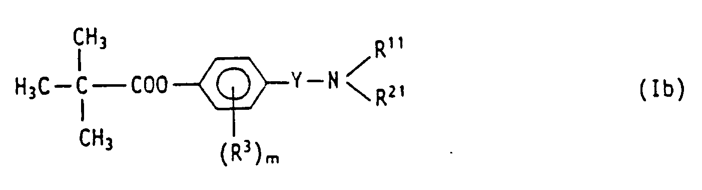 worin R¹¹ und R²¹ die in Anspruch 1 für R¹ und R² angegebene Bedeutung besitzen, wobei gilt, daß mindestens einer der Reste R¹¹ und R²¹ für eine eine Benzyloxycarbonylgruppe enthaltende Gruppe steht, und die anderen Symbole die in Anspruch 1 angegebene Bedeutung besitzen, durch N-Alkylierung einer Verbindung der allgemeinen Formel: 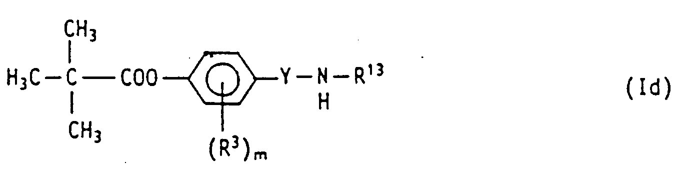 worin R¹³ die in Anspruch 1 für R¹ und R² angegebene Bedeutung (mit Ausnahme von Wasserstoff) besitzt und R³ Y und m die in Anspruch 1 angegebene Bedeutung besitzen, oder durch Deacylierung einer Verbindung der allgemeinen Formel: 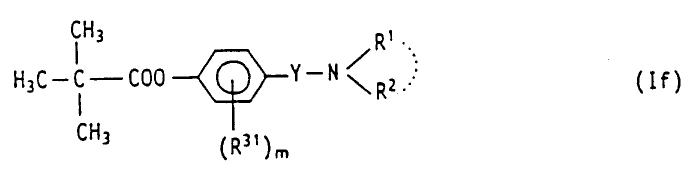 worin R³¹ für eine Acyloxygruppe steht und R¹, R², Y und m die in Anspruch 1 angegebene Bedeutung besitzen.

9. Arzneimittel mit einem Pivalinsäureesterderivat der allgemeinen Formel (I) in der Definition gemäß Anspruch 1 oder einem pharmazeutisch akzeptablen Salz desselben als aktivem Bestandteil in Verbindung mit einem pharmazeutisch akzeptablen Träger oder Überzug.

10. Verbindung nach Anspruch 1 zur Verwendung bei der Verhinderung und/oder Behandlung von Lungenerweiterung, Arteriosklerose oder rheumatischer Arthritis.

## Patentansprüche (Patentansprüche für folgende(n) Vertragsstaat(en) : ES, GR)

1. Verfahren zur Herstellung eines Pivalinsäureesterderivats der allgemeinen Formel: 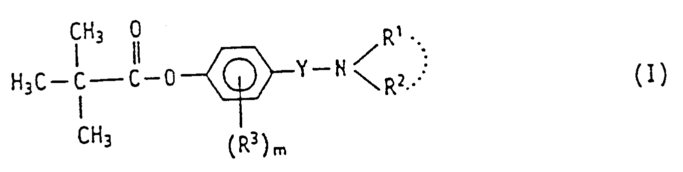 worin bedeuten: Y eine Sulfonyl (-S0₂-)-Gruppe oder Carbonyl 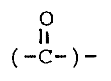 Gruppe;
(i) R¹ und R² jeweils unabhängig voneinander ein Wasserstoffatom; eine Alkylgruppe mit bis zu 16 Kohlenstoffatomen oder eine durch eine Carboxy (-COOH)-Gruppe substituierte Alkylgruppe mit bis zu 16 Kohlenstoffatomen; eine Gruppe der Formel: 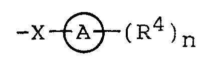 mit X gleich einer Einfachbindung, einer Sulfonylgruppe einer Alkylengruppe mit bis zu 4 Kohlenstoffatomen oder einer durch eine Carboxy- oder Benzyloxycarbonyl 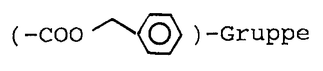 substituierten Alkylengruppe mit bis zu 4 Kohlenstofatomen, Ⓐ gleich einem carbocyclischen Ring oder heterocyclischen Ring, n gleich einer ganzen Zahl von 1 bis 5 und den Gruppen R⁴ jeweils unabhängig gleich einem Wasserstoffatom oder einer Alkylgruppe mit bis zu 8 Kohlenstoffatomen, einer Alkoxygruppe mit bis zu 14 Kohlenstoffatomen, einer Alkylthiogruppe mit bis zu 6 Kohlenstoffatomen, einer Hydroxygruppe, einem Halogenatom, einer Nitrogruppe oder einer Trihalogenmethylgruppe, einer Gruppe der Formel -NR⁴'R⁴², worin R⁴¹ und R⁴² jeweils unabhängig voneinander für ein Wasserstoffatom oder eine Alkylgruppe mit bis zu 4 Kohlenstoffatomen stehen, einer Tetrazolgruppe, einer Sulfonsäure (-S0₃H)-Gruppe oder Hydroxymethyl (-CH₂0H)-Gruppe, einer Gruppe der Formel: -SO₂NR⁴¹R⁴², worin R⁴¹ und R⁴² die zuvor angegebene Bedeutung besitzen, einer Gruppe der Formel: -Z⁴¹⁻COOR⁴³, worin Z⁴¹ eine Einfachbindung, eine Alkylengruppe mit bis zu 4 Kohlenstoffatomen oder eine Alkenylengruppe mit 2 bis 4 Kohlenstoffatomen darstellt und R⁴³ ein Wasserstoffatom, eine Alkylgruppe mit bis zu 4 Kohlenstoffatomen oder eine Benzylgruppe bedeu- tₑt, einer Gruppe der Formel -CONR⁴¹R⁴², worin R⁴¹ und R⁴² die zuvor angegebene Bedeutung besitzen, einer Gruppe der Formel: -COO-Z⁴²⁻COOR⁴³, worin Z⁴² eine Alkylengruppe mit bis zu 4 Kohlenstoffatomen darstellt und R⁴³ die zuvor angegebene Bedeutung besitzt, einer Gruppe der Formel: -COO-Z⁴²⁻CONR⁴¹R⁴², worin Z⁴², R⁴¹ und R⁴² die zuvor angegebene Bedeutung besitzen, einer Gruppe der Formel: -OCO-R⁴⁵, worin R⁴⁵ eine Alkylgruppe mit bis zu 8 Kohlenstoffatomen oder eine p-Guanidinophenylgruppe darstellt, einer Gruppe der Formel: -CO-R⁴⁶, worin R⁴⁶ eine Alkylgruppe mit bis zu 4 Kohlenstoffatomen bedeutet, einer Gruppe der Formel: -O-Z⁴³⁻COOR⁴⁵°, worin Z⁴³ eine Alkylengruppe mit bis zu 6 Kohlenstoffatomen darstellt und R450 für ein Wasserstoffatom, eine Alkylgruppe mit bis zu 8 Kohlenstoffatomen oder eine p-Guanidinophenylgruppe steht, einer Gruppe der Formel 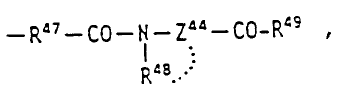 worin 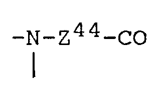 einen Aminosäurerest darstellt, R⁴⁷ für eine Einfachbindung oder eine Alkylgruppe mit bis zu 4 Kohlenstoffatomen steht, R⁴⁸ ein Wasserstoffatom oder eine Alkylgruppe mit bis zu 4 Kohlenstoffatomen darstellt und R⁴⁹ einer Hydroxygruppe, Alkoxygruppe mit bis zu 4 Kohlenstoffatomen, Aminogruppe, ein- oder zweifach alkyl (mit bis zu 4 Kohlenstoffatomen)-substituierten Aminogruppe, einer Carbamoylmethoxygruppe oder einer am Stickstoffatom der Carbamoylgruppe ein- oder zweifach alkyl (mit bis zu 4 Kohlenstoffatomen)-substituierten Carbamoylmethoxygruppe entspricht oder worin 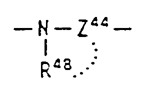 einen heterocyclischen Ring mit 4 bis 7 Atomen im Ring einschließlich des Stickstoffatoms, an dem Z⁴⁴ und R⁴⁸ hängen, und R⁴⁷ und R⁴⁹ die zuvor angegebene Bedeutung besitzen, oder
(ii) R¹, R² und das an R¹ und R² gebundene Stickstoffatom zusammen einen mindestens ein Stickstoffatom enthaltenden und durch -COOH substituierten heterocyclischen Ring oder einen mindestens ein Stickstoffatom enthaltenden unsubstituierten heterocyclischen Ring; R³
(1) ein Wasserstoffatom,
(2) eine Hydroxygruppe,
(3) eine Alkylgruppe mit bis zu 6 Kohlenstoffatomen,
(4) ein Halogenatom,
(5) eine Alkoxygruppe mit bis zu 4 Kohlenstoffatomen oder
(6) eine Acyloxygruppe mit 2 bis 5 Kohlenstoffatomen, und
m eine ganze Zahl von bis zu 4, oder eines pharmazeutisch akzeptablen Salzes desselben, durch Verestern einer Verbindung der allgemeinen Formel: 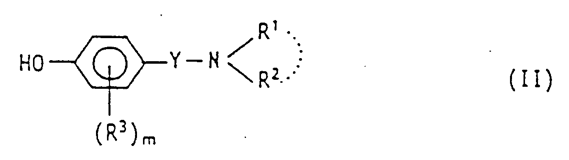
worin R¹, R², R³ und m die zuvor angegebene Bedeutung besitzen, Ausbilden einer Amidbindung durch Umsetzen einer Verbindung der allgemeinen Formel: 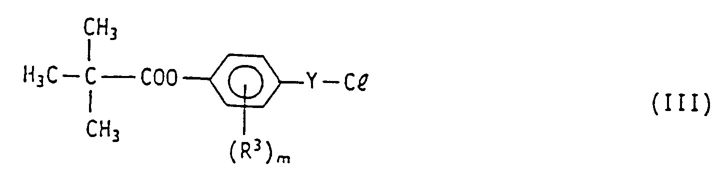 worin R³ Y und m die zuvor angegebene Bedeutung besitzen, mit einem Amin der Formel HNR¹R², worin R¹ und R² die zuvor angegebene Bedeutung besitzen, hydrogenolytische Entfernung einer Benzylgruppe aus einer Verbindung der allgemeinen Formel: 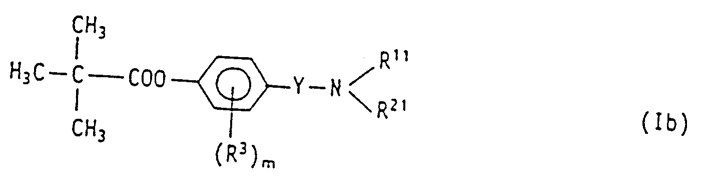 worin R¹¹ und R²¹ die zuvor für R¹ und R² angegebene Bedeutung besitzen, wobei gilt, daß mindestens einer der Reste R¹¹ und R²¹ für eine eine Benzyloxycarbonylgruppe enthaltende Gruppe steht, und die anderen Symbole die zuvor angegebene Bedeutung besitzen, durch N-Alkylierung einer Verbindung der allgemeinen Formel: 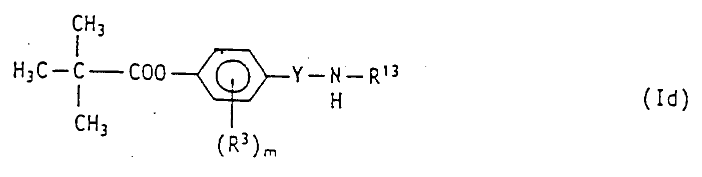 worin R¹³ die zuvor für R¹ und R² angegebene Bedeutung (mit Ausnahme von Wasserstoff) besitzt und R³ Y und m die zuvor angegebene Bedeutung besitzen, oder durch Deacylierung einer Verbindung der allgemeinen Formel: 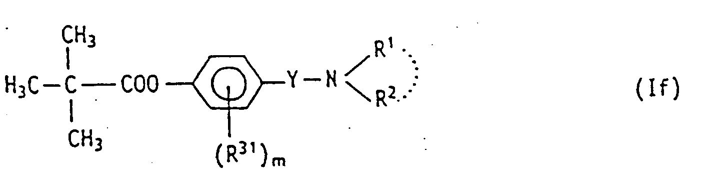 worin R³¹ für eine Acyloxygruppe steht und R¹, R², Y und m die zuvor angegebene Bedeutung besitzen.

2. Verfahren nach Anspruch 1, worin Y für Sulfonyl (-SO₂-) steht.

3. Verfahren nach Anspruch 1 oder 2, worin Ⓐ für einen carbocyclischen Ring steht.

4. Verfahren nach Anspruch 1 oder 2, worin Ⓐ für eine Phenylgruppe steht.

5. Verfahren nach einem der vorhergehenden Ansprüche, worin einer der Reste R⁴ für einen Aminosäurerest steht.

6. Verfahren nach Anspruch 5, worin der Aminosäurerest R⁴ aus einem Glycin- oder Alaninrest besteht.

7. Verfahren nach Anspruch 6 zur Herstellung einer aus
N-[o-(p-Pivaloyloxybenzol)-sulfonylaminobenzoyl]-glycin,
N-[2-(p-Pivaloyloxybenzol)-sulfonylamino-5-chlorbenzoyl]-glycin,
N-[5-Methylthio-2-(p-pivaloyloxybenzol)-sulfonylaminobenzoyl]-glycin,
N-[2-(p-Pivaloyloxybenzol)-sulfonylamino-5-propylthiobenzoyl]-glycin,
N-[5-Methyl-2-(p-pivaloyloxybenzol)-sulfonylaminobenzoyl]-glycin,
N-[o-(p-Pivaloyloxybenzol)-sulfonylaminobenzoyl]-glycinmethylester,
N-[o-(3-Methyl-4-pivaloyloxybenzol)-sulfonylaminobenzoyl]-dl-alanin,
N-[o-(3-Methyl-4-pivaloyloxybenzol)-sulfonylaminobenzoyl]-β-alanin,
N-[o-(3-Methyl-4-pivaloyloxybenzol)-sulfonylaminobenzoyl]-1-alanin,
N-[5-Chlor-2-(3-methyl-4-pivaloyloxybenzol)-sulfonylaminobenzoyl]-1-alanin oder
N-[5-Chlor-2-(3-methyl-4-pivaloyloxybenzol)-sulfonylaminobenzoyl]-β-alanin bestehenden Verbindung.

8. Verfahren nach einem der vorhergehenden Ansprüche, dem die Vereinigung eines (hierbei) erhaltenen Pivalinsäureesterderivats der allgemeinen Formel (I) oder eines pharmazeutisch akzeptablen Salzes desselben als aktivem Bestandteil mit einem pharmazeutisch akzeptablen Träger oder Überzug nachgeschaltet wird.

## Revendications (Revendications pour l'(les) Etat(s) contractant(s) suivant(s) : AT, BE, CH, DE, FR, GB, IT, LI, LU, NL, SE)

1. Un dérivé d'ester de l'acide pivalique de formule générale : 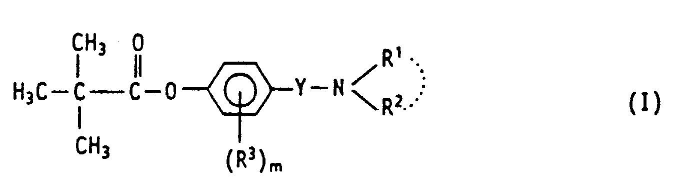 dans laquelle Y représente un groupe sulfonyle (-S0₂-) ou carbonyle 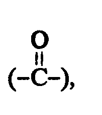 (i) R¹ et R² représentent chacun indépendamment un atome d'hydrogène ;
un groupe alkyle ayant jusqu'à 16 atomes de carbone ou un groupe alkyle ayant jusqu'à 16 atomes de carbone substitué par un groupe carboxy (-COOH); un groupe de formule : 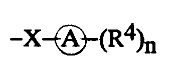 dans laquelle X représente une liaison simple, un groupe sulfonyle, un groupe alkylène ayant jusqu'à 4 atomes de carbone ou un groupe alkylène ayant jusqu'à 4 atomes de carbone substitué par un groupe carboxy ou benzyloxycarbonyle 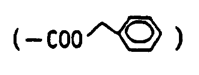 Ⓐ représente un noyau carbocyclique ou un noyau hétérocyclique, n représente un nombre entier de 1 à 5 ;
les groupes R⁴ représentent indépendamment un atome d'hydrogène ou un groupe alkyle ayant jusqu'à 8 atomes de carbone;
un groupe alcoxy ayant jusqu'à 14 atomes de carbone ;
un groupe alkylthio ayant jusqu'à 6 atomes de carbone ;
un groupe hydroxy, un atome d'halogène, un groupe nitro ou un groupe trihalométhyle ;
un groupe de formule -NR^{4l}_{R}⁴²
dans laquelle R⁴¹ et R⁴² représentent chacun indépendamment un atome d'hydrogène ou un groupe alkyle ayant jusqu'à 4 atomes de carbone ;
un groupe tétrazole ;
un groupe acide sulfonique (-SO₃H) ou
un groupe hydroxyméthyle (-CH₂0H);
un groupe de formule : -SO₂NR⁴¹R⁴²
dans laquelle R⁴¹ et R⁴² sont comme définis précédemment ;
un groupe de formule -Z⁴'-COOR⁴³
dans laquelle Z⁴¹ représente une liaison simple, un groupe alkylène ayant jusqu'à 4 atomes de carbone ou un groupe alcénylène de 2 à 4 atomes de carbone et
R⁴³ représente un atome d'hydrogène, un groupe alkyle ayant jusqu'à 4 atomes de carbone ou un groupe benzyle ;
un groupe de formule -CONR^{4l}_{R}⁴²
dans laquelle R⁴¹ et R⁴² sont comme définis précédemment ;
un groupe de formule -COO-Z⁴²⁻COO_{R}⁴3
dans laquelle Z⁴² représente un groupe alkylène ayant jusqu'à 4 atomes de carbone, et
R⁴³ est comme défini précédemment;
un groupe de formule : -COO-Z⁴²-CONR⁴¹R⁴²
dans laquelle Z⁴², R⁴¹ et R⁴² sont comme définis précédemment ;
un groupe de formule : -OCO-R⁴⁵
dans laquelle R⁴⁵ représente un groupe alkyle ayant jusqu'à 8 atomes de carbone ou un groupe p-guanidinophényle ;
un groupe de formule : -CO-R⁴⁶
dans laquelle R⁴⁶ représente un groupe alkyle ayant jusqu'à 4 atomes de carbone ;
un groupe de formule : -O-Z⁴³⁻C_{OOR}45o
dans laquelle Z⁴³ représente un groupe alkylène ayant jusqu'à 6 atomes de carbone, et
R⁴⁵⁰ représente un atome d'hydrogène, un groupe alkyle ayant jusqu'à 8 atomes de carbone ou un groupe p-guanidinophényle ;
un groupe de formule : 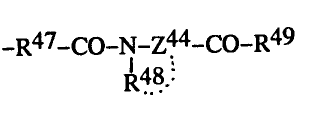 dans laquelle 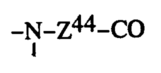 représente un résidu d'aminoacide,
R⁴⁷ représente une liaison simple ou un groupe alkyle ayant jusqu'à 4 atomes de carbone, R⁴⁸ représente un atome d'hydrogène ou un groupe alkyle ayant jusqu'à 4 atomes de carbone, et R⁴⁹ représente un groupe hydroxy, un groupe alcoxy ayant jusqu'à 4 atomes de carbone, un groupe amino, un groupe amino substitué par un ou deux groupes alkyles ayant jusqu'à 4 atomes de carbone, un groupe carbamoylméthoxy ou carbamoylméthoxy substitué par un ou deux groupes alkyles ayant jusqu'à 4 atomes de carbone au niveau de l'atome d'azote du groupe carbamoyle, ou dans laquelle 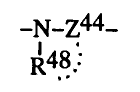 représente un noyau hétérocyclique contenant de 4 à 7 atomes dans le noyau incluant l'atome d'azote auquel Z⁴⁴ et R⁴⁸ sont attachés et R⁴⁷ et R⁴⁹ sont chacun définis comme précédemment, ou bien
(ii) R¹, R² et l'atome d'azote lié à R¹ et R² ensemble représentent un noyau hétérocyclique contenant au moins un atome d'azote et substitué par -COOH, ou bien un noyau hétérocyclique non substitué contenant au moins un atome d'azote, et R³ représente :
(1) un atome d'hydrogène ;
(2) un groupe hydroxy ;
(3) un groupe alkyle ayant jusqu'à 6 atomes de carbone ;
(4) un atome d'halogène ;
(5) un groupe alcoxy ayant jusqu'à 4 atomes de carbone ou
(6) un groupe acyloxy de 2 à 5 atomes de carbone,
et m représente un nombre entier pouvant aller jusqu'à 4, et les sels pharmaceutiquement acceptables de ce dérivé.

2. Un composé selon la revendication 1, selon laquelle Y est un groupe sulfonyle (-S0₂-).

3. Un composé selon la revendication 1 ou 2, selon laquelle Ⓐ est un noyau carbocyclique.

4. Un composé selon la revendication 1 ou 2, selon laquelle Ⓐ est un groupe phényle.

5. Un composé selon l'une quelconque des revendications précédentes, selon laquelle l'un des R⁴ est un résidu d'aminoacide.

6. Un composé selon la revendication 5, selon laquelle le résidu d'aminoacide R⁴ est un résidu de glycine ou d'alanine.

7. Un composé selon la revendication 6, qui est le suivant :
N-[o-(p-pivaloyloxybenzène)sulfonylaminobenzoyl]glycine,
N-[2-(p-pivaloyloxybenzène)sulfonylamino-5-chlorobenzoyl]glycine,
N-[5-méthylthio-2-(p-pivaloyloxybenzène)sulfonylaminobenzoyl]glycine,
N-[2-(p-pivaloyloxybenzène)sulfonylamino-5-propylthiobenzoyl]glycine,
N-[5-méthyl-2-(p-pivaloyloxybenzène)sulfonylaminobenzoyl)glycine,
N-[o-(p-pivaloyloxybenzène)sulfonylaminobenzoyl]glycine, ester méthylique
N-[o-(3-méthyl-4-pivaloyloxybenzène)sulfonylaminobenzoyl]-dl-alanine,
N-[o-(3-méthyl-4-pivaloyloxybenzène)sulfonylaminobenzoyl]-β-alanine,
N-[o-(3-méthyl-4-pivaloyloxybenzène)sulfonylaminobenzoyl]-1-alanine,
N-[5-chloro-2-(3-méthyl-4-pivaloyloxybenzène)sulfonylaminobenzoyl]-1-alanine ou
N-[5-chloro-2-(3-méthyl-4-pivaloyloxybenzène)sulfonylaminobenzoyl]-β-alanine.

8. Un procédé de préparation d'un dérivé de l'acide pivalique de formule générale : 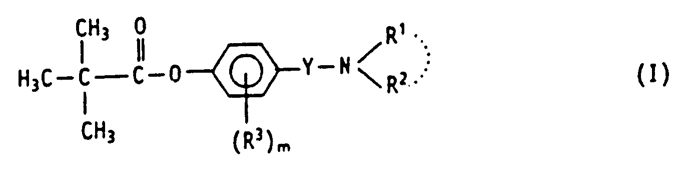 dans laquelle R¹, R², R³ et m sont comme définis dans la revendication 1, qui comprend l'estérification d'un composé de formule générale : 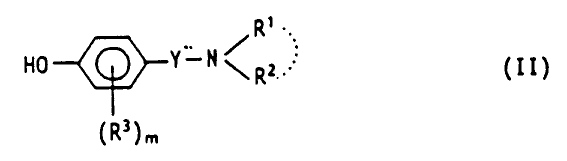 dans laquelle R¹, R², R³ et m sont comme définis dans la revendication 1, la formation d'une liaison amide par réaction d'un composé de formule générale : 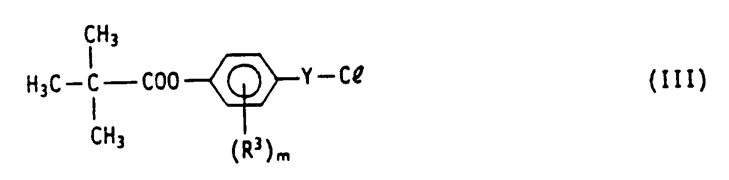 dans laquelle R³, Y et m sont comme définis dans la revendication 1, avec une amine de formule HNR¹R², où R¹ et R² sont comme définis dans la revendication 1, l'élimination par hydrogénolyse d'un groupe benzyle d'un composé de formule générale : 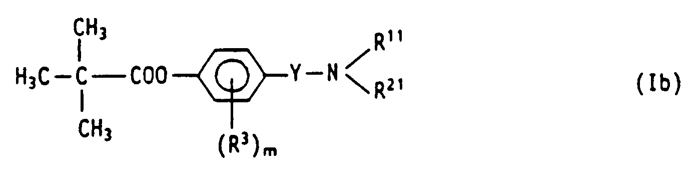 dans laquelle R¹¹ et R²¹ sont comme définis dans la revendication 1 pour R¹ et R² à la condition que au moins l'un des R¹¹ et R²¹ représente un groupe contenant un groupe benzyloxycarbonyle, et les autres symboles sont comme définis dans la revendication 1, par N-alkylation d'un composé de formule générale : 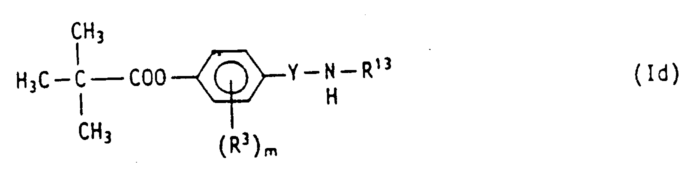 dans laquelle R¹³ est comme défini dans la revendication 1 pour R¹ et R², sauf pour l'hydrogène et R³, Y et m sont comme définis dans la revendication 1, ou par désacylation d'un composé de formule générale : 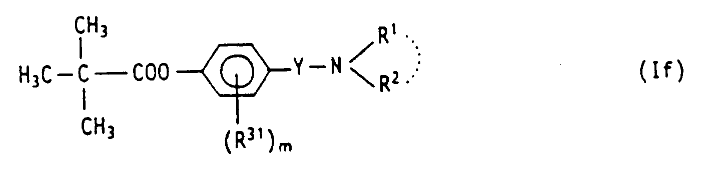 dans laquelle R³¹ représente un groupe acyloxy, et R¹, R², Y et m sont comme définis dans la revendication 1.

9. Une composition pharmaceutique qui comprend, comme ingrédient actif, un dérivé d'ester de l'acide pivalique de formule générale (I) défini dans la revendication 1, ou un sel pharmaceutiquement acceptable de ce dérivé, en association avec un support ou un enrobage pharmaceutiquement acceptable.

10. Un composé selon la revendication 1 à utiliser dans la prévention et/ou le traitement de l'emphysème pulmonaire, de l'athérosclérose ou de l'arthrite rhumatoïde.

## Revendications (Revendications pour l'(les) Etat(s) contractant(s) suivant(s) : ES, GR)

1. Un procédé de préparation d'un dérivé d'ester de l'acide pivalique de formule générale : 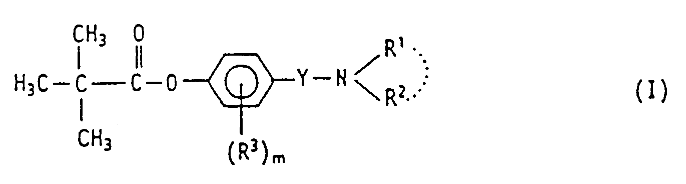 dans laquelle Y représente un groupe sulfonyle (-S0₂-) ou carbonyle 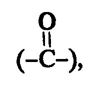 (i) R¹ et R² représentent chacun indépendamment un atome d'hydrogène ;
un groupe alkyle ayant jusqu'à 16 atomes de carbone ou un groupe alkyle ayant jusqu'à 16 atomes de carbone substitué par un groupe carboxy (-COOH);
un groupe de formule : 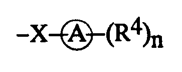 dans laquelle X représente une liaison simple, un groupe sulfonyle, un groupe alkylène ayant jusqu'à 4 atomes de carbone ou un groupe alkylène ayant jusqu'à 4 atomes de carbone substitué par un groupe carboxy ou benzyloxycarbonyle 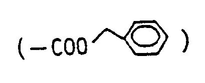 Ⓐ représente un noyau carbocyclique ou un noyau hétérocyclique,
n représente un nombre entier de 1 à 5 ;
les groupes R⁴ représentent indépendamment un atome d'hydrogène ou un groupe alkyle ayant jusqu'à 8 atomes de carbone ;
un groupe alcoxy ayant jusqu'à 14 atomes de carbone ;
un groupe alkylthio ayant jusqu'à 6 atomes de carbone ;
un groupe hydroxy, un atome d'halogène, un groupe nitro ou un groupe trihalométhyle ;
un groupe de formule -NR^{4l}_{R}⁴2
dans laquelle R⁴¹ et R⁴² représentent chacun indépendamment un atome d'hydrogène ou un groupe alkyle ayant jusqu'à 4 atomes de carbone ;
un groupe tétrazole ;
un groupe acide sulfonique (-SO₃H) ou
un groupe hydroxyméthyle (-CH₂0H) ;
un groupe de formule : -SO₂NR⁴¹R⁴²
dans laquelle R⁴¹ et R⁴² sont comme définis précédemment ;
un groupe de formule -Z⁴'-COOR⁴³
dans laquelle Z⁴¹ représente une liaison simple, un groupe alkylène ayant jusqu'à 4 atomes de carbone ou un groupe alcénylène de 2 à 4 atomes de carbone et
R⁴³ représente un atome d'hydrogène, un groupe alkyle ayant jusqu'à 4 atomes de carbone ou un groupe benzyle ;
un groupe de formule -CONR^{4l}_{R}⁴²
dans laquelle R⁴¹ et R⁴² sont comme définis précédemment ;
un groupe de formule -COO-Z⁴²⁻COO_{R}⁴3
dans laquelle Z⁴² représente un groupe alkylène ayant jusqu'à 4 atomes de carbone, et
R⁴³ est comme défini précédemment ;
un groupe de formule : -COO-Z⁴²-CONR⁴¹R⁴²
dans laquelle Z⁴², R⁴¹ et R⁴² sont comme définis précédemment ;
un groupe de formule : -OCO-R⁴⁵
dans laquelle R⁴⁵ représente un groupe alkyle ayant jusqu'à 8 atomes de carbone ou un groupe p-guanidinophényle ;
un groupe de formule : -CO-R⁴⁶
dans laquelle R⁴⁶ représente un groupe alkyle ayant jusqu'à 4 atomes de carbone ;
un groupe de formule : -O-Z⁴³⁻C_{OOR}45o
dans laquelle Z⁴³ représente un groupe alkylène ayant jusqu'à 6 atomes de carbone, et
R⁴⁵⁰ représente un atome d'hydrogène, un groupe alkyle ayant jusqu'à 8 atomes de carbone ou un groupe p-guanidinophényle ;
un groupe de formule : 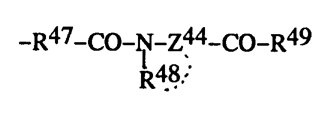 dans laquelle -N-Z⁴⁴⁻CO représente un résidu d'aminoacide,
R⁴⁷ représente une liaison simple ou un groupe alkyle ayant jusqu'à 4 atomes de carbone, R⁴⁸ représente un atome d'hydrogène ou un groupe alkyle ayant jusqu'à 4 atomes de carbone, et R⁴⁹ représente un groupe hydroxy, un groupe alcoxy ayant jusqu'à 4 atomes de carbone, un groupe amino, un groupe amino substitué par un ou deux groupes alkyles ayant jusqu'à 4 atomes de carbone, un groupe carbamoylméthoxy ou carbamoylméthoxy substitué par un ou deux groupes alkyles ayant jusqu'à 4 atomes de carbone au niveau de l'atome d'azote du groupe carbamoyle, ou dans laquelle 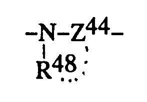 représente un noyau hétérocyclique contenant de 4 à 7 atomes dans le noyau incluant l'atome d'azote auquel Z⁴⁴ et R⁴⁸ sont attachés et R⁴⁷ et R⁴⁹ sont chacun définis comme précédemment, ou bien
(ii) R¹, R² et l'atome d'azote lié à R¹ et R² ensemble représentent un noyau hétérocyclique contenant au moins un atome d'azote et substitué par -COOH, ou bien un noyau hétérocyclique non substitué contenant au moins un atome d'azote, et R³ représente :
(1) un atome d'hydrogène ;
(2) un groupe hydroxy ;
(3) un groupe alkyle ayant jusqu'à 6 atomes de carbone ;
(4) un atome d'halogène ;
(5) un groupe alcoxy ayant jusqu'à 4 atomes de carbone ou
(6) un groupe acyloxy de 2 à 5 atomes de carbone,
et m représente un nombre entier pouvant aller jusqu'à 4, et les sels pharmaceutiquement acceptables de ce dérivé, ledit procédé comprenant l'estérification
d'un composé de formule générale : 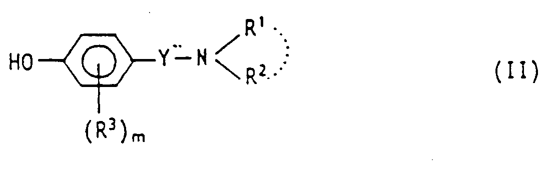 dans laquelle R¹, R², R³ et m sont comme définis précédemment, la formation d'une liaison amide par réaction d'un composé de formule générale : 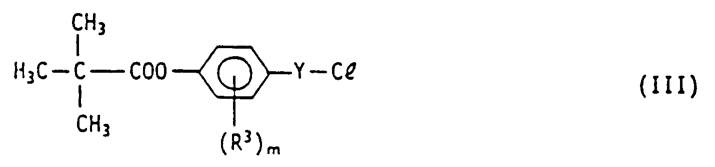 dans laquelle R³, Y et m sont comme définis précédemment, avec une amine de formule HNR¹R², où R¹ et R² sont comme définis précédemment, l'élimination par hydrogénolyse d'un groupe benzyle d'un composé de formule : 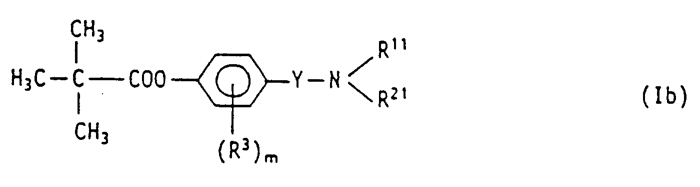 dans laquelle R¹¹ et R²¹ sont comme définis précédemment pour R¹ et R² à la condition que au moins l'un des R¹¹ et R²¹ représente un groupe contenant un groupe benzyloxycarbonyle, et les autres symboles sont comme définis précédemment, par N-alkylation d'un composé de formule générale : 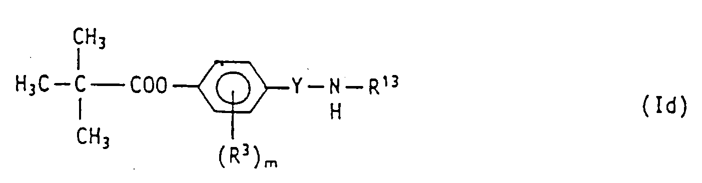 dans laquelle R¹³ est comme défini précédemment pour R¹ et R², sauf pour l'hydrogène et R³, Y et m sont comme définis précédemment, ou bien par désacylation d'un composé de formule générale : 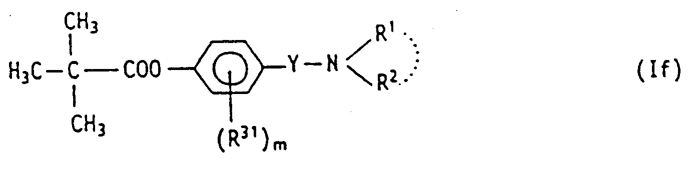 dans laquelle R³¹ représente un groupe acyloxy, et R¹, R², Y et m sont comme définis précédemment, et éventuellement la conversion d'un dérivé d'ester de l'acide pivalique ainsi obtenu en un sel pharmaceutiquement acceptable.

2. Un procédé selon la revendication 1, selon laquelle Y est un groupe sulfonyle (-SO₂-).

3. Un procédé selon la revendication 1 ou 2, selon laquelle Ⓐ est un noyau carbocyclique.

4. Un procédé selon la revendication 1 ou 2, selon laquelle Ⓐ est un groupe phényle.

5. Un procédé selon l'une quelconque des revendications précédentes, selon laquelle l'un des R⁴ est un résidu d'aminoacide.

6. Un procédé selon la revendication 5, selon laquelle le résidu d'aminoacide R⁴ est un résidu de glycine ou d'alanine.

7. Un procédé selon la revendication 6 pour la préparation d'un composé qui est le suivant :
N-[o-(p-pivaloyloxybenzène)sulfonylaminobenzoyl]glycine,
N-[2-(p-pivaloyloxybenzène)sulfonylamino-5-chlorobenzoyl]glycine,
N-[5-méthylthio-2-(p-pivaloyloxybenzène)sulfonylaminobenzoyl]glycine,
N-[2-(p-pivaloyloxybenzène)sulfonylamino-5-propylthiobenzoyl]glycine,
N-[5-méthyl-2-(p-pivaloyloxybenzène)sulfonylaminobenzoyl)glycine,
N-[o-(p-pivaloyloxybenzène)sulfonylaminobenzoyl]glycine, ester méthylique
N-[o-(3-méthyl-4-pivaloyloxybenzène)sulfonylaminobenzoyl]-dl-alanine,
N-[o-(3-méthyl-4-pivaloyloxybenzène)sulfonylaminobenzoyl]-β-alanine,
N-[o-(3-méthyl-4-pivaloyloxybenzène)sulfonylaminobenzoyl]-1-alanine,
N-[5-chloro-2-(3-méthyl-4-pivaloyloxybenzène)sulfonylaminobenzoyl]-1-alanine ou
N-[5-chloro-2-(3-méthyl-4-pivaloyloxybenzène)sulfonylaminobenzoyl]-β-alanine.

8. Un procédé selon l'une quelconque des revendications précédentes suivi de la formulation, comme ingrédient actif, d'un dérivé d'ester de l'acide pivalique de formule générale (1), ou d'un sel pharmaceutiquement acceptable de ce dérivé, ainsi obtenu avec un support ou un enrobage pharmaceutique acceptable.
